# EUROPÄISCHE PATENTSCHRIFT

(11) **EP 3 480 315 B1**
(45) Veröffentlichungstag und Bekanntmachung des Hinweises auf die Patenterteilung: **07.08.2019**
(21) Anmeldenummer: 18188971.8
(22) Anmeldetag: 15.10.2014
(51) Int. Cl.: C12N 15/86, C12N 15/70

(54) **MINICIRCLES MIT VIRALEN EXPRESSIONSKASSETTEN UND IHRE VERWENDUNG ZUR TRANSFORMATION VON ZELLEN ZUR ERZEUGUNG REKOMBINANTER VIREN ODER VIRALER GENVEKTOREN**
MINICIRCLES WITH VIRAL EXPRESSION CASSETTES AND THEIR USE IN THE TRANSFORMATION OF CELLS TO GENERATE RECOMBINANT VIRUSES OR VIRAL GENE VECTORS
MINI-CERCLES DOTÉS DE CASSETTES D'EXPRESSION VIRALES ET LEUR UTILISATION POUR LA TRANSFORMATION DE CELLULES DESTINÉE À LA PRODUCTION DE VIRUS RECOMBINANTS OU DE VECTEURS GÉNÉTIQUES VIRAUX

(30) Priorität: 15.10.2013 DE 102013220859
(43) Veröffentlichungstag der Anmeldung: 08.05.2019
(62) Teilanmeldung aus: 14188981.6
(73) Patentinhaber: PlasmidFactory GmbH & Co. KG, 33607 Bielefeld (DE)
(72) Erfinder: SCHLEEF, Martin, 33739 Bielefeld (DE)
(74) Vertreter: Grund, Martin

(56) Entgegenhaltungen:
- WO-A1-2004/099420
- WO-A2-97/09441
- US-A1- 2013 210 897
- PETER MAYRHOFER ET AL: "Minicircle-DNA production by site specific recombination and protein-DNA interaction chromatography", JOURNAL OF GENE MEDICINE, Bd. 10, Nr. 11, 1. November 2008 (2008-11-01), Seiten 1253-1269, XP002629446, ISSN: 1099-498X, DOI: 10.1002/JGM.1243 [gefunden am 2008-09-02]
- DENNIS KOBELT ET AL: "Performance of High Quality Minicircle DNA for In Vitro and In Vivo Gene Transfer", MOLECULAR BIOTECHNOLOGY, Bd. 53, Nr. 1, 1. April 2012 (2012-04-01), Seiten 80-89, XP035151762, ISSN: 1559-0305, DOI: 10.1007/S12033-012-9535-6
- MARIA SCHNÖDT ET AL: "DNA Minicircle Technology Improves Purity of Adeno-associated Viral Vector Preparations", MOLECULAR THERAPY-NUCLEIC ACIDS, Bd. 5, 1. Januar 2016 (2016-01-01), Seite e355, XP055406390, ISSN: 2162-2531, DOI: 10.1038/mtna.2016.60

## Beschreibung

### Technisches Gebiet

Die vorliegende Erfindung liegt im Gebiet der Biotechnologie. Genauer betrifft die Erfindung die Bereitstellung von Vektoren für die Transformation von Zellen zur Herstellung viraler Vektoren und/oder Viren.

### Hintergrund

Herkömmliche Verfahren zur Gentherapie verwenden häufig Plasmid-DNS als Vektoren zum Einschleusen von erwünschten DNS-Abschnitten in die Zielzellen. Plasmide haben in solchen Anwendungen jedoch häufig den Nachteil einer - verglichen mit viralen Vektoren - geringeren Transfektionseffizienz, weshalb virale Vektoren bevorzugt verwendet werden.

Etwa 25% aller Gentherapieprotokolle, die bisher in klinischen Studien angewendet worden sind, basierten direkt auf Plasmid-DNS-Vektoren (Edelstein et al., J. Gene Med. 2007; 9: 833-42). Es wurde anfänglich erwartet, dass der Marktanteil von Plasmid-DNS-Vektoren bezogen auf alle Impfstoffe auf etwa 60% steigen würde (Jain: "Vectors for gene therapy: Current status and future prospects", PJB Publications Ltd, London, 1996). Dies schließt auch Plasmid-DNS ein, die zur Herstellung viraler Vektoren verwendet wird - z.B. mittels transienter Transfektion von Producerzellen für adenoassoziierte (AAV) Vektoren, lentivirale (LV) Vektoren, retrovirale (RV) Vektoren oder adenovirale (Ad) Vektoren. Die Verwendung von mindestens einem Plasmid zur Herstellung viraler Vektoren oder Viren wurde bereits z.B. für AAV beschrieben
(WO 03/016521 A2), aber auch die Verteilung auf verschiedene (mindestens zwei, aber auch mehr) Plasmide für die o.g. Viren oder viralen Vektoren ist bekannt. In solchen Fällen erfolgt die Kotransfektion der Zellen mit Plasmiden.

Das auf zwei Plasmiden basierende AAV-Verpackungs/Helfer-System aus dem Labor von Jürgen Kleinschmidt (Grimm et al., Hum. Gene Therapy 1998; 9: 2745-60) ist anfänglich für Serotypen 1-6 entwickelt worden. Mutanten mit z.B. Heparinbindungstellen-Defizienz (pDG(R484E/r585E), Kern et al., J. Virol. 2003; 77: 11072-81) und weitere - auch synthetische - Serotypen sind für die Kotransfektion mit nur dem Transferplasmid (das die ITRs enthält) einerseits dem Verpackungs/Helfer-Plasmid (beide Funktionen auf einem einzigen weiteren Plasmid mit über 20 kbp Größe) andererseits erhältlich. Weitere Versionen solcher Systeme sind veröffentlicht worden (Lock et al., Hum. Gene Ther. 21, 1273-1285, WO 97/09441 A2, US 2013/210897 A1), und zwei internationale Referenzstandards sind angewendet worden, um die angemessene klinische Herstellung dieses AAV durch Verwendung des pDG-Plasmidsystems sicherzustellen (Moullier und Snyder, Mol. Ther. 2008, 16:1185-1188). In solchen Fällen ist die Optimierung der Transfektion relativ einfach, da nur das richtige Verhältnis der Mengen beider Plasmide bestimmt werden muss, wenn die Arbeit mit einer neuen Charge eines der Plasmide aufgenommen wird. Das ist bedeutend schwieriger, wenn 3 (oder mehr) Plasmide tripeltransfiziert werden müssen und ihre individuellen relativen Mengen jedes Mal (neu) optimiert werden müssen, wenn eine neue Charge verwendet wird. Einen Überblick geben Ayuso et al. (Curr.: Gene Ther. 2010, 10:423-436).

Die Transferplasmide der Kotransfektion zur AAV-Herstellung enthalten bei Wildtyp-Viren die Sequenzen zur Kodierung der Replikations- und Hüllproteine (rep und cap) zwischen den ITR-Sequenzen. Dieser Bereich wurde im Rahmen der Entwicklung von Systemen zur Herstellung von AAV-Vektoren inklusive der rep- und cap-Gene auf die anderen Plasmide / das andere Plasmid der Kotransfektion verlagert, um auf dem Transferplasmid Platz für die Sequenzen von Interesse zu schaffen, die später im viralen Partikel Platz finden sollen. Die Expression dieser Sequenzen wird bei den herkömmlichen viralen AAV-Vektoren nach deren Einsatz zur Infektion einer Zielzelle (z.B. bei Verwendungen zur Gentherapie) zunächst verzögert beginnen, da die Synthese des Zweitstrangs der DNS (die im viralen Partikel enthaltene DNS ist einzelsträngig) nur mit Hilfe des zellulären Replikationssystems erfolgen kann und dadurch erst die Bildung eines transkriptionskompetenten Doppelstrangs ermöglicht wird. Die Entwicklung sogenannter selbstkomplementärer AAV-Vektoren (Heilbron und Weger, Handb. Exp. Pharmacol. 2010, 197:143-70.) lösen dieses Problem durch die Verwendung doppelsträngiger "Genome" (ITRbegrenzte Sequenzen von Interesse) in den Vektoren. Es hat sich gezeigt, dass diese unmittelbar nach der Vektor-Infektion in der Zielzelle als Doppelstrang zur Verfügung stehen. Erhalten wurden solche doppelsträngigen Virus-Sequenzen dadurch, dass die "terminal resolution site" in einem ITR deletiert wurde und die rep-Proteine diese DNS bei der Vermehrung für den Einbau in die viralen Partikel nicht mehr schneiden konnten. Daher lief die Replikation durch diesen modifizierten ITR hindurch und bildete - unter Verwendung des soeben synthetisierten Strangs als Vorlage - einen komplementären Strang. Der so entstandene DNS-Strang bestand im vorderen Teil aus dem Sinn-Strang der Sequenz von Interesse und - unterbrochen vom nicht aufgelösten modifizierten ITR - aus dem Gegensinn-Strang der Sequenz von Interesse. Die so erzeugten viralen Vektoren sind den bisherigen nicht selbstkomplementären Vektoren bezüglich ihrer Transgenexpression überlegen (D. M. McCarty et al., Gene Ther. 2003, 10(26):2112-2118; Z. Wang et al., Gene Ther. 2003, 10(26):2105-2111).

Nachdem Chardeuf et al. (Mol Ther 2005; 12:744-53) gezeigt hatten, dass die strukturellen Elemente von Plasmidvektoren zur Herstellung von AAV-Partikeln - nämlich Elemente der vom Transferplasmid getragenen Antibiotika-Resistenzgene - in Viruspräparationen nachweisbar waren, verlangten die Regulierungsbehörden nachdrücklich das Vermeiden solcher Sequenzen in AAV-Präparationen. Das dabei auftretende Problem wird auch als "retropackaging" bezeichnet und bedeutet, dass einzelne Sequenzabschnitte derjenigen Plasmide, die Signalstrukturen zur teilweisen Verpackung in die Viren oder viralen Vektoren tragen (sog. "Transferplasmide", gelegentlich auch als "Vektorplasmide" bezeichnet), fehlerhaft in die Viren oder viralen Vektoren verpackt werden. Ein Transferplasmid enthält neben seinen regulatorischen Elementen (bakterieller Replikationsursprung und Selektionsmarker) die zu transferierenden Sequenzen von Interesse (z.B. ein Gen). Diese Sequenzen von Interesse werden nach dem Stand der Technik durch Signalsequenzen flankiert (z.B. sog. ITRs - inverted terminal repeats - bei AAV oder LTRs - long terminal repeats - bei LV). Da ein (intaktes) Plasmid jedoch zirkulär aufgebaut ist, bedeutet die Einrahmung einer Sequenzen von Interesse unter Ausschluss von Replikationsursprung und/oder Selektionsmarker, dass die ausgeschlossenen Elemente, oder wenigstens eines davon (Replikationsursprung und/oder Selektionsmarker) auf der rückwärtigen Seite des Plasmids ebenfalls von diesen Signalsequenzen flankiert sind. Somit können die dort codierten Sequenzen (und das wurde für AAV von Chardeuf *et al.* 2005 gezeigt) ebenfalls - wenn auch mit geringerer Frequenz - in die viralen Capside verpackt werden und zu funktionslosen oder gar gefährlichen viralen Vektoren führen. Diese sind in Präparationen von Viren oder viralen Vektoren nachweisbar und führen neben ihrer pharmazeutischen Gefährlichkeit auch zu einer Mischung aus funktionellen und nicht funktionellen Viren oder viralen Vektoren - dementsprechend mit verringerter Effizienz.

Dies führte uns dazu, die Entwicklung eines Minicircle-Systems - wie hier offenbart - zu erwägen, um dies in der Zukunft zu vermeiden.

Seit kurzem werden sogenannte Minicircle (MC) verwendet, um Zellen zu transfizieren, worunter man kleine, zirkuläre DNS-Moleküle versteht, die eine gewünschte Expressionskassette und möglichst wenige unerwünschte prokaryotische Sequenzen enthalten. Ein Verfahren zur Herstellung von Minicircles wurde in WO 96/26270 beschrieben. Es wurde ferner gezeigt, dass Minicircles, abgesehen von der höheren Biosicherheit auf Grund ihrer geringen Größe, auch bessere Gentransfereigenschaften aufweisen (A. M. Darquet et al., Gene Ther. 1997, 4:1341-1349; A. M. Darquet et al., Gene Ther. 1999, 6:209-218).

Bigger et al. (J. Biol. Chem. 2001, 276:23018-23027) beschreiben die Herstellung von Minicircles mittels der Einführung von Plasmiden mit loxP-Stellen in Bakterien, die die Cre-Rekombinase exprimieren können. Das Plasmid umfasst ferner eine eukaryotische Expressionskassette und eine Markersequenz. Nach Induktion von Cre wird das Plasmid in Minicircle und Miniplasmid gespalten, wobei der Minicircle nur die Expressionskassette enthält. Außerdem sind die loxP-Stellen mutiert, so dass die Reversibilität der Rekombination verringert wird.

Weitere Publikationen beschreiben ebenfalls die Herstellung von Minicircles unter Verwendung anderer Rekombinationssysteme, z.B. Kreiss et al. (Appl. Microbiol. Biotechnol. 1998, 49:560-567) mittels λ-Integrase und Chen et al. (Mol. Ther. 2003, 8:495-500) mittels ΦC31-Integrase. Daher sind Minicircles heute als alternative Vektoren zur Transfektion von eukaryotischen Zellen etabliert.

### Kurze Beschreibung der Erfindung

Die Erfindung wird durch die zugehörigen Ansprüche definiert.

Die vorliegende Erfindung basiert auf der Idee, die Minicircle-Technologie zur Transfektion von Zellen mit der Transfektion (bevorzugt Kotransfektion) von Zellen zur Herstellung von Viren oder viralen Vektoren zu kombinieren. Neben den bekannten Vorteilen, die Transfektion mit Minicircles bietet, führt die Verwendung von Minicircle-basierten DNS-Molekülen an Stelle von Plasmiden zu signifikant sichereren Präparationen von Viren oder viralen Vektoren als die Verwendung herkömmlicher Vektoren, wie etwa eines Plasmids. Insbesondere die virale Verpackung von Sequenzen außer den dafür bestimmten Transfersequenzen kann vermieden werden, da diese gar nicht an der Kotransfektion beteiligt sind oder - in einer weiteren Ausführungsform - zumindest der Transfervektor praktisch keine unerwünschten anderen Sequenzen enthält. In einer weiteren Ausführungsform kann mindestens einer oder auch mehrere der Kotransfektionspartner ein Minicircle sein.

Dementsprechend bezieht sich die Erfindung auf einen Minicircle-Transfervektor umfassend eine Transfersequenz und spezifische Verpackungssignale zu beiden Seiten der Transfersequenz zur Verpackung der Transfersequenz in Partikel eines viralen Vektors. Der Minicircle kann jeweils ein Verpackungssignal oberhalb und unterhalb der Transfersequenz enthalten.

In einer besonderen Ausführungsform ist jener virale Vektor AAV oder ein Retrovirus, wie z.B. ein Lentivirus.

Ferner kann die Transfersequenz eine Expressionskassette mit mindestens einem Gen, mindestens einer siRNA- oder shRNA-kodierenden Sequenz, mindestens einer Isolatorsequenz oder einer Kombination davon umfassen. In einer weiteren Ausführungsform kann der Minicircle auch mindestens eine Stuffersequenz innerhalb des Bereichs zwischen den spezifischen Verpackungssignalen umfassen, oder er kann mindestens eine Stuffersequenz außerhalb des Bereichs zwischen den spezifischen Verpackungssignalen umfassen.

In einer weiteren Ausführungsform umfasst der Minicircle mindestens eine Verpackungs-Expressionskassette, wobei auf der mindestens einen Verpackungs-Expressionskassette sämtliche Proteine kodiert sind und exprimiert werden können, die für die Verpackung der Transfersequenz in Partikel des viralen Vektors notwendig sind.

Ein Aspekt bezieht sich auch auf einen Minicircle-Verpackungsvektor umfassend mindestens eine Verpackungs-Expressionskassette, wobei auf der mindestens einen Verpackungs-Expressionskassette mindestens ein Protein kodiert ist und exprimiert werden kann, das für die Verpackung einer Transfersequenz in Partikel eines viralen Vektors notwendig ist. Auf der mindestens einen Verpackungs-Expressionskassette können aber auch sämtliche Proteine kodiert sein und exprimiert werden, die für die Verpackung einer Transfersequenz in Partikel eines viralen Vektors notwendig sind. In bestimmten Aspekten ist jener virale Vektor AAV oder ein Retrovirus, wie z.B. ein Lentivirus. Wenn der Vektor AAV ist, kann er vom Serotyp 1, 2, 3, 4, 5, 6, oder einem synthetischen Serotyp sein.

Die Erfindung bezieht sich auch auf Zellen umfassend einen Minicircle gemäß einer der erwähnten Ausführungsformen.

Unter einem weiteren Gesichtspunkt bezieht sich die Erfindung auf ein Verfahren zur Herstellung eines viralen Vektors, das Verfahren umfassend entweder i) Transfektion einer eukaryotischen Zelle mit mindestens einem Verpackungsvektor umfassend mindestens eine Verpackungs-Expressionskassette, wobei auf der mindestens einen Verpackungs-Expressionskassette sämtliche Proteine kodiert sind und exprimiert werden können, die für die Verpackung jener Transfersequenz in Partikel jenes viralen Vektors notwendig sind; und ii) Transfektion besagter eukaryotischen Zelle oder eines ihrer Nachkommen mit einem erfindungsgemäßen Minicircle-Transfervektor umfassend eine Transfersequenz; oder iii) Transfektion einer eukaryotischen Zelle mit einem Minicircle, der sowohl die Transfersequenz als auch kodierende Sequenzen für sämtliche Proteine trägt, die für die Verpackung jener Transfersequenz in Partikel jenes viralen Vektors notwendig sind; ferner Expression der mindestens einen Verpackungs-Expressionskassette; und Isolierung des viralen Vektors aus der eukaryotischen Zelle oder einem ihrer Nachkommen, oder aus dem Medium, in dem diese sich befinden.

In einem weiteren Aspekt wird in Schritt (i) ein einzelner Vektor verwendet, der vorzugsweise ein Minicircle der Erfindung ist.

In einer anderen Ausführungsform bezieht sich die Erfindung auf ein Verfahren zur Herstellung eines viralen Vektors, das Verfahren umfassend i) Transfektion einer eukaryotischen Zelle mit mindestens einem erfindungsgemäßen Minicircle-Verpackungsvektor, wobei auf dem mindestens einen Minicircle-Verpackungsvektor sämtliche Proteine kodiert sind und exprimiert werden können, die für die Verpackung jener Transfersequenz in Partikel jenes viralen Vektors notwendig sind; und ii) Transfektion besagter eukaryotischen Zelle oder eines ihrer Nachkommen mit einem Transfervektor umfassend eine Transfersequenz und spezifische Verpackungssignale zu beiden Seiten der Transfersequenz zur Verpackung der Transfersequenz in Partikel eines viralen Vektors; ferner Expression der mindestens einen Verpackungs-Expressionskassette; und Isolierung des viralen Vektors aus der eukaryotischen Zelle oder einem ihrer Nachkommen, oder aus dem Medium, in dem diese sich befinden.

Die Schritte (i) und (ii) können zugleich in einer Kotransfektion durchgeführt werden; es kann aber auch Schritt (i) vor Schritt (ii) durchgeführt werden oder Schritt (i) nach Schritt (ii) durchgeführt werden. In einem weiteren Aspekt wird in Schritt (i) ein Verpackungsvektor transfiziert, der episomal stabil in der eukaryotischen Zelle verbleibt, und Schritt (ii) wird mit einem Nachkommen besagter eukaryotischen Zelle durchgeführt.

In einer bevorzugten Ausführungsform kann die eukaryotische Zelle eine Säugetierzelle sein.

Des Weiteren bezieht sich die Erfindung auch auf einen viralen Partikel, der durch ein erfindungsgemäßes Verfahren erhalten wurde.

Schließlich umfasst die Offenbarung auch ein Kit zur Herstellung eines viralen Vektors umfassend: einen erfindungsgemäßen Minicircle-Transfervektor; und mindestens einen Verpackungsvektor umfassend mindestens eine Verpackungs-Expressionskassette, wobei auf der mindestens einen Verpackungs-Expressionskassette sämtliche Proteine kodiert sind und exprimiert werden können, die für die Verpackung jener Transfersequenz in Partikel jenes viralen Vektors notwendig sind. Der mindestens eine Verpackungsvektor kann ein einzelner erfindungsgemäßer Minicircle-Verpackungsvektor sein. Offenbart ist auch ein Kit zur Herstellung eines viralen Vektors umfassend: einen Transfervektor umfassend eine Transfersequenz und spezifische Verpackungssignale zu beiden Seiten der Transfersequenz zur Verpackung der Transfersequenz in Partikel jenes viralen Vektors; und mindestens einen erfindungsgemäßen Minicircle-Verpackungsvektor, wobei auf dem mindestens einen Minicircle-Verpackungsvektor sämtliche Proteine kodiert sind und exprimiert werden können, die für die Verpackung jener Transfersequenz in Partikel jenes viralen Vektors notwendig sind.

Jener virale Vektor kann AAV oder ein Retrovirus, wie z.B. ein Lentivirus, sein. Wenn der Vektor AAV ist, kann er vom Serotyp 1, 2, 3, 4, 5, 6, oder einem synthetischen Serotyp sein.

### Beschreibung der Abbildungen

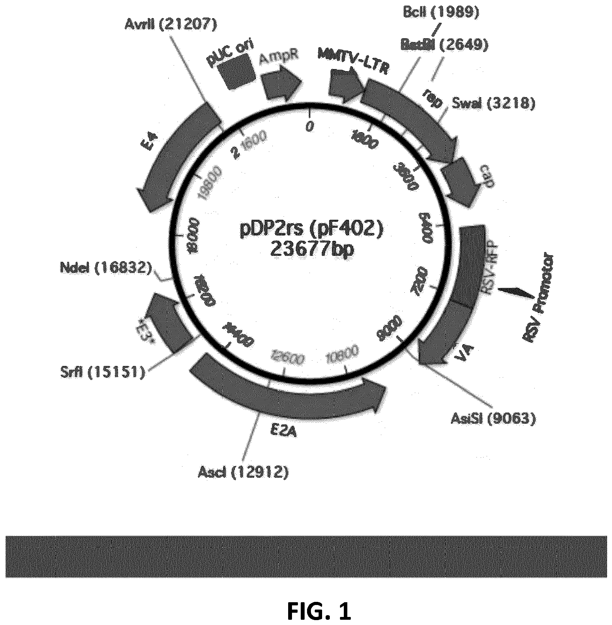

FIG. 1 zeigt die Plasmidkarte von pDP2rs. Alle Helfer- und Verpackungssequenzen sind als cap, rep, VA, E2A, E4 und E3 gekennzeichnet. Zur Identifizierung einer erfolgreichen Transfektion wird das rot fluoreszierende Protein (RFP) verwendet. Das Gen dafür befindet sich stromabwärts von cap. Einige singuläre Schnittstellen sind zur Orientierung angegeben.

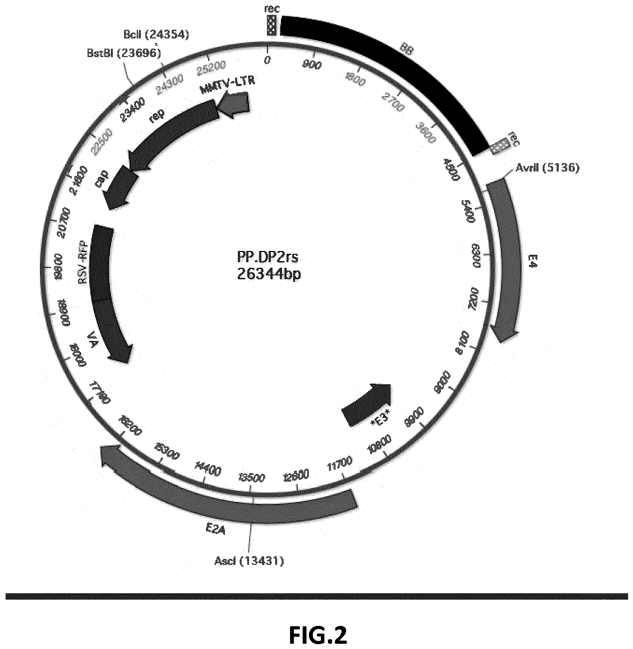

FIG. 2 zeigt die Karte des Parentalplasmids PP.DP2rs. Die Rekombinase-Erkennungssequenzen sind als "rec" bezeichnet und grenzen den Abschnitt des Parentalplasmids, der nach Rekombination der Minicircle wird (Minicircle-Bereich), vom bakteriellen Plasmidanteil ("BB"; Miniplasmid-Bereich) ab, welcher nach Rekombination Miniplasmid wird und die dafür notwendigen regulatorischen Elemente wie z.B. ein Gen für die Rekombinase (hier ParA-Resolvase), aber auch Antibiotika-Resistenzgene (hier Kanamycin-Resistenz) und den bakteriellen Replikationsursprung enthält. Einige singuläre Schnittstellen sind zur Orientierung angegeben.

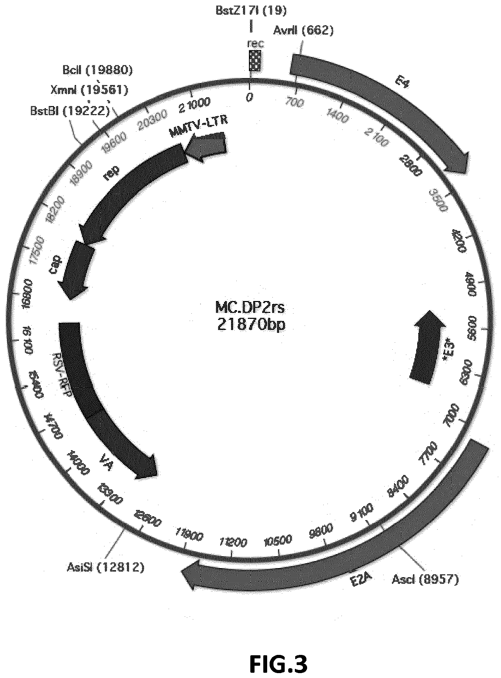

FIG. 3 zeigt die Karte des Minicircles MC.DP2rs. "rec" stellt hierbei die rekombinierte Rekombinase-Erkennungssequenz dar, die nach der Rekombination im Minicircle verbleibt. Einige singuläre Schnittstellen sind zur Orientierung angegeben.

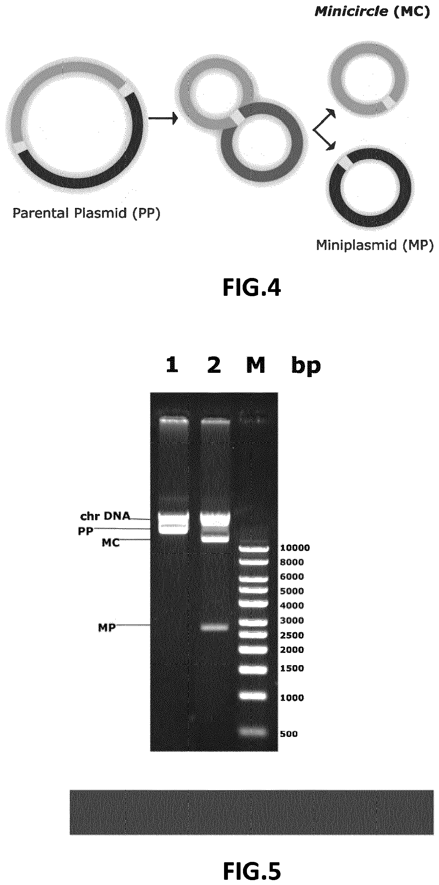

FIG. 4 zeigt schematisch die Erzeugung von Minicircle und Miniplasmid aus einem Parentalplasmid.
FIG. 5 zeigt ein Agarosegel mit unverdautem monomerem PP.DP2rs (PP) (Spur 1), unverdautem monomerem MC.DP2rs (MC) und unverdautem monomerem Mini-Plasmid (unten: MP) (beides Spur 2) im Vergleich zu einem DNA-Längenstandard aus linearen DNA-Fragmenten definierter Länge (1 Kb-Leiter, PlasmidFactory, Bielefeld; auf ganze 500 bp gerundete Größenangaben rechts neben dem Gel). Chr DNA = bakterielle chromosomale DNA aus Kit-grade DNA Extraktion.

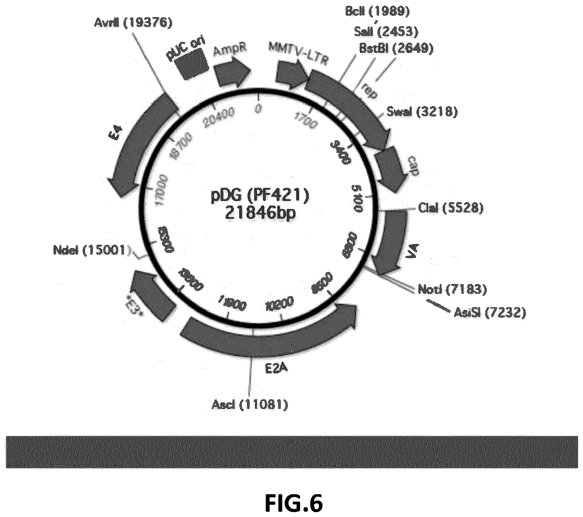

FIG. 6 zeigt die Plasmidkarte von pDG. Alle Helfer- und Verpackungssequenzen sind als cap, rep, VA, E2A, E4 und E3 gekennzeichnet. Dieses Plasmid enthält kein Gen für RFP. Einige singuläre Schnittstellen sind zur Orientierung angegeben.

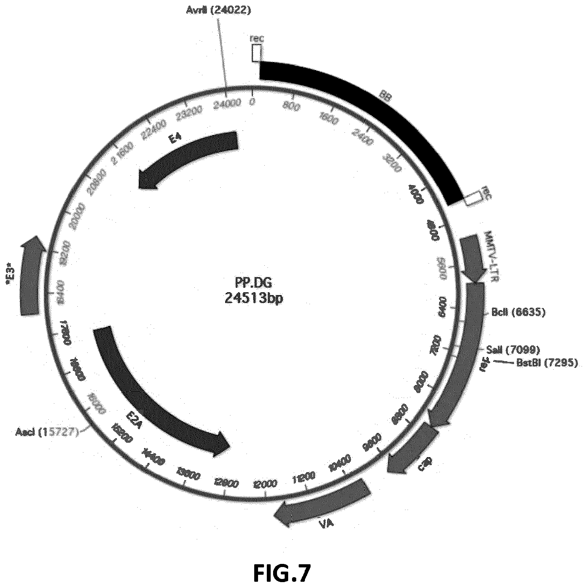

FIG. 7 zeigt die Karte des Parentalplasmids PP.DG. Die Rekombinase-Erkennungssequenzen sind als "rec" bezeichnet und grenzen den Abschnitt des Parentalplasmids, der nach Rekombination der Minicircle wird (Minicircle-Bereich), vom bakteriellen Plasmidanteil ("BB"; Miniplasmid-Bereich) ab, welcher nach Rekombination Miniplasmid wird und die dafür notwendigen regulatorischen Elemente wie z.B. ein Gen für die Rekombinase (hier ParA-Resolvase), aber auch Antibiotika-Reesitenzgene (hier Kanamycin-Resistenz) und den bakteriellen Replikationsursprung enthält. Einige singuläre Schnittstellen sind zur Orientierung angegeben.

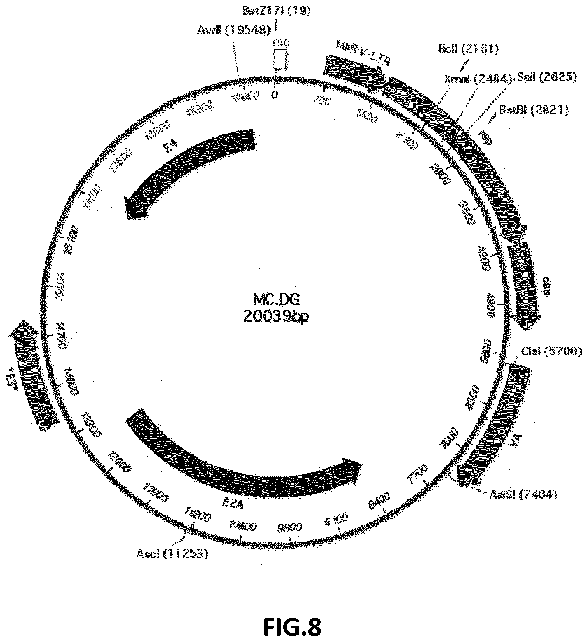

FIG. 8 zeigt die Karte des Minicircles MC.DG. "rec" stellt hierbei die rekombinierte Rekombinase-Erkennungssequenz dar, die nach der Rekombination im Minicircle verbleibt. Einige singuläre Schnittstellen sind zur Orientierung angegeben.

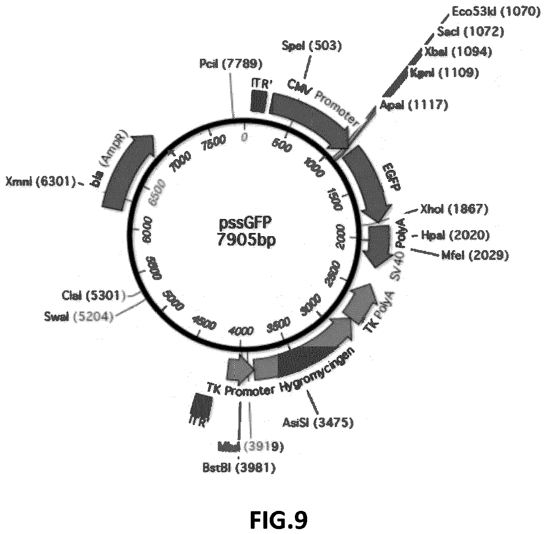

FIG. 9 zeigt die Karte des Plasmids pssGFP. Im linken Teil des Plasmids befindet sich neben dem bakteriellen Replikationsurpsprung (nicht dargestellt) das Antibiotika-Resistenzgen (bla). Der rechte Teil wird durch die ITR-Sequenzen begrenzt und enthält zwei aufeinander gerichtete Expressionseinheiten: für grün fluoreszierendes Protein (EGFP) unter der Kontrolle eines CMV-Promoters und mit einer Poly-Adenylierungssequenz aus SV40 (SV40 PolyA), sowie für Hygromycin unter der Kontrolle des TK-Promoters mit einer TK-Polyandenylierungssequenz (TK PolyA). Einige singuläre Schnittstellen sind zur Orientierung angegeben.

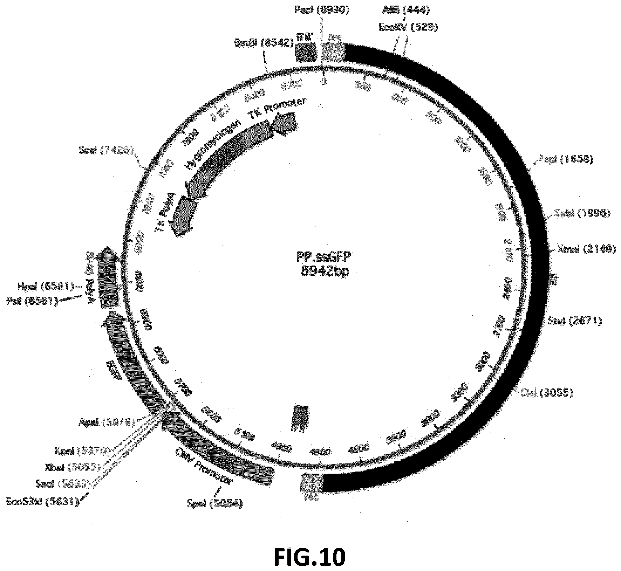

FIG. 10 zeigt die Karte des Parentalplasmids (PP-ssGFP). Die Rekombinase-Erkennungssequenzen sind als "rec" bezeichnet und grenzen den Abschnitt des Parentalplasmids, der nach Rekombination der Minicircle wird (Minicircle-Bereich), vom bakteriellen Plasmidanteil ("BB"; Miniplasmid-Bereich) ab, welcher nach Rekombination Miniplasmid wird und die dafür notwendigen regulatorischen Elemente wie z.B. ein Gen für die Rekombinase (hier ParA-Resolvase), aber auch Antibiotika-Resistenzgene (hier Kanamycin-Resistenz) und den bakteriellen Replikationsursprung enthält. Einige singuläre Schnittstellen sind zur Orientierung angegeben.

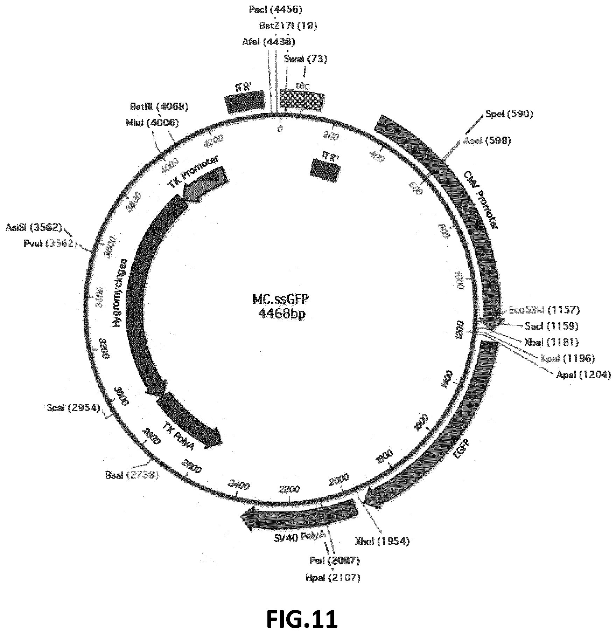

FIG. 11 zeigt die Karte des Minicircle MC.ssGFP. "rec" stellt hierbei die rekombinierte Rekombinase-Erkennungssequenz dar, die nach der Rekombination im Minicircle verbleibt. Einige singuläre Schnittstellen sind zur Orientierung angegeben.

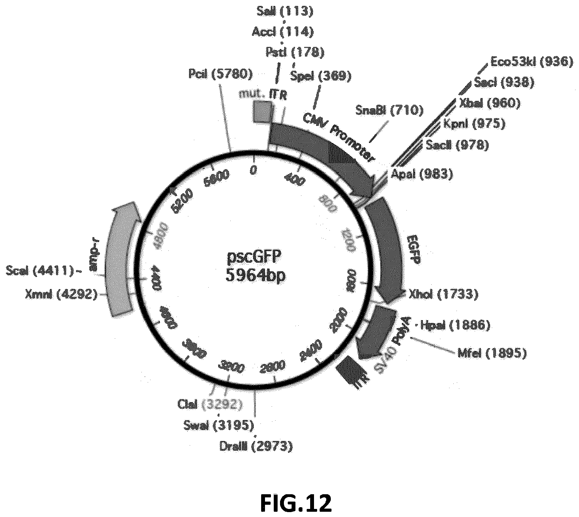

FIG. 12 zeigt die Karte des Plasmids pscGFP. Im Gegensatz zu pssGFP (FIG. 9) wird dieses Plasmid zur Erzeugung selbst-complementärer AAV Viren verwendet. Im linken Teil des Plasmids befindet sich neben dem bakteriellen Replikationsursprung (nicht dargestellt) das Antibiotika-Resistenzgen (amp-r). Der rechte Teil wird durch die ITR-Sequenzen begrenzt und enthält eine Expressionseinheit für grün fluoreszierendes Protein (EGFP) unter der Kontrolle eines CMV-Promoters und mit einer Poly-Adenylierungssequenz aus SV40 (SV40 PolyA). Einige singuläre Schnittstellen sind zur Orientierung angegeben. Die eine der beiden ITR-Sequenzen (mut. ITR) ist mutiert.

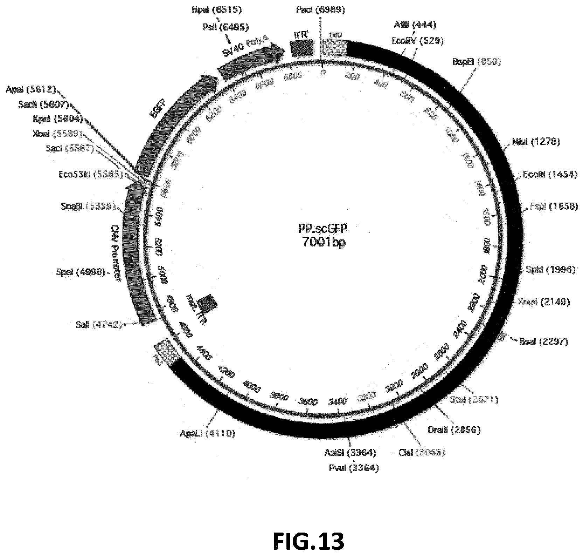

FIG. 13 zeigt die Karte des Parentalplasmids (PP-scGFP). Die Rekombinase-Erkennungssequenzen sind als "rec" bezeichnet und grenzen den Abschnitt des Parentalplasmids, der nach Rekombination der Minicircle wird (Minicircle-Bereich), vom bakteriellen Plasmidanteil ("BB"; Miniplasmid-Bereich) ab, welcher nach Rekombination Miniplasmid wird und die dafür notwendigen regulatorischen Elemente wie z.B. ein Gen für die Rekombinase (hier ParA-Resolvase), aber auch Antibiotika-Resistenzgene (hier Kanamycin-Resistenz) und den bakteriellen Replikationsursprung enthält. Einige singuläre Schnittstellen sind zur Orientierung angegeben.

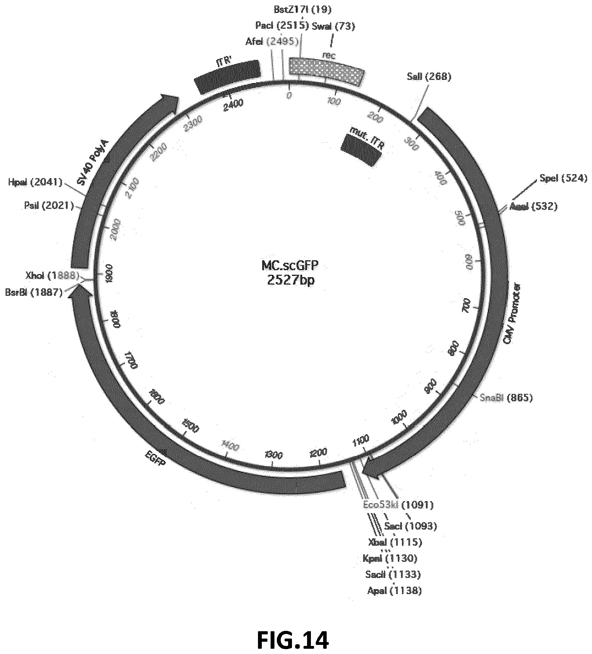

FIG. 14 zeigt die Karte des Minicircle MC.scGFP. "rec" stellt hierbei die rekombinierte Rekombinase-Erkennungssequenz dar, die nach der Rekombination im Minicircle verbleibt. Einige singuläre Schnittstellen sind zur Orientierung angegeben.

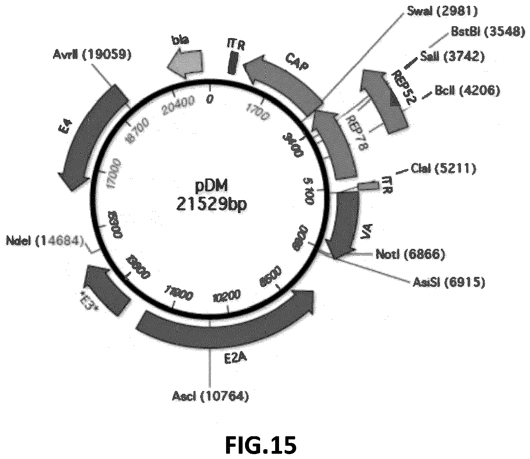

FIG. 15 zeigt die Plasmidkarte von pDM - ein 1-Plasmid-Vektor zur Erzeugung von AAV-Partikeln. Alle Helfer- und Verpackungssequenzen sind als cap, rep, VA, E2A, E4 und E3 gekennzeichnet. Die rep- (REP52 und REP78) und cap- (CAP) Sequenzen werden als Cluster von ITR-Sequenzen des AAV flankiert. Einige singuläre Schnittstellen sind zur Orientierung angegeben.

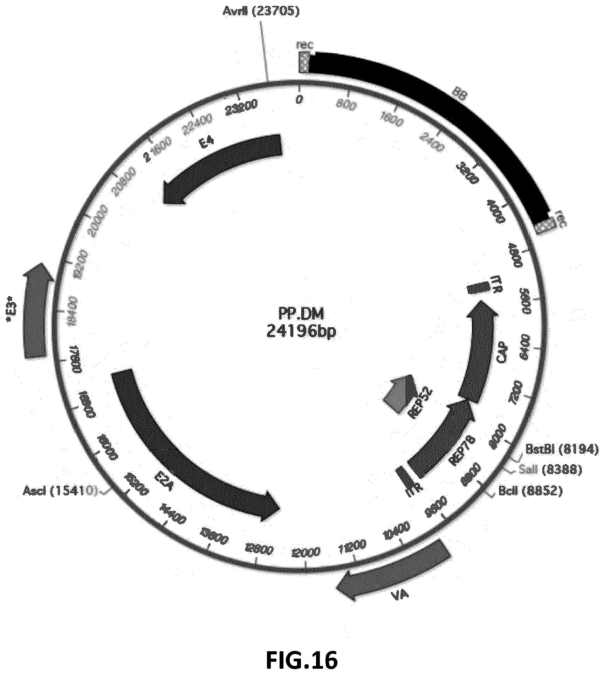

FIG. 16 zeigt die Karte des Parentalplasmids PP.DM. Die Rekombinase-Erkennungssequenzen sind als "rec" bezeichnet und grenzen den Abschnitt des Parentalplasmids, der nach Rekombination der Minicircle wird (Minicircle-Bereich), vom bakteriellen Plasmidanteil ("BB"; Miniplasmid-Bereich) ab, welcher nach Rekombination Miniplasmid wird und die dafür notwendigen regulatorischen Elemente wie z.B. ein Gen für die Rekombinase (hier ParA-Resolvase), aber auch Antibiotika-Reesitenzgene (hier Kanamycin-Resistenz) und den bakteriellen Replikationsursprung enthält. Einige singuläre Schnittstellen sind zur Orientierung angegeben.

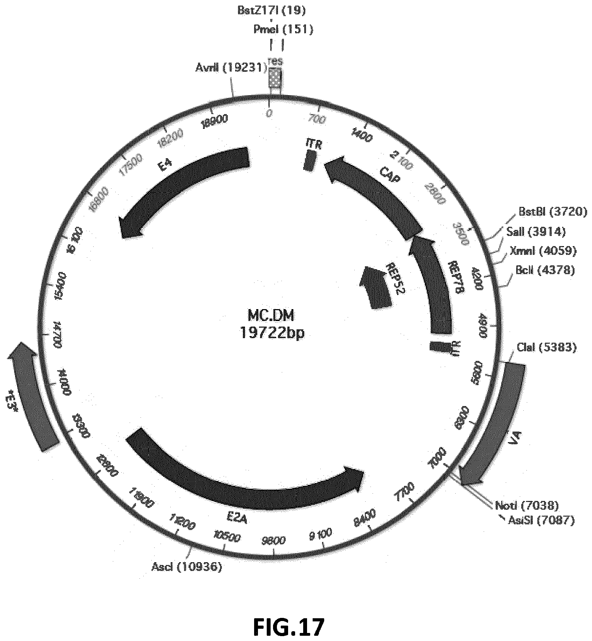

FIG. 17 zeigt die Karte des Minicircles MC.DM. "rec" stellt hierbei die rekombinierte Rekombinase-Erkennungssequenz dar, die nach der Rekombination im Minicircle verbleibt. Die anderen Elemente sind in **FIG. 17** beschrieben. Einige singuläre Schnittstellen sind zur Orientierung angegeben.

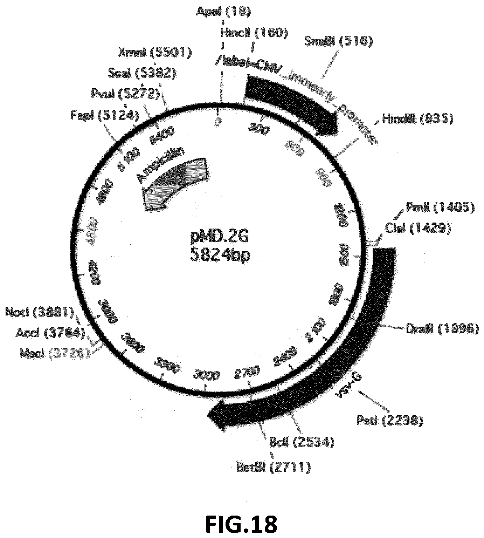

FIG. 18 zeigt die Plasmidkarte von pMD.2G (Addgene-Nr. 12259). Es kodiert unter der Kontrolle des CMV-Promoters für Glykoprotein G des Vesikuläre-Stomatitis-Virus (VSV-G) und wird zur Hersetllung von LV kotransfiziert. Einige singuläre Schnittstellen sind zur Orientierung angegeben.

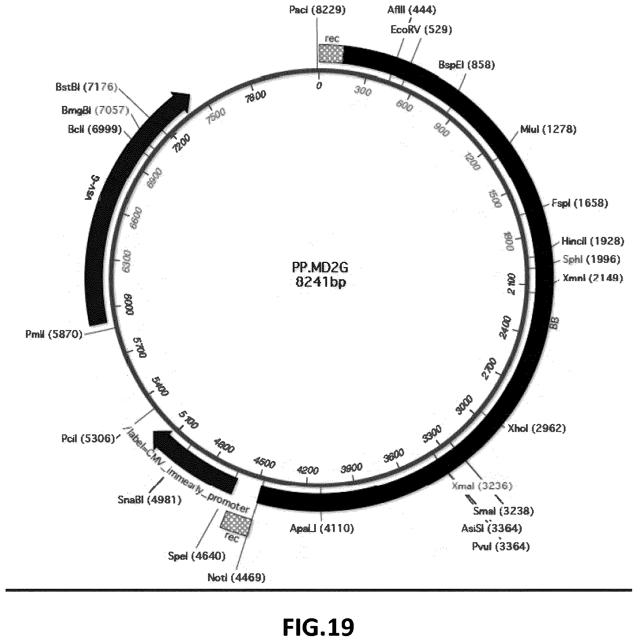

FIG. 19 zeigt die Karte des Parentalplasmids PP.MD2G. Die Rekombinase-Erkennungssequenzen sind als "rec" bezeichnet und grenzen den Abschnitt des Parentalplasmids, der nach Rekombination der Minicircle wird (Minicircle-Bereich), vom bakteriellen Plasmidanteil ("BB"; Miniplasmid-Bereich) ab, welcher nach Rekombination Miniplasmid wird und die dafür notwendigen regulatorischen Elemente wie z.B. ein Gen für die Rekombinase (hier ParA-Resolvase), aber auch Antibiotika-Reesitenzgene (hier Kanamycin-Resistenz) und den bakteriellen Replikationsursprung enthält. Einige singuläre Schnittstellen sind zur Orientierung angegeben.

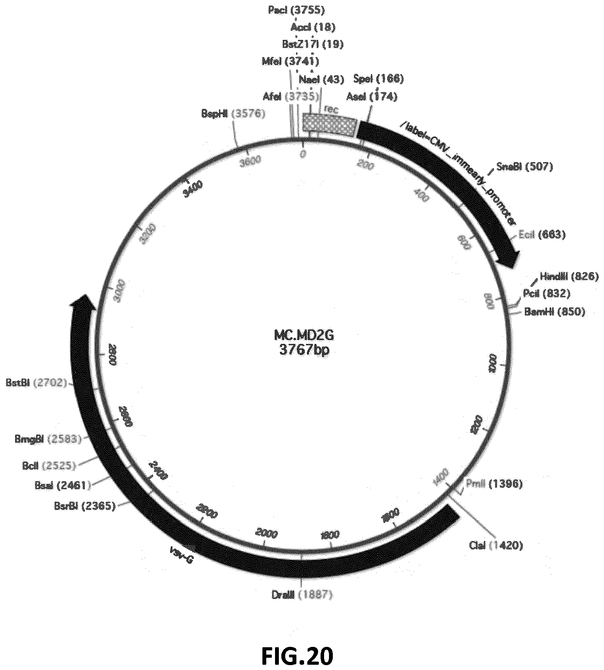

FIG. 20 zeigt die Karte des Minicircles MC.MD2G. "rec" stellt hierbei die rekombinierte Rekombinase-Erkennungssequenz dar, die nach der Rekombination im Minicircle verbleibt. Die anderen Elemente entsprechen denjenigen in FIG. 18. Einige singuläre Schnittstellen sind zur Orientierung angegeben.

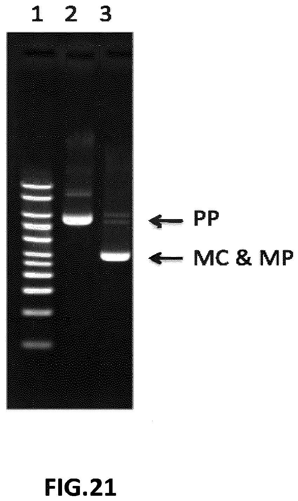

FIG. 21 zeigt ein Agarosegel mit unverdautem monomerem PP.ssGFP (PP) (Spur 2) sowie unverdautem monomerem MC.ssGFP (MC) und unverdautem monomerem Miniplasmid (MP) (Spur 3). MP und MC liegen wegen beinahe gleicher Größe als Doppelbande vor. Als Vergleich dient ein DNA-Längenstandard aus linearen DNA-Fragmenten definierter Länge (1 kbp-Leiter, PlasmidFactory, Bielefeld).

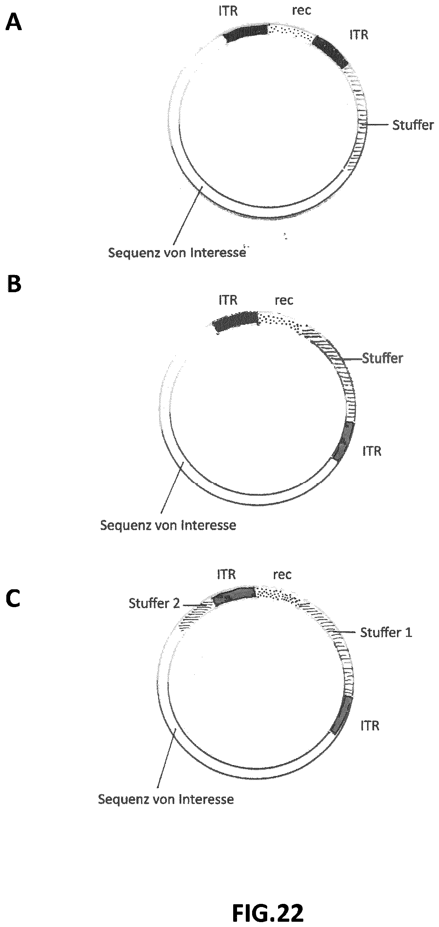

FIG. 22A zeigt die Skizze eines Minicircle als Transfervektor zur AAV-Herstellung, bei dem zwischen den ITR-Sequenzen (ausgefüllter Abschnitt, proximaler Bereich) die Transfersequenz (offener Abschnitt) und eine Stuffersequenz (gestreifter Abschnitt) positioniert ist. Jenseits der ITR-Sequenzen (distaler Bereich) befindet sich eine "rec"-Sequenz (gepunkteter Abschnitt) auf dem Minicircle. Die ITR-Sequenzen könnten auch LTR-Sequenzen sein oder andere Signal-Sequenzen zur Begrenzung des Sequenzabschnittes zur viralen Verpackung.
FIG. 22B zeigt die Skizze eines Minicircle als Transfervektor zur AAV-Herstellung, bei dem zwischen den ITR-Sequenzen (ausgefüllter Abschnitt; proximaler Bereich) die Transfersequenz (offener Abschnitt) positioniert ist. Jenseits der ITR-Sequenzen (distaler Bereich) befindet sich eine "rec"-Sequenz (gepunkteter Abschnitt) und eine Stuffersequenz (gestreifter Abschnitt). Die ITR-Sequenzen könnten auch LTR-Sequenzen sein oder andere Signal-Sequenzen zur Begrenzung des Sequenzabschnittes zur viralen Verpackung.
FIG. 22C zeigt die Skizze eines Minicircle als Transfervektor zur AAV-Herstellung, bei dem zwischen den ITR-Sequenzen (ausgefüllter Abschnitt; proximaler Bereich) die Transfersequenz (offener Abschnitt) und eine Stuffersequenz (gestreifter Abschnitt: Stuffer 2) positioniert ist. Jenseits der ITR-Sequenzen (distaler Bereich) befindet sich eine "rec"-Sequenz (gepunkteter Abschnitt) und eine Stuffersequenz (gestreifter Abschnitt: Stuffer 1). Die ITR-Sequenzen könnten auch LTR-Sequenzen sein oder andere Signal-Sequenzen zur Begrenzung des Sequenzabschnittes zur viralen Verpackung.

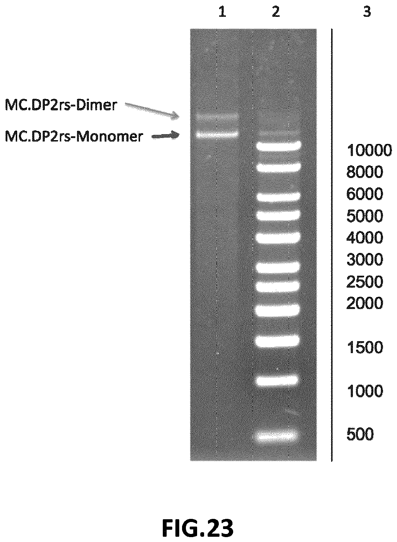

FIG. 23 zeigt ein Agarosegel mit unverdautem monomerem und dimerem PP.DP2rs (Spur 1) neben einem DNA-Längenstandard aus linearen DNA-Fragmenten definierter Länge (1 Kb-Leiter, PlasmidFactory, Bielefeld; auf ganze 500 bp gerundete Größenangaben rechts neben dem Gel).

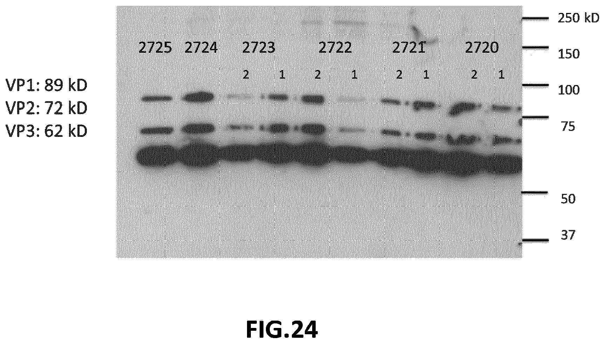

FIG. 24 zeigt einen Western Blot der Capsidproteine aus dem Experiment von Beispiel 15.

### Ausführliche Beschreibung der Erfindung

### MINICIRCLES

Die vorliegende Erfindung betrifft unter einem Gesichtspunkt einen Minicircle, umfassend mindestens eine Transfersequenz und spezifische Verpackungssignale zu beiden Seiten der Transfersequenz zur Verpackung der Transfersequenz in Partikel eines viralen Vektors ("Minicircle-Transfervektor"). Bei dem viralen Vektor handelt es sich in bevorzugten Ausführungsformen um einen AAV-Vektor oder einen lentiviralen Vektor.

"Minicircle" im Sinne dieser Erfindung bezeichnet eine zirkuläre doppelsträngige DNS, die mindestens eine Nukleinsäuresequenz von Interesse enthält, und im Wesentlichen frei ist von prokaryotischen oder bakteriellen Nukleinsäuresequenzen, wie sie typischerweise in Plasmiden vorgefunden werden, wie etwa Replikationsstartpunkten, Markergenen oder Resistenzgenen. Von Promotoren enthalten Minicircles, wenn überhaupt, nur solche, die zur Expression von Genen in der Nukleinsäuresequenz von Interesse dienen. Insbesondere sind die Minicircles der Erfindung frei von Resistenzgenen gegen Antibiotika und bakteriellen Replikationsstartpunkten. Dadurch sind Minicircles besonders geeignet zum Einbringen von gewünschten Nukleinsäuresequenzen in Zielzellen, da Wirkungen unerwünschter DNS-Elemente, wie etwa die Expression oder Rekombination von Resistenzgenen, oder die Anwesenheit von CpG-Moviven ausgeschlossen oder reduziert werden können und z.B. überflüssige und funktionslose Sequenzen vermieden werden.

Eine "Nukleinsäuresequenz von Interesse" kann jede doppelsträngige DNS-Sequenz sein, die in eine eukaryotische Zielzelle eingebracht in der Zielzelle exprimiert oder auch in eine Nukleinsäure der Zielzelle transponiert werden soll. Die Nukleinsäuresequenz von Interesse kann eine Expressionskassette für ein oder mehr Gene sein.

Eine "Expressionskassette" ist eine DNS-Sequenz, die ein oder mehr Gene enthält sowie Sequenzen, die ihre Expression kontrollieren. Insbesondere umfasst eine Expressionskassette Promotorsequenzen, offene Leserahmen, die für die zu exprimierenden Polypeptide kodieren, und 3'-untranslatierte Bereiche, die bei Expression in Eukaryoten gewöhnlich eine Polyadenylierungssequenz enthalten. Eine Expressionskassette kann auch mehr als ein Gen enthalten, z.B. 2, 3, 4, 5, 6, 7, 8, 9, 10 oder mehr Gene.

Unter einem Gesichtspunkt dienen Minicircles der Erfindung als Transfervektoren für die Herstellung viraler Vektoren, die eine Transfersequenz enthalten. Als "Transfersequenz" wird hierbei jegliche Nukleinsäuresequenz verstanden, die in einen viralen Vektor verpackt und anschließend in weitere Zielzellen transferiert werden soll. Typischerweise enthalten Transfersequenzen Expressionskassetten, auf denen ein oder mehr Gene kodiert sind. In einem anderen Aspekt kann es sich jedoch bei einer Transfersequenz auch um eine Sequenz handeln, die nicht für ein Protein kodiert, sondern lediglich dazu dient, einen Abstand zwischen zwei DNS-Abschnitten zu generieren oder DNS-bindende Faktoren (z.B. Nukleinsäuren oder Proteine) unter bestimmten Bedingungen zu binden bzw. zu entlassen und so in die Genregulation der Zielzelle einzugreifen. Weiterhin kann es sich bei der Transfersequenz um eine RNS-kodierende Sequenz (z.B. für siRNA oder shRNA) handeln oder um andere genetische Elemente, die in der Zielzelle wirksam werden sollen, z.B. Isolatorsequenzen (also Sequenzmotive, die, wenn sie in das Genom integriert werden, die regulierende Wirkung eines chromosomalen Abschnitts auf seine benachbarten Regionen beeinflussen). Außerdem kann eine Transfersequenz eine beabsichtigte Wirkung in Form einer Stimulation zellulärer oder immunologischer Reaktionen (z.B. durch CpG-Motive, siehe A. Krieg et al., Nature 1995, 374:546-9) haben. Zur Herstellung solcher viraler Vektoren können verschiedene Strategien verwendet werden. Sehr verbreitet ist es, solche Vektoren in eukaryotischen Zellen, sog. "Producerzellen", herzustellen. Geeignete Producerzelllinien sind z.B. HEK293, HeLa, oder XDC293.

Zur Vektorproduktion muss in die Producerzellen sowohl die Transfersequenz eingebracht werden als auch weitere Nukleinsäuresequenzen mit Expressionskassetten, sog. "Verpackungs-Expressionskassetten", welche bei ihrer Expression die Produktion der viralen Proteine und die Verpackung der Transfersequenz in virale Vektoren vermitteln. Es besteht die Möglichkeit, sowohl die Transfersequenz als auch die Verpackungs-Expressionskassetten auf demselben Vektor in die Producerzellen einzubringen, als auch, dies mittels Kotransfektion mehrerer verschiedener Vektoren zu tun. Derjenige Vektor, der die Transfersequenz enthält, wird hierbei "Transfervektor" genannt. Werden weitere Verpackungs-Expressionskassetten auf anderen Vektoren kodiert, nennt man diese "Verpackungsvektoren".

Unter einem Gesichtspunkt umfasst die Erfindung daher Transfervektoren, die als Minicircles ausgebildet sind ("Minicircle-Transfervektoren"). Hierbei ist die Transfersequenz Bestandteil der Nukleinsäuresequenz von Interesse des Minicircles. Ferner umfasst die Nukleinsäure von Interesse "spezifische Verpackungssignale" zu beiden Seiten der Transfersequenz. Dies sind Nukleinsäuresequenzen, die in der Producerzelle die Verpackung der Transfersequenz in den viralen Vektor vermitteln. Typische spezifische Verpackungssignale sind ITR-Sequenzen für AAV und Ad Vektoren sowie LTR-Sequenzen für lentivirale und retrovirale Vektoren. Beispielhafte Transfervektoren sind pTRUF11 (ATCC MBA-331) oder pSUB 201 (Chardeuf et al., Mol. Ther. 2005; 12:744-53) für AAV- oder pSEW (C. Demaison et al., Hum. Gene Ther. 2005, 12:900-912) für lentivirale Vektoren.

In einemAspekt enthält ein Minicircle-Transfervektor jeweils ein spezifisches Verpackungssignal, z.B. eine ITR-Sequenz, oberhalb und unterhalb der Transfersequenz.

In einer bevorzugten Ausführungsform kann außerdem ein Minicircle-Transfervektor zur Verbesserung der viralen Verpackungseffizienz eine "Stuffersequenz" umfassen. Hierbei handelt es sich um ein zusätzliches Stück Nukleinsäuresequenz, die eingefügt wird, um die Verpackungseffizienz zu erhöhen. Die Stuffersequenz kann prinzipiell eine beliebige Sequenz aufweisen, jedoch ist darauf zu achten, dass sie keine Elemente enthält, die mit einem Minicircle inkompatibel sind (insbesondere z.B. bakterielle Replikationsstartpunkte und Resistenzgene gegen Antibiotika). Die Stuffersequenz kann auch eine beliebige Länge haben, z.B. mindestens 1, 10, 100, 200, 300, 400, 500, 1000, 2000, 5000, oder 10000 bp. In einem weiteren Aspekt befindet sich die Stuffersequenz, wie in FIG. 22A dargestellt, zusammen mit der Transfersequenz zwischen den spezifischen Verpackungssignalen, um die Transfersequenz derart zu verlängern, dass sie die Mindestlänge zur viralen Verpackung erreicht. Beispielsweise ist für die effiziente Verpackung in einen AAV-Vektor eine Sequenz von 3,5 bis 4 kbp zwischen den spezifischen Verpackungssignalen (den ITR-Sequenzen) optimal. In einem weiteren Aspekt kann aber eine Stuffersequenz auch außerhalb der spezifischen Verpackungssignale platziert werden, wie in FIG. 22B dargestellt. In solchen Fällen dient sie dazu, den "äußeren" Abstand der Verpackungssignale (also den nicht über die Transfersequenz, sondern in der anderen Richtung auf dem kreisförmigen Minicircle gemessenen Abstand) für die Verpackung zu optimieren. In einem weiteren Aspekt der Minicircle-Transfervektoren befinden sich Stuffersequenzen sowohl zwischen den spezifischen Verpackungssignalen als auch außerhalb davon (FIG. 22C).

In einer Ausführungsform kann der virale Vektor, in den die Transfersequenz verpackt werden soll, ein AAV-Vektor sein. In diesem Fall wird mindestens eine Verpackungs-Expressionskassette benötigt, die die Gene rep und cap von AAV sowie die adenoviralen Gene VA, E2A und E4 enthält. In einem sog. Mono-Transfektionsverfahren können alle diese Gene in der Nukleinsäure von Interesse eines Minicircle-Transfervektors enthalten sein. Daher umfasst ein Aspekt einen Minicircle-Transfervektor, der eine Transfersequenz, die Gene rep und cap von AAV sowie die adenoviralen Gene VA, E2A und E4 enthält.

In einem anderen Aspekt kann aber auch ein Doppel-Transfektionsverfahren verwendet werden, bei dem die genannten Gene auf einem Verpackungsvektor enthalten sind, der von dem Minicircle-Transfervektor verschieden ist. Zur Produktion der viralen Vektoren ist dann eine Kotransfektion der Producerzellen mit Transfervektor und Verpackungsvektor erforderlich. Bei dem Verpackungsvektor kann es sich selbst um einen Minicircle handeln. In einem anderen Aspekt kann auch ein Tripel-Transfektionsverfahren verwendet werden, bei dem die für die Verpackung notwendigen Gene auf zwei verschiedenen Verpackungsvektoren kodiert sind, bei denen es sich jeweils wieder um Minicircles handeln kann. Beispielsweise können die Gene rep und cap auf einem ersten Verpackungsvektor und die Gene VA, E2A und E4 auf einem zweiten Verpackungsvektor enthalten sein.

In einem weiteren Aspekt sind daher auch Verpackungsvektoren umfasst, die als Minicircles ausgebildet sind ("Minicircle-Verpackungsvektoren"). Die Nukleinsäure von Interesse in solchen Minicircles umfasst mindestens eine Verpackungs-Expressionskassette, auf der mindestens ein Protein kodiert ist und exprimiert werden kann, das für die Verpackung einer Transfersequenz in Partikel eines viralen Vektors notwendig ist. Ein Minicircle-Verpackungsvektor kann für sämtliche Proteine kodieren, die für die Verpackung der Transfersequenz notwendig sind, oder nur einige davon.

In einem Aspekt umfasst die Erfindung einen Minicircle-Verpackungsvektor, der die Gene rep und cap aus AAV enthält. In einem weiteren Aspekt umfasst die Erfindung einen Minicircle-Verpackungsvektor, der die adenoviralen Gene VA, E2A und E4 enthält. In einem weiteren Aspekt umfasst die Erfindung einen Minicircle-Verpackungsvektor, der die Gene rep und cap aus AAV und die adenoviralen Gene VA, E2A und E4 enthält.

Minicircle-Verpackungsvektoren können zusammen mit Minicircle-Transfervektoren oder auch mit anderen Transfervektoren, die nicht Minicircles sind, eingesetzt werden, um die Verpackung einer Transfersequenz in virale Partikel zu erreichen.

Monomere Minicircles gemäß der vorliegenden Erfindung können sehr unterschiedliche Größen haben, zum Beispiel zwischen 500 und 30000 bp, wie etwa 500 bp, 1000 bp, 1500 bp, 2000 bp, 2500 bp, 3000 bp, 4000 bp, 5000 bp, 6000 bp, 8000 bp, 10000 bp, 12000 bp, 15000 bp, 20000 bp, 25000 bp oder 30000 bp. Bevorzugt sind für Minicircle-Transfervektoren Größen zwischen 1000 bp und 6000 bp. In Minicircle-Verpackungsvektoren kann eine höhere Größe notwendig sein. Beispielsweise hat der Minicircle-Verpackungsvektor MC.DP2rs aus Beispiel 4 eine Größe von 21870 bp.

Minicircle DNS kann darüber hinaus auch in di- oder multimerer Form auftreten, z. B. zwischen 100 und 100000 bp. Beispielsweise hat ein Dimer des Minicircles MC.DP2rs eine Größe von 43740 bp (s. auch FIG. 23).

### Vorzugsweise sind die Minicircles superspiralisiert.

In einem weiteren Aspekt haben hierin offenbarte Minicircles die zusätzliche Eigenschaft, dass sie episomal stabil in der Producerzelle verbleiben können und so - ohne Integration in das zelluläre Chromosom - die erworbene Eigenschaft zur Herstellung von Viren oder viralen Vektoren länger bestehen bleibt, als dies bei klassischen transienten Transfektionen zu beobachten ist. Die Nicht-Integration in das Genom der Zelle hat den Vorteil, dass die chromosomale Struktur der Producerzelle - anders als bei Integration (s. z.B. Hendrie und Russell, Mol. Ther. 2005, 12:9-17) - intakt bleibt und selbst bei nicht-mutagener Insertion die auf dem Minicircle kodierten Sequenzen nicht herunterreguliert werden. Episomale Transfektion kann beispielsweise durch eine S/MAR-Sequenz zur episomalen Stabilisierung verwirklicht werden (s. z.B. Haase et al., BMC Biotechnol. 2010, 10:20). Die Transfektion einer Zelle, in der sich bereits ein episomal stabilisiertes Helfer- und/oder Verpackungsplasmid befindet, durch ein Transferplasmid führt dann ebenfalls zur Produktion von viralen Vektoren, selbst wenn zwischen den beiden Transfektionen sehr lange Zeit vergeht oder sogar viele Generationen der Zellteilung liegen.

### HERSTELLUNG VON MINICIRCLES

Die Minicircles der Erfindung können mittels Rekombination aus entsprechenden Plasmiden hergestellt werden, wie in WO 96/26270 oder EP 1620559 beschrieben. Hierbei geht man von einem Plasmid ("Parentalplasmid") aus, das die komplette Sequenz des gewünschten Minicircles (Minicircle-Region) und zusätzlich ein Plasmidrückgrat (Miniplasmid-Region) enthält (siehe FIG. 2, FIG.7, FIG. 10). Zwischen der Minicircle-Region und der Miniplasmid-Region befinden sich beiderseits spezifische Rekombinase-Erkennungssequenzen, die von einer entsprechenden Rekombinase erkannt werden können. Eine Rekombinase ist hierbei jedes Enzym, das die spezifische Rekombination des Parentalplasmids zu einem Minicircle und einem verbleibenden Rest, dem Miniplasmid, katalysieren kann. Hierbei müssen die beiden Rekombinase-Erkennungssequenzen so orientiert sein, dass bei der Rekombination die Trennung in Minicircle und Miniplasmid erfolgt und nicht ein einzelnes modifiziertes Plasmid entsteht.

Die Rekombinase-Erkennungssequenzen können sich unmittelbar an die Nukleinsäuresequenz von Interesse anschließen, oder die Sequenzen können durch eine Stuffersequenz getrennt sein. Stuffersequenzen können zu beiden Seiten der Nukleinsäuresequenz von Interesse angebracht sein, oder nur an einer Seite. Die Stuffersequenzen haben vorzugsweise eine Länge von 1-1000 bp, z.B. 1, 10, 20, 50, 100, 200, 300, 400, 500, 600, 700, 800, 900 oder 1000 bp. Wenn zu beiden Seiten der Nukleinsäuresequenz von Interesse Stuffersequenzen vorliegen, können diese gleiche oder unterschiedliche Längen haben. Insbesondere kann eine solche Stuffersequenz auch eine oder mehrere Identifikationssequenzen wie unten beschrieben umfassen.

Beispiele für mögliche Rekombinasen sind die Integrasen aus den Bakteriophagen λ (siehe A. Landy et al., Science 1977, 197(4309):1147-1160), P22 und Φ80 (siehe J. M. Leong et al., J. Biol. Chem. 1985, 260(7):4468-4470), die HP1-Integrase aus *Haemophilus influenzae* (siehe M. A. Hauser et al., J. Biol. Chem. 1992, 267(10):6859-6864), die Cre-Integrase aus dem Phagen P1, die Integrase aus dem Plasmid pSAM2 (siehe EP350341), die Flp-Rekombinase aus dem Plasmid 2µ oder die ΦC31-Integrase. Alternativ können auch Rekombinasen aus Transposons der Tn3-Familie verwendet werden, wie die parA-Resolvase aus RP4 (siehe L. Eberl et al., Mol. Microbiol. 1994, 12(1):131-141), die Resolvasen der Transposons Tn3, Tn21 oder Tn522 (siehe W. M. Stark et al., Trends Genet. 1992, 8(12):432-439), oder die Gin-Invertase aus dem Bakteriophagen µ.

Eine beispielhafte Anordnung der Nukleinsäuresequenz von Interesse auf einem erfindungsgemäßen Parentalplasmid (PP-DP2rs) ist in FIG. 2 abgebildet. Die "rec"-Sequenzen werden hierbei zur Herstellung eines Minicircle-Verpackungsvektors für AAV-Partikel genutzt. Die Nukleinsäuresequenz von Interesse wird durch die rec-Sequenzen beschränkt und enthält Verpackungs-Expressionskassetten mit den Genen rep, cap, VA, E2A, E3 und E4.

Erfindungsgemäße Parentalplasmide können durch dem Fachmann bekannte mikrobiologische Strategien und Methoden hergestellt werden. Als Ausgangspunkt kann beispielsweise das Plasmid pDP2rs (PlasmidFactory, Bielefeld, DE, Art.-Nr. PF402; siehe FIG. 1, SEQ ID NO:1) dienen, das notwendige Verpackungsgene zur AAV-Herstellung enthält. Die dafür relevanten Sequenzen befinden sich zwischen den Restriktionsschnittstellen für Pacl. Ein DNS-Fragment kann mit diesem Enzym herausgeschnitten werden, wenn innerhalb der Nukleinsäuresequenz von Interesse keine weitere PacI-Schnittstelle vorliegt. Alternativ kann ein solches Fragment auch durch Amplifikation mittels PCR ausgehend von Primern, die außerhalb des aber nahe zum gewünschten Sequenzfragment liegen, gewonnen werden.

Das erhaltene Restriktionsfragment, das die Nukleinsäuresequenz von Interesse umfasst, kann nun in ein Vorläuferplasmid kloniert werden, das den Miniplasmid-Bereich sowie die Rekombinase-Erkennungssequenzen des gewünschten Parentalplasmids enthält. Falls erforderlich, kann das Fragment auch zunächst in ein weiteres Plasmid umkloniert werden, um ein mit dem Vorläuferplasmid kompatibles Restriktionsenzym verwenden zu können. Die Klonierung eines PCR-Fragments kann entweder auf die gleiche Weise oder über einen Shuttle-Vektor zur Klonierung von PCR-Fragmenten erfolgen. In allen Fällen muss sich die Insertionsstelle in dem Gebiet zwischen den Rekombinase-Erkennungssequenzen des Vorläuferplasmids befinden. Dadurch entsteht das zur Bildung von Minicircle und Miniplasmid notwendige Parentalplasmid. Die Klonierung erfolgt durch Einfügen des PacI-Fragments in eine PacI-Schnittstelle des Vorläuferplasmids.

Dieses "Einfügen" kann mittels klassischer Ligation geeigneter DNS-Enden (stumpf oder "klebrig") erfolgen oder auch per Rekombination (wie in Hartley et al., Genome Research 2000, 10:1788-1795) beschrieben, wobei darauf zu achten ist, dass die im zuletzt genannten Fall verwendeten Rekombinationsmechanismen nicht störend mit denen der Spaltung in Minicircle und Miniplasmid interagieren.

Selbstverständlich kann ein geeignetes Parentalplasmid auch auf der Grundlage anderer Plasmide als der genannten konstruiert werden. Die verwendeten Restriktionsenzyme können ebenfalls andere sein. Entscheidend ist die Struktur des entstehenden Parentalplasmids, das eine oder mehrere Nukleinsäuresequenzen von Interesse zwischen zwei geeignet orientierten Rekombinase-Erkennungssequenzen enthalten muss.

Die Rekombination des Parentalplasmids kann *in vitro* oder *in vivo* erfolgen. Ein Vorteil der *in vivo-*Rekombination in Wirtszellen ist, dass das Parentalplasmid nicht aufgereinigt zu werden braucht, sofern die Expression der entsprechenden Rekombinase in den Wirtszellen induziert werden kann. Das hierfür notwendige Gen, das für die Rekombinase kodiert, kann in der Wirtszelle auf einem anderen Nukleinsäuremolekül vorliegen. Besonders vorteilhaft ist es, wenn das Rekombinase-Gen auf dem Parentalplasmid innerhalb der Miniplasmid-Region vorliegt. Vorzugsweise ist die Expression der Rekombinase induzierbar, zum Beispiel über eine Temperaturänderung oder Zugabe eines Metaboliten. Nach Induktion der Rekombinase-Expression in der Wirtszelle wird das Parentalplasmid in zwei superspiralisierte, zirkuläre Moleküle, den Minicircle und das Miniplasmid gespalten, die nach Zelllyse voneinander getrennt werden können oder bereits in der Zelle die auf der Nukleinsäuresequenz von Interesse kodierte Information realisieren.

Wirtszelle kann somit auch die Producerzelle sein, wobei dafür die Expressionssteuerung der Rekombinase in eukaryotischen Zellen gewährleistet sein muss.

Alternativ kann ein Minicircle auch durch herkömmliche Restriktions- und Ligationstechniken aus einem Plasmid hergestellt werden. Hierbei wird der Bereich des Plasmids, der den Minicircle bilden soll, zum Beispiel mittels eines Restriktionsenzyms aus dem Plasmid herausgeschnitten und zu einem Minicircle ligiert. Der Minicircle kann auch aus mehreren getrennten Abschnitten zusammengesetzt werden. Zu beachten ist hierbei jedoch, dass ein so hergestellter Minicircle im Allgemeinen nicht superhelikal ist, sofern er nicht nachträglich *in vitro* superspiralisiert wird.

In einem Aspekt kann der Minicircle weiterhin mindestens eine Identifikationssequenz zur Aufreinigung des Minicircles, aber auch zu anderen Zwecken enthalten. Als Identifikationssequenz kann jede Sequenz dienen, die die Trennung der Minicircles von den Miniplasmiden ermöglicht. Insbesondere kann es sich dabei um Sequenzen handeln, die an einen spezifischen Liganden binden und so den Minicircle mit dem Liganden komplexieren können. Bei dem Liganden kann es sich um ein DNS-bindendes Protein oder um eine Nukleinsäure handeln. Die Aufreinigung kann anschließend z. B. über chromatographische Verfahren, insbesondere gegen die Identifikationssequenz gerichtete Affinitätschromatographie unter Verwendung des immobilisierten Liganden, vorgenommen werden. Geeignete Aufreinigungssysteme umfassen z.B. die Tripelhelix-Affinitätschromatographie (THAC; siehe P. Wils et al., Gene Therapy 1997, 4(4):323-330), das lacO/lacI-System (siehe J. Lundeberg et al, Genet. Anal. Techn. Appl. 1990, 7:47-52), das repU/dso-System (siehe A. Müller et al., Nucleic Acids Research 1995, 23(11):1894-1900) oder Systeme aus dem Repressor des Bakteriophagen λ oder des Bakteriophagen 434 und dem entsprechenden Promotor, für welchen der jeweilige Repressor spezifisch ist.

Besonders bevorzugt sind Aspekte, in denen sich die Identifikationssequenz im Minicirclebereich des Parentalplasmids, oder innerhalb der Sequenz von Interesse befindet. Findet in einem solchen Minicircle-Bereich eines Parentalplasmids (oder seinen Ursprungs- oder Vorläuferplasmiden) während des Herstellungsprozesses eine spontane und unbeabsichtigte Rekombination der Rekombinase-Erkennungssequenzen statt, entsteht ein defektes Produkt, in dem neben der Nukleinsäuresequenz von Interesse auch die Identifikationssequenz deletiert ist. Durch gegen die Identifikationssequenz gerichtete Affinitätsaufreinigung werden somit nur intakte Minicircles isoliert. Dadurch wird das Problem der Verunreinigung von Minicircle-Präparationen durch defekte Deletionsprodukte vermieden.

Ein Minicircle kann auch mehr als eine Identifikationssequenz enthalten. Hierbei können die Identifikationssequenzen identisch sein und als direkte Wiederholungen oder durch einen Spacer getrennte Wiederholungen vorliegen. Es können aber auch zwei oder mehr verschiedene Identifikationssequenzen zu unterschiedlichen Zwecken verwendet werden. Beispielsweise kann eine Sequenz, die mit einem Oligonukleotid-Liganden eine Tripelhelix ausbilden kann, außerhalb der Nukleinsäuresequenz von Interesse vorliegen, um die Minicircles vor der Transfektion aufzureinigen, und zugleich eine lacOs-Sequenz innerhalb der Nukleinsäuresequenz von Interesse enthalten sein, um nach der Transfektion die Anwesenheit in der Zelle nachzuweisen oder ihre Effizienz zu messen.

Veranschaulicht am Parentalplasmid PP-DP2rs aus FIG. 2 ist der Bereich innerhalb der rec-Sequenzen, in dem auch die Verpackungs-Expressionskassetten liegen, geeignet, um an den Rändern des Bereichs oder zwischen den darauf befindlichen Verpackungs-Expressionskassetten eine oder mehrere Identifikationssequenzen aufzunehmen, mit denen ein Minicircle identifiziert und vom bei der Herstellung entstehenden Miniplasmid getrennt werden soll.

### ZELLEN

Unter einem anderen Gesichtspunkt betrifft die Erfindung auch eine Zelle, die einen Minicircle gemäß der Erfindung enthält. Hierbei kann es sich um eine Zelle aus einer beliebigen Spezies handeln. Insbesondere sind prokaryotische und eukaryotische Zellen, die Minicircles gemäß der Erfindung enthalten, von der Erfindung umfasst. In manchen Ausführungsformen ist die Zelle eine Wirbeltierzelle, bevorzugt eine menschliche Zelle. In manchen Aspekten ist die Zelle eine Producerzelle für virale Vektoren, beispielsweise eine HEK 293-, oder HeLa-Zelle.

Zellen, die einen Minicircle gemäß der Erfindung enthalten, können durch herkömmliche Transfektionsverfahren hergestellt werden, die dem Fachmann bekannt sind. Verwendet werden kann beispielsweise die chemische Transfektion mittels Calciumphosphat (siehe F. L. Graham et al., Virology 1973, 52(2):456-467), mittels Dendrimeren (seihe H. L. Fu et al., Journal of Control Release 2007, 124(3):181-188) oder mittels kationischen Polymeren (siehe EP 1505089). Weitere Methoden sind Lipofektion (siehe P. L. Felgner et al., P.N.A.S. 1987, 84(21):7413-7417), Elektroporation (siehe E. Neumann et al., EMBO J. 1982, 1(7):841-845), optische Transfektion (siehe M. Tsukakoshi et al., Applied Physics B-Photophysics and Laser Chemistry 1984, 35(3):135-140), Magnetofektion (siehe F. Scherer et al., Gene Ther. 2009, 9(2):102-109) oder Impalefektion (siehe T. E. McKnight et al., Nano Letters 2004, 4(7):1213-1219). Auch können partikelbasierte Verfahren wie die Genkanone angewendet werden (siehe US 5,219,746). Bevorzugte Verfahren sind die Calciumphosphat-Transfektion, die Lipofektion und die Elektroporation.

Des Weiteren kann ein Minicircle gemäß der Erfindung direkt in der Zielzelle hergestellt werden, wie schon oben erwähnt. Hierbei wird die Zielzelle zunächst mit einem Parentalplasmid des Minicircles transfiziert und anschließend die Expression der entsprechenden Rekombinase in der Zielzelle veranlasst, so dass die Prozessierung des Parentalplasmids mittels Rekombination zu Minicircle und Miniplasmid ermöglicht wird. Hierfür muss die Expressionssteuerung der Rekombinase in eukaryotischen Zellen gewährleistet sein, und ein für die Rekombinase kodierendes Gen muss in der Zielzelle vorliegen. Das Rekombinase-Gen kann sowohl in die genomische DNS der Zielzelle integriert sein oder auch auf einem anderen Nukleinsäuremolekül vorliegen. Besonders vorteilhaft ist es, wenn das Rekombinase-Gen auf dem Parentalplasmid innerhalb der Miniplasmid-Region vorliegt.

### VERFAHREN ZUR HERSTELLUNG VIRALER VEKTOREN

Virale Vektoren werden vorzugsweise durch transiente Transfektion, aber auch durch stabile Transfektion, von eukaryotischen Producerzellen mit DNS hergestellt. Dabei werden die DNS-Moleküle gleichzeitig oder nacheinander in die Zelle verbracht und die darauf befindliche Information zur zellulären Virusproduktion aktiviert. Die DNS-Moleküle stellen alle notwendigen Komponenten zur Herstellung viraler Partikel in der Wirtszelle her und fügen sie zu viralen Partikeln zusammen.

Typische virale Vektoren sind HSV (Herpes-simplex-Virus), Ad (Adenovirus), die bereits angesprochenen AAV und Lentiviren (LV) sowie Retroviren (RV). Die beiden zuletzt genannten Viren integrieren in das Genom der Wirtszelle, während die anderen genannten episomal oder zumindest vorwiegend episomal in der Zelle vorliegen. Sie haben unterschiedliche Verpackungskapazitäten: RV und LV ca. 7-8 kbp, Ad ca. 8 kbp (bzw. in den besonderen Varianten HC/gutless 30 kbp) und HSV ca. 150 kbp (amplicon) oder 40 kbp (replikationsdefekt).

### Herstellung Adeno-assoziierter Viren (AAV):

Nachfolgend wird ein bevorzugtes Verfahren zur Herstellung Adeno-Assoziierter Viren dargestellt.

Durch Kultivierung der zu transfizierenden Producerzellen werden ausreichende Mengen dieser Zellen bereitgestellt. Anschließend werden die Producerzellen mit den notwendigen Transfer- und Verpackungsvektoren transfiziert. Für jeden dieser Vektoren kann ein Minicircle verwendet werden. In Frage kommt beispielsweise die chemische Transfektion mittels Calciumphosphat (siehe F. L. Graham et al., Virology 1973, 52(2):456-467), mittels Dendrimeren (seihe H. L. Fu et al., Journal of Control Release 2007, 124(3):181-188) oder mittels kationischen Polymeren (siehe EP 1505089). Weitere Methoden sind Lipofektion (siehe P. L. Felgner et al., P.N.A.S. 1987, 84(21):7413-7417), Elektroporation (siehe E. Neumann et al., EMBO J. 1982, 1(7):841-845), optische Transfektion (siehe M. Tsukakoshi et al., Applied Physics B-Photophysics and Laser Chemistry 1984, 35(3):135-140), Magnetofektion (siehe F. Scherer et al., Gene Ther. 2009, 9(2):102-109) oder Impalefektion (siehe T. E. McKnight et al., Nano Letters 2004, 4(7):1213-1219). Auch können partikelbasierte Verfahren wie die Genkanone angewendet werden (siehe US 5,219,746). Bevorzugte Verfahren sind die Calciumphosphat-Transfektion, die Lipofektion und die Elektroporation.

Hierbei ist es wichtig, dass sowohl die rep- und cap-Gene von AAV als auch die adenoviralen Helfersequenzen VA, E2A und E4 transfiziert werden. Wie oben bereits beschrieben, werden hierzu typischerweise Mono- (Transfersequenz und alle Verpackungssequenzen auf demselben Vektor), Doppel- (Transfersequenz und Verpackungssequenzen auf verschiedenen Vektoren) oder Tripel-Transfektionssysteme (Verpackungssequenzen auf zwei vom Transfervektor verschiedene Vektoren verteilt) verwendet. Sollen mehrere Vektoren transfiziert werden, kann dies in Form einer Kotransfektion geschehen. In diesem Fall sollte darauf geachtet werden, dass alle Vektoren in äquimolaren Mengen vorliegen.

Alternativ kann bei einer Mehrfach-Transfektionsstrategie der zuerst transfizierte Vektor in den Producerzellen episomal stabilisiert werden. Typischerweise wird es sich hierbei um den Verpackungsvektor handeln. In einem solchen Fall kann die zweite Transfektion Tage, Wochen oder Monate nach der ersten Transfektion stattfinden, selbst wenn die ursprünglich transfizierten Zellen sich schon etliche Male geteilt haben.

Die Ernte der Virenpartikel erfolgt typischerweise 2-3 Tage nach der letzten Transfektion. Die Producerzellen werden hierzu abzentrifugiert, lysiert und durch wiederholtes Einfrieren in flüssigem Stickstoff und Auftauen aufgeschlossen. Zelluläre DNS und RNS sowie noch vorhandene Plasmid-DNS wird durch eine Benzonase-Behandlung entfernt. Die Abtrennung von zellulären Proteinen erfolgt über eine Iodixanolgradientenzentrifugation. Anschließend wird die Viruspräparation durch eine Affinitätschromatographie oder Gelfiltration von Iodixanol getrennt und weiter gereinigt.

### Herstellung von lentiviralen Vektoren (LV):

Auch bei der Herstellung von lentiviralen Vektoren wird neben dem Transfervektor weitere DNS kotransfiziert. Dabei handelt es sich um Plasmide mit Sequenzen für die Helfer- und Verpackungsinformationen (bei einer Tripeltransfektion wird so neben dem Transferplasmid auch noch eines mit gag und pol eingesetzt sowie eines mit env). Die gag und pol-Sequenzen sind z.B. auf den Verpackungsvektoren pCMVdeltaR8.9 (s. R. Zufferey et al., Nature Biotechnol. 1997, 15(9):871-875) oder pHR'(s. H. Miyoshi et al., PNAS 1997, 94(19):10319-10323) positioniert und kodieren ein gag-pol-Vorläuferprotein, welches die Strukturproteine des Vektorpartikels enthält. Die env-Sequenzen z.B. auf den Plasmiden pCG-Fcdelta30 oder pCG-H-alphaCD20 kodieren ein Glykoprotein, welches die Zellbindung und den Zelleintritt ermöglicht. Die Herstellung der viralen Partikel wird in S. Funke et al. (Mol. Therapy 2008, 16:1427-1436) sowie in C. J. Buchholz et al. (Trend Biotechnol. 2009, 27(5):259-265) ausführlich beschrieben.

Die Durchführung einer Pseudotypisierung (Austausch der viralen Hüllproteine durch artfremde Hüllproteine, um z.B. den Tropismus - also die Zielerkennung beim Infizieren einer Wirtszelle - zu verändern) ist für diese viralen Partikel ebenfalls möglich. Dafür werden die env-tragenden Verpackungsvektoren durch solche ersetzt, die andere Glycoproteine kodieren. So wird z.B. das Glykoprotein G des Vesikuläre-Stomatitis-Virus (VSV-G) eingesetzt, welches die Transduktion nahezu aller Zelltypen ermöglicht. Ein Beispiel dafür bietet das Plasmid pMD.G oder pMD.2G (Addgene-Nr. 12259) oder pHIT123 (Y. Soneoka et al., Nucleic Acids Res. 1995, 23:628-633), welches für das env-Protein des Moloney-murine-Leukämie-Virus (MoMLV) kodiert.

### KITS

Des Weiteren sind auch Kits zur Herstellung viraler Vektoren in einer Producerzelle offenbart. Ein Kit umfasst hierbei einen Transfervektor und mindestens einen Verpackungsvektor. Mindestens einer dieser Vektoren ist ein erfindungsgemäßer Minicircle (Minicircle-Transfervektor und/oder Minicircle-Verpackungsvektor). In einer besonderen Ausführungsform sind die herzustellenden viralen Vektoren AAV-Vektoren oder retrovirale Vektoren, wie z.B. lentivirale Vektoren.

Der Transfervektor und der mindestens eine Verpackungsvektor können in dem Kit trocken, z.B. lyophilisiert, vorliegen. Alternativ können sie auch in einem Puffer gelöst und die Lösung flüssig oder gefroren sein. Des Weiteren können sie gemischt oder voneinander getrennt in verschiedenen Behältern vorhanden sein. Zusätzlich zu den Transfer- und Verpackungsvektoren kann der Kit auch weitere Bestandteile enthalten, die zur Ausführung eines Verfahrens gemäß der Erfindung nützlich sind, wie etwa Chemikalien, Reagenzien, Puffer, Lösungsmittel oder Medien für die Ausführung der Verfahren der Erfindung. Insbesondere kann der Kit Reagenzien enthalten, die für eine chemische Transfektion oder eine Lipofektion notwendig sind. Manche oder alle der Reagenzien können in abgemessenen Einheitsmengen vorliegen, z.B. um den Pipettieraufwand des Nutzers auf ein Mindestmaß zu beschränken.

Zusätzlich kann der Kit auch Beschreibungen des Transfervektors und/oder des mindestens einen Verpackungsvektors, wie etwa Vektorkarten oder Sequenzen enthalten. Ebenso können Anweisungen zur Durchführung der betreffenden Ausführungsform der Erfindung Bestandteil des Kits sein.

### Beispiele

### BEISPIEL 1:

### Konstruktion eines Parentalplasmids (PP.DP2rs) für Minicircles umfassend die helper/packaging Sequenzen aus pDP2rs

Das Plasmid **pDP2rs** (23677 bp, Artikel-Nr. PF402, PlasmidFactory, Bielefeld, DE), das mehrere Expressionskassetten für die *AAV-he*/*per*/*packaging-*Funktionen und speziell das cap-Protein des Serotyps 2 sowie ein Gen für rot fluoreszierendes Protein (RFP) enthält (**FIG. 1**, SEQ ID NO:1), wird als Ausgangsmaterial zur gezielten Extraktion desjenigen Abschnitts verwendet, auf dem die o.g. genetischen Eigenschaften liegen. Dabei wird durch Restriktionsverdau mit Pacl (Artikel Nr. R0547L, NEB, Frankfurt, DE) ein ca. 21,5 kb großes DNS-Fragment und ein (unerwünschtes) ca. 2 kb großes DNS-Fragement erzeugt. Das 21.5 kb Fragment wird mittels Agarosegelelektrophorese, Gelextraktion und DNS-Extraktion (Macherey-Nagel, Düren, DE) aufgereinigt.

Das Vorläuferplasmid pP11, das zur Aufnahme des 21,5 kb-Fragments dient (siehe Mayrhofer et al., J. Gene Med. 2008, 10(11):1253-1269, nun jedoch ohne MCS, ohne Spacer und mit einer statt zwei Identifikationssequenzen, wie in DE 10 2011 118 018 beschrieben), enthält eine Pacl-Restriktionsschnittstelle, die von Rekombinase-Erkennungssequenzen der parA-Resolvase flankiert ist und eine Expressionskassette der parA-Resolvase außerhalb der Rekombinase-Erkennungssequenzen. Dieses Plasmid wird mit dem Enzym Pacl (Artikel Nr. R0547L, NEB, Frankfurt, DE) geschnitten und mit Alkalischer Phosphatase (Artikel Nr. M0290L, NEB, Frankfurt, DE) dephosphoryliert. Anschließend wird das Fragment mit T4-Ligase in den linearisierten Vektor ligiert, um **PP.DP2rs** (**FIG. 2**) zu erhalten.

### BEISPIEL 2:

### Konstruktion eines Parentalplasmids (PP.DG) für Minicircles umfassend die helper/packaging Sequenzen aus pDG

Das Plasmid **pDG** (21849 bp, Artikel-Nr. PF421, PlasmidFactory, Bielefeld, DE), das mehrere Expressionskassetten für die AAV-*helper*/*packaging-*Funktionen und speziell das cap-Protein des Serotyps 2, jedoch kein Gen für rot floureszierendes Protein (RFP) enthält (**FIG. 6**, SEQ ID NO:2), wird als Ausgangsmaterial zur gezielten Extraktion desjenigen Abschnitts verwendet, auf dem die o.g. genetischen Eigenschaften liegen. Dabei wird durch Restriktionsverdau mit Pacl (Artikel Nr. R0547L, NEB, Frankfurt, DE) ein ca. 20 kb großes DNS-Fragment und ein (unerwünschtes) ca. 2 kb großes DNS-Fragement erzeugt. Das 20 kb Fragment wird mittels Agarosegelelektrophorese, Gelextraktion und DNS-Extraktion (Macherey-Nagel, Düren, DE) aufgereinigt.

Das Vorläuferplasmid pP11, das zur Aufnahme des 20 kb-Fragments dient (siehe Mayrhofer et al., J. Gene Med. 2008, 10(11):1253-1269, nun jedoch ohne MCS, ohne Spacer und mit einer statt zwei Identifikationssequenzen, wie in DE 10 2011 118 018 beschrieben), enthält eine Pacl-Restriktionsschnittstelle, die von Rekombinase-Erkennungssequenzen der parA-Resolvase flankiert ist und eine Expressionskassette der parA-Resolvase außerhalb der Rekombinase-Erkennungssequenzen. Dieses Plasmid wird mit dem Enzym Pacl (Artikel Nr. R0547L, NEB, Frankfurt, DE) geschnitten und mit Alkalischer Phosphatase (Artikel Nr. M0290L, NEB, Frankfurt, DE) dephosphoryliert. Anschließend wird das Fragment mit T4-Ligase in den linearisierten Vektor ligiert, um **PP.DG** (**FIG. 7**) zu erhalten.

### BEISPIEL 3:

### Konstruktion eines Parentalplasmids (PP.ssGFP) für Minicircles umfassend die Transferplasmid-Sequenzen aus pssGFP

Das Plasmid **pssGFP** (7905 bp, H. Büning, Univ. Köln, DE), das Expressionskassetten für eGFP unter der Kontrolle des CMV-Promoters und für Hygromycin unter der Kontrolle eines TK-Promoters enthält (**FIG. 9A****,** SEQ ID NO:3), wird als Ausgangsmaterial zur gezielten Extraktion desjenigen Abschnitts verwendet, auf dem die o.g. genetischen Eigenschaften liegen. Dabei wird durch Restriktionsverdau mit PvuII (Artikel Nr. R0151M, NEB, Frankfurt, DE) ein ca. 4,3 kb großes DNS-Fragment und ein (unerwünschtes) ca. 3,6 kb großes DNS-Fragement erzeugt. Das 4,3 kb-Fragment enthält an seinen beiden Enden jeweils eine ITR-Sequenz - das Verpackungssignal zur Verpackung der flankierten Sequenz im Zuge der AAV-Genese in Zellen (5'-CGCGCTCGCTCGCTCACTGAGGCCGCCCGGGCAAAGCCCGGGCGTCGGGCGA CCTTTGGTCGCCCGGCCTCAGTGAGCGAGCGAGCGCGCAGAGAGGGAGTGGCCA ACTCCATCACTAGGGGTTCCT-3',SEQ ID NO:7; und 5'-AGGAACCCCTAGTGAT GGAGTTGGCCACTCCCTCTCTGCGCGCTCGCTCGCTCACTGAGGCCGGGCGACCA AAGGTCGCCCGACGCCCGGGCTTTGCCCGGGCGGCCTCAGTGAGCGAGCGAG-3', SEQ ID NO:8) - und wird mittels Agarosegelelektrophorese, Gelextraktion und DNS-Extraktion (Macherey-Nagel, Düren, DE) aufgereinigt.

Das Vorläuferplasmid pP11, das zur Aufnahme des 4,3 kb-Fragments dient (siehe Mayrhofer et al., J. Gene Med. 2008, 10(11):1253-1269, nun jedoch ohne MCS, ohne Spacer und mit einer statt zwei Identifikationssequenzen, wie in DE 10 2011 118 018 beschrieben), enthält eine Pmel Restriktionsschnittstelle, die von Rekombinase-Erkennungssequenzen der parA-Resolvase flankiert ist und eine Expressionskassette der parA-Resolvase außerhalb der Rekombinase-Erkennungssequenzen. Dieses Plasmid wird mit dem Enzym Pmel (Artikel Nr. R0560L, NEB, Frankfurt, DE) geschnitten und mit Alkalischer Phosphatase (Artikel Nr. M0290L, NEB, Frankfurt, DE) dephosphoryliert. Anschließend wird das Fragment mit T4-Ligase in den linearisierten Vektor ligiert, um das Plasmid **PP.ssGFP** (**FIG. 10**) zu erhalten.

### BEISPIEL 4:

### Konstruktion eines Parentalplasmids (PP.scGFP) für Minicircles umfassend die Transferplasmid-Sequenzen aus pscGFP zur Erzeugung selbstkomplementärer AAV-Vektoren

Das Plasmid **pscGFP** (5964 bp, H. Büning, Univ. Köln, DE), das Expressionskassetten für eGFP unter der Kontrolle des CMV-Promoters (**FIG. 12****,** SEQ ID NO:4) enthält, wird als Ausgangsmaterial zur gezielten Extraktion desjenigen Abschnitts verwendet, auf dem die o.g. genetischen Eigenschaften liegen. Dabei wird durch Restriktionsverdau mit PvuII (Artikel Nr. R0151M, NEB, Frankfurt, DE) ein ca. 2,3 kb großes DNS-Fragment und ein (unerwünschtes) ca. 3,6 kb großes DNS-Fragment erzeugt. Das 2,3 kb-Fragment enthält an seinen beiden Enden jeweils eine ITR-Sequenz - das Verpackungssignal zur Verpackung der flankierten Sequenz im Zuge der AAV-Genese in Zellen (5'-CGCGCTCGCTCGCTCACTGAGGCCGCCCGGGCAA AGCCCGGGCGTCGGGCGACCTTTGGTCGCCCGGCCTCAGTGAGCGAGCGAGCGC GCAGAGAGGGAGTGG-3', SEQ ID NO:9 und 5'-AGGAACCCCTAGTGATGGAGTTG GCCACTCCCTCTCTGCGCGCTCGCTCGCTCACTGAGGCCGGGCGACCAAAGGTCCC GACGCCCGGGCTTTGCCCGGGCGGCCTCAGTGAGCGAGCGAG-3', SEQ ID NO:8). Die Sequenz von SEQ ID NO:8 stellt hierbei eine mutierte ITR dar, die die Herstellung selbstkomplementärer Vektoren ermöglicht, wie oben beschrieben. Das Fragment wird mittels Agarosegelelektrophorese, Gelextraktion und DNS-Extraktion (Macherey-Nagel, Düren, DE) aufgereinigt.

Das Vorläuferplasmid pP11, das zur Aufnahme des 2,3 kb-Fragments dient (siehe Mayrhofer et al., J. Gene Med. 2008, 10(11):1253-1269, nun jedoch ohne MCS, ohne Spacer und mit einer statt zwei Identifikationssequenzen, wie in DE 10 2011 118 018 beschrieben), enthält eine Pmel Restriktionsschnittstelle, die von Rekombinase-Erkennungssequenzen der parA-Resolvase flankiert ist und eine Expressionskassette der parA-Resolvase außerhalb der Rekombinase-Erkennungssequenzen. Dieses Plasmid wird mit dem Enzym Pmel (Artikel Nr. R0560L, NEB, Frankfurt, DE) geschnitten und mit Alkalischer Phosphatase (Artikel Nr. M0290L, NEB, Frankfurt, DE) dephosphoryliert. Anschließend wird das Fragment mit T4-Ligase in den linearisierten Vektor ligiert, um das Plasmid **PP.scGFP** (**FIG. 13**) zu erhalten.

### BEISPIEL 5:

### Konstruktion eines Parentalplasmids (PP.DM) für Minicircles umfassend die helper/packaging Sequenzen aus pDG und eine ITR-flankierte Transfersequenz zur Erzeugung von AAV-Partikeln durch Transfektion mit nur einem DNS-Vektor

Das Plasmid **pDM** (21529 bp, Artikel-Nr. PF400, PlasmidFactory, Bielefeld, DE), das mehrere Expressionskassetten für die *AAV-he*/*per*/*packaging-*Funktionen und speziell das cap- des Serotyps 2 und zwei rep-Proteine (als Cluster flankiert von ITR-Sequenzen aus AAV) enthält (**FIG. 15****,** SEQ ID NO:5) wird als Ausgangsmaterial zur gezielten Extraktion desjenigen Abschnitts verwendet, auf dem alle die o.g. genetischen Eigenschaften liegen. Dabei wird durch Restriktionsverdau mit Pacl (Artikel Nr. R0547L, NEB, Frankfurt, DE) ein ca. 20 kb großes DNS-Fragment und ein (unerwünschtes) ca. 2 kb großes DNS-Fragment erzeugt. Das 20 kb Fragment wird mittels Agarosegelelektrophorese, Gelextraktion und DNS-Extraktion (Macherey-Nagel, Düren, DE) aufgereinigt.

Das Vorläuferplasmid pP11, das zur Aufnahme des 20 kb-Fragments dient (siehe Mayrhofer et al., J. Gene Med. 2008, 10(11):1253-1269, nun jedoch ohne MCS, ohne Spacer und mit einer statt zwei Identifikationssequenzen, wie in DE 10 2011 118 018 beschrieben), enthält eine Pacl-Restriktionsschnittstelle, die von Rekombinase-Erkennungssequenzen der parA-Resolvase flankiert ist und eine Expressionskassette der parA-Resolvase außerhalb der Rekombinase-Erkennungssequenzen. Dieses Plasmid wird mit dem Enzym Pacl (Artikel Nr. R0547L, NEB, Frankfurt, DE) geschnitten und mit Alkalischer Phosphatase (Artikel Nr. M0290L, NEB, Frankfurt, DE) dephosphoryliert. Anschließend wird das Fragment mit T4-Ligase in den linearisierten Vektor ligiert, um **PP.DM** (**FIG. 16**) zu erhalten.

### BEISPIEL 6:

### Konstruktion eines Parentalplasmids (PP.MD2G) für Minicircles umfassend das Glykoprotein G des Vesikuläre-Stomatitis-Virus (VSV-G) zur Verwendung bei der Erzeugung von lentiviralen Partikeln durch Kotransfektion

Das Plasmid **pMD.2G** (5824 bp), das eine Expressionskassette für das Glykoprotein G des Vesikuläre-Stomatitis-Virus (VSV-G) unter der Kontrolle des CMV-Promotors trägt (**FIG. 18**, SEQ ID NO:6), wird als Ausgangsmaterial zur gezielten Extraktion desjenigen Abschnitts verwendet, auf dem alle die o.g. Expressionscassete liegt. Dabei wird durch Restriktionsverdau mit HincII (Artikel Nr. R0103L, NEB, Frankfurt, DE) und mit MscI (Artikel Nr. R0534M, NEB, Frankfurt, DE) ein ca. 3,5 kb großes DNS-Fragment und ein (unerwünschtes) ca. 2,3 kb großes DNS-Fragement erzeugt. Das 3,5 kb Fragment wird mittels Agarosegelelektrophorese, Gelextraktion und DNS-Extraktion (Macherey-Nagel, Düren, DE) aufgereinigt.

Das Vorläuferplasmid pP11, das zur Aufnahme des 3,5 kb-Fragments dient (siehe Mayrhofer et al., J. Gene Med. 2008, 10(11):1253-1269, nun jedoch ohne MCS, ohne Spacer und mit einer statt zwei Identifikationssequenzen, wie in DE 10 2011 118 018 beschrieben), enthält eine Pacl-Restriktionsschnittstelle, die von Rekombinase-Erkennungssequenzen der parA-Resolvase flankiert ist und eine Expressionskassette der parA-Resolvase außerhalb der Rekombinase-Erkennungssequenzen. Dieses Plasmid wird mit dem Enzym Pacl (Artikel Nr. R0547L, NEB, Frankfurt, DE) geschnitten und mit Alkalischer Phosphatase (Artikel Nr. M0290L, NEB, Frankfurt, DE) dephosphoryliert. Anschließend wird das Fragment mit T4-Ligase in den linearisierten Vektor ligiert, um **PP.MD2G** (**FIG. 19**) zu erhalten.

### BEISPIEL 7:

### Herstellung von Minicircles aus den Parentalplasmiden PP.ssGFP, PP.DG, PP.PD2rs, PP.scGFP, PP.DM und PP.MD2G

Die Kultivierung wird bei 37°C in einem MBR-Bioreaktor (MBR BIO REACTOR, Schweiz) mit einem Gesamtvolumen von 7 l bei einer Füllmenge von 5 l durchgeführt. Die Anpassung des pHs auf pH 7 wird mit 2 M Natriumhydroxidlösung und 2 M Phosphorsäure durchgeführt. Die Flussrate der Luft wird auf 5 1/min eingestellt. Die Sauerstoffkonzentration (60%) wird durch die Variierung der Rührergeschwindigkeit im Bereich von 500 bis 2000 pro Minute kontrolliert. Es wird LB-Medium ohne Zugabe von Antibiotika verwendet. Das Verfahren wird mit jedem der sechs genannten Parentalplasmide durchgeführt.

Der Bioreaktor wird mit 50 ml einer Vorkultur von *E*. *coli* K12, die mit Parentalplasmid transformiert und für etwa 15 h bei 28°C kultiviert wurden, angeimpft. Die Vorkulturen werden unter selektiven Bedingungen unter Zugabe von 75 mg/ml Kanamycin kultiviert. Das LB-Medium der Vorkulturen wird mit Glucose angereichert, um eine vorzeitige Expression der parA-Resolvase, die unter der Kontrolle des P_{BAD}-Promotors steht, zu verhindern.

Die Expression der parA-Resolvase wird bei einer OD₆₀₀ zwischen 3,5 und 5,0 durch die Zugabe von L-Arabinose zum Medium induziert. Nach einer Stunde weiteren Wachstums werden die Zellen mittels Zentrifugation für 6 min bei 9039 g geerntet, in Lagerbeutel überführt, eingefroren und bei -20°C gelagert, bevor die Rekombinationsprodukte aufgereinigt werden.

Während der Kultivierung im Bioreaktor werden Proben entnommen und bei 4°C zur weiteren Analyse gelagert. Die OD₆₀₀ wird gemessen und die Plasmide werden aufgereinigt (NucleoBond® PC 100, Macherey-Nagel, Düren), um die Plasmidausbeute und die Rekombinationseffizienz zu bestimmen.

Aus der erhaltenen Biomasse werden die Rekombinationsprodukte Minicircle und Miniplasmid mittels kommerziell erhältlicher Plasmidisolierungskits (NucleoBond® PC 10 000, Macherey & Nagel, Düren) aufgereinigt.

Zur Isolierung des Minicircles aus der Mischung aus Minicircle und Miniplasmid wir eine spezifische Affinitätschromatographie verwendet. Hierzu wird ein biotinylierter Repressor des Lactose-Operons an Streptavidin-Sepharose-High-Performance (GE Healthcare) gekoppelt (vgl. Mayrhofer et al., J. Gene Med. 2008, 10(11):1253-1269).

Mit 5 ml dieser Chromatographiematrix wird eine XK 16 Chromatographiesäule befüllt und mit dem fünffachen Säulenvolumen 50 mM Tris pH 8, 400 mM NaCl äquilibriert. Die Mischung der Rekombinationsprodukte (1 mg/ml in 50 mM Tris pH 8, 400 mM NaCl) wird anschließend in einem ÄKTA-System (GE Healthcare) bei einer Flussrate von 0,5 ml/min auf das Säulenmaterial aufgetragen. Die Säule wird mit 50 mM Tris pH 8, 400 mM NaCl bei einer Flussrate von 1 ml/min gewaschen bis das am Gerät detektierte UV₂₆₀ₙₘ-Signal auf eine stabile Basislinie absinkt. Nun wird die Minicircle-DNS mit 50 mM Tris pH8, 500 mM NaCl, 5 mM IPTG eluiert, danach wird die Säule mit 50 mM Tris pH 8, 1 M NaCl und 50 mM Tris pH 8 gewaschen und zur weiteren Verwendung erneut äquilibriert.

Durch Präzipitation wird die DNS aus der Hochsalzmischung entfernt und schließlich in Wasser resuspendiert.

Die DNS der Minicircles **MC.ssGFP** (FIG. 11), **MC.DG** (FIG. 8), **MC.DP2rs** (FIG. 3), **MC.scGFP** (FIG. 14), **MC.DM** (FIG. 17) und **MC.MD2G** (FIG. 20) werden jeweils auf eine Konzentration von 1 mg/ml eingestellt.

Die erzeugten Minicircles werden anschließend einer Qualitätskontrolle unterzogen. Hierbei werden Tests auf Aussehen (klare Lösung, keine Partikel), DNS-Konzentration (mittels UV-Absorption bei 260 nm), DNS-Reinheit (mittels UV-Scan 220-320 nm), die korrekte Minicircle-Identität (mittels Restriktionsverdau und Agarosegelelektrophorese), Abwesenheit von RNS und bakterieller chromosomaler DNS (durch visuelle Inspektion nach Gelelektrophorese), DNS-Homogenität (mittels Kapillargelelektrophorese) und die Endotoxin-Konzentration (mittels Limulus-Amöbozyt-Lysat-Test) durchgeführt.

### BEISPIEL 8:

### Herstellung von AAV-Vektoren mit Hilfe von MC.ssGFP und MC.DP2rs

7,5 x10⁶ 293-Zellen wurden in einer 15 cm Petrischale in 25 ml DMEM mit Glutamax plus 10% fötalesm Kälberserum (Invitrogen) und 1% Penicillin/Streptomycin (Invitrogen) ausgesät und für 24 Stunden kultiviert. Für die Herstellung größerer Mengen wurden mehrere solcher Ansätze hergestellt. Bei ca. 80% Konfluenz erfolgte ein Medienwechsel auf 25 ml DMEM mit Glutamax plus 10% fötalem Kälberserum und 1% Penicillin/Streptomycin. Nach weiteren 2 Stunden erfolgte die Transfektion der Zellen mit Hilfe der Calciumphosphat-Methode. Hierbei werden 31,9 µg DNS (enthält 4,3 µg MC.ssGFP und 27,6 µg MC.DP2rs in einem 1:1 molaren Verhältnis) zu 1 ml 250 mM CaCl₂ gegeben und gemischt. Anschließend wird tropfenweise 1 ml HBS-Puffer zugesetzt (pH 7,29, 5,955 g HEPES, 8,18 g NaCl, 1,5 ml Na₂HPO₄ in 400 ml Endvolumen) und danach kurz gemischt. Nach 2 Minuten Inkubation wird die Lösung zu den Zellen gegeben. Nach Fortsetzung der Kultivierung wurde 24 Stunden später ein Medienwechsel auf 20 ml Dulbecco's Modified Eagle Medium (DMEM, Invitrogen) mit Glutamax plus 2% fötalem Kälberserum und 1% Penicillin/Streptomycin durchgeführt. Weitere 24 Stunden später wurden die Zellen durch Abkratzen geerntet und bei 3000xg zentrifugiert. Der Überstand wurde verworfen und das Pellet in 7,5 ml Lysispuffer (150 mM NaCl; 50 mM Tris/HCl, pH 8,5) aufgenommen. Der Aufschluss der Zellen erfolgte durch dreimaliges Einfrieren in fl. Stickstoff und Auftauen bei 37°C im Wasserbad. Zelluläre DNA und RNA, sowie noch vorhandene Plasmid-DNA wurde durch eine Benzonase-Behandlung entfernt (50U Benzonase pro ml Suspension; 30 Minuten, 37°C). Die Suspension wurde anschließend für 20 Minuten bei 3700xg zentrifugiert und der Überstand in ein steriles Ultrazentrifugen-Röhrchen überführt. Die Abtrennung von zellulären Proteinen erfolgte über eine Iodixanolgradientenzentrifugation (Peng et al., Anal. Biochem. 2006, 354(1): 140-147) gefolgt von einer Gelfiltration (ebenfalls nach Peng *et al.* 2006).

### BEISPIEL 9:

### Herstellung von AAV-Vektoren mit Hilfe von MC.ssGFP und MC.DG

7,5 x10⁶ 293-Zellen wurden in einer 15 cm Petrischale in 25 ml DMEM mit Glutamax plus 10% fötalesm Kälberserum (Invitrogen) und 1% Penicillin/Streptomycin (Invitrogen) ausgesät und für 24 Stunden kultiviert. Für die Herstellung größerer Mengen wurden mehrere solcher Ansätze hergestellt. Bei ca. 80% Konfluenz erfolgte ein Medienwechsel auf 25 ml DMEM mit Glutamax plus 10% fötalem Kälberserum und 1% Penicillin/Streptomycin. Nach weiteren 2 Stunden erfolgte die Transfektion der Zellen mit Hilfe der Calciumphosphat-Methode. Hierbei werden 31,9 µg DNS (enthält 4,3 µg MC.ssGFP und 27,6 µg MC.DG in einem 1:1 molaren Verhältnis) zu 1 ml 250 mM CaCl₂ gegeben und gemischt. Anschließend wird tropfenweise 1 ml HBS-Puffer zugesetzt (pH 7,29, 5,955 g HEPES, 8,18 g NaCl, 1,5 ml Na₂HPO₄ in 400 ml Endvolumen) und danach kurz gemischt. Nach 2 Minuten Inkubation wird die Lösung zu den Zellen gegeben. Nach Fortsetzung der Kultivierung wurde 24 Stunden später ein Medienwechsel auf 20 ml Dulbecco's Modified Eagle Medium (DMEM, Invitrogen) mit Glutamax plus 2% fötalem Kälberserum und 1% Penicillin/Streptomycin durchgeführt. Weitere 24 Stunden später wurden die Zellen durch Abkratzen geerntet und bei 3000xg zentrifugiert. Der Überstand wurde verworfen und das Pellet in 7,5 ml Lysispuffer (150 mM NaCl; 50 mM Tris/HCl, pH 8,5) aufgenommen. Der Aufschluss der Zellen erfolgte durch dreimaliges Einfrieren in fl. Stickstoff und Auftauen bei 37°C im Wasserbad. Zelluläre DNA und RNA, sowie noch vorhandene Plasmid-DNA wurde durch eine Benzonase-Behandlung entfernt (50U Benzonase pro ml Suspension; 30 Minuten, 37°C). Die Suspension wurde anschließend für 20 Minuten bei 3700xg zentrifugiert und der Überstand in ein steriles Ultrazentrifugen-Röhrchen überführt. Die Abtrennung von zellulären Proteinen erfolgte über eine Iodixanolgradientenzentrifugation (Peng et al., Anal. Biochem. 2006, 354(1): 140-147) gefolgt von einer Gelfiltration (ebenfalls nach Peng *et al.* 2006).

Dieses Experiment führte zu denselben Ergebnissen wie Beispiel 5.

### BEISPIEL 10:

### Herstellung von AAV-Vektoren mit Hilfe von pssGFP und MC.DP2rs

7,5 x10⁶ 293-Zellen wurden in einer 15 cm Petrischale in 25 ml DMEM mit Glutamax plus 10% fötalesm Kälberserum (Invitrogen) und 1% Penicillin/Streptomycin (Invitrogen) ausgesät und für 24 Stunden kultiviert. Für die Herstellung größerer Mengen wurden mehrere solcher Ansätze hergestellt. Bei ca. 80% Konfluenz erfolgte ein Medienwechsel auf 25 ml DMEM mit Glutamax plus 10% fötalem Kälberserum und 1% Penicillin/Streptomycin. Nach weiteren 2 Stunden erfolgte die Transfektion der Zellen mit Hilfe der Calciumphosphat-Methode. Hierbei werden 35,1 µg DNS (enthält 7,5 µg pssGFP und 27,6 µg MC.DP2rs in einem 1:1 molaren Verhältnis) zu 1 ml 250 mM CaCl₂ gegeben und gemischt. Anschließend wird tropfenweise 1 ml HBS-Puffer zugesetzt (pH 7,29, 5,955 g HEPES, 8,18 g NaCl, 1,5 ml Na₂HPO₄ in 400 ml Endvolumen) und danach kurz gemischt. Nach 2 Minuten Inkubation wird die Lösung zu den Zellen gegeben. Nach Fortsetzung der Kultivierung wurde 24 Stunden später ein Medienwechsel auf 20 ml Dulbecco's Modified Eagle Medium (DMEM, Invitrogen) mit Glutamax plus 2% fötalem Kälberserum und 1% Penicillin/Streptomycin durchgeführt. Weitere 24 Stunden später wurden die Zellen durch Abkratzen geerntet und bei 3000xg zentrifugiert. Der Überstand wurde verworfen und das Pellet in 7,5 ml Lysispuffer (150 mM NaCl; 50 mM Tris/HCl, pH 8,5) aufgenommen. Der Aufschluss der Zellen erfolgte durch dreimaliges Einfrieren in fl. Stickstoff und Auftauen bei 37°C im Wasserbad. Zelluläre DNA und RNA, sowie noch vorhandene Plasmid-DNA wurde durch eine Benzonase-Behandlung entfernt (50U Benzonase pro ml Suspension; 30 Minuten, 37°C). Die Suspension wurde anschließend für 20 Minuten bei 3700xg zentrifugiert und der Überstand in ein steriles Ultrazentrifugen-Röhrchen überführt. Die Abtrennung von zellulären Proteinen erfolgte über eine Iodixanolgradientenzentrifugation (Peng et al., Anal. Biochem. 2006, 354(1): 140-147) gefolgt von einer Gelfiltration (ebenfalls nach Peng *et al.* 2006).

Dieses Experiment führte zu denselben Ergebnissen wie Beispiel 5.

### BEISPIEL 11:

### Herstellung von AAV-Vektoren mit Hilfe von MC.ssGFP und pDP2rs

7,5 x10⁶ 293-Zellen wurden in einer 15 cm Petrischale in 25 ml DMEM mit Glutamax plus 10% fötalem Kälberserum (Invitrogen) und 1% Penicillin/Streptomycin (Invitrogen) ausgesät und für 24 Stunden kultiviert. Für die Herstellung größerer Mengen wurden mehrere solcher Ansätze hergestellt. Bei ca. 80% Konfluenz erfolgte ein Medienwechsel auf 25 ml DMEM mit Glutamax plus 10% fötalem Kälberserum und 1% Penicillin/Streptomycin. Nach weiteren 2 Stunden erfolgte die Transfektion der Zellen mit Hilfe der Calciumphosphat-Methode. Hierbei werden 34,3 µg DNS (enthält 4,3 µg MC.ssGFP und 30 µg pDP2rs in einem 1:1 molaren Verhältnis) zu 1 ml 250 mM CaCl₂ gegeben und gemischt. Anschließend wird tropfenweise 1 ml HBS-Puffer zugesetzt (pH 7,29, 5,955 g HEPES, 8,18 g NaCl, 1,5 ml Na₂HPO₄ in 400 ml Endvolumen) und danach kurz gemischt. Nach 2 Minuten Inkubation wird die Lösung zu den Zellen gegeben. Nach Fortsetzung der Kultivierung wurde 24 Stunden später ein Medienwechsel auf 20 ml Dulbecco's Modified Eagle Medium (DMEM, Invitrogen) mit Glutamax plus 2% fötalem Kälberserum und 1% Penicillin/Streptomycin durchgeführt. Weitere 24 Stunden später wurden die Zellen durch Abkratzen geerntet und bei 3000xg zentrifugiert. Der Überstand wurde verworfen und das Pellet in 7,5 ml Lysispuffer (150 mM NaCl; 50 mM Tris/HCl, pH 8,5) aufgenommen. Der Aufschluss der Zellen erfolgte durch dreimaliges Einfrieren in fl. Stickstoff und Auftauen bei 37°C im Wasserbad. Zelluläre DNA und RNA, sowie noch vorhandene Plasmid-DNA wurde durch eine Benzonase-Behandlung entfernt (50U Benzonase pro ml Suspension; 30 Minuten, 37°C). Die Suspension wurde anschließend für 20 Minuten bei 3700xg zentrifugiert und der Überstand in ein steriles Ultrazentrifugen-Röhrchen überführt. Die Abtrennung von zellulären Proteinen erfolgte über eine Iodixanolgradientenzentrifugation (Peng et al., Anal. Biochem. 2006, 354(1): 140-147) gefolgt von einer Gelfiltration (ebenfalls nach Peng *et al.* 2006).

Dieses Experiment führte zu denselben Ergebnissen wie Beispiel 5.

### BEISPIEL 12:

### Herstellung von lentiviralen Vektoren mit Hilfe von MC.MD2G, MC.CMVdR8.74 und MC.HRsinpptSEW

Zur Herstellung von lentiviralen Vektoren wurde ein Tripel-Transfektionssystem verwendet, bei dem die Verpackungssequenzen auf zwei Verpackungsvektoren verteilt sind. Alle drei Vektoren waren Minicircles. Als Verpackungsvektor verwendet wurde einerseits der oben beschriebene Minicircle MC.MD2G, der für das env-Protein aus vesikulärem Stomatitisvirus (VSV) kodiert. Als zweiter Verpackungsvektor wurde MC.CMVdR8.74. Dieser Minicircle hat 7472 bp und kodiert für die Proteine gag-pol, rev, und tat von HIV-1. Als Transfervektor wurde der Minicircle MC.HRsinpptSEW verwendet, der 5351 bp hat und für gfp kodiert. Die Minicircles MC.CMVdR8.74 und MC.HRsinpptSEW sind von den Plasmiden pCMVΔR8.74 bzw. pHRsinpptSEW aus Natarajan et al. (Neurogastroenterology & Motility 2014, 26:1513-1518) abgeleitet. Die Herstellung der Vektoren erfolgte auf Basis des in dieser Referenz beschriebenen Verfahrens, nur dass Minicircles statt konventioneller Plasmide verwendet wurden. 80% konfluente 293T-Zellen wurden über 4h mit 22,15 µg MC.HRsinpptSEW, 6,47 µg MC.MD2G und 18,80 µg MC.CMVdR8.74 transfiziert. Die viralen Partikel wurden nach 36 Stunden geerntet, filtriert und gefroren gelagert.

### BEISPIEL 13:

### Vergleich der Effizienz der Kotransfektion zwischen dem Verfahren nach Stand der Technik (Kotransfektion von ausschließlich Plasmid-basierten DNS) und ausschließlich oder teilweise Minicircle-basierten DNS zur AAV-Produktion mit dem sogenannten "2-Plasmid-System".

Die Effizienz der Produktion viraler AAV-Partikel mit Hilfe von Minicircle-Verpackungsvektor MC.PG2rs und Minicircle-Transfervektor MC.ssGFP in Kotransfektion (vgl. Beispiel 5) bzw. mit Hilfe von Minicircle-Verpackungsvektor MC.PG2rs und Plasmid-Transfervektor pssGFP (vgl. Beispiel 7) führt im äqimolaren Vergleich zur herkömmlichen Produktion mit Plasmid-basierter Kotransfektion (Plasmid-Transfervektor und Plasmid-Verpackungsvektor) zu gesteigerten Virus-Titern. Durch den Einsatz äqimolarer Mengen von Minicircle-DNS wird außerdem die insgesamt verwendete Menge DNS reduziert und eine verringerte (DNS-basierte) Toxizität erreicht.

### BEISPIEL 14:

### Vergleich der Effizienz der Kotransfektion zwischen dem Verfahren nach Stand der Technik (Kotransfektion von ausschließlich Plasmid-basierten DNS) und teilweise Minicircle-basierten DNS zur AAV-Produktion mit dem sogenannten "3-Plasmid-System".

Die Effizienz der Produktion viraler AAV Partikel mit Hilfe von Plasmid-DNS-basierten Helfer-/Verpackungsfunktionen im Tripel-Transfektionssystem wurde wie in Beispielen 5-8 getestet. Zur Transfektion verwendet wurden 7,5 µg pRC und 22,5 µg pXX6-80 (J. Rabinowitz et al., j. Virol. 2002, 76:791-801.) und 4,3 µg eines Minicircle-Transfervektors mit einer Stuffersequenz wie in FIG. 22B dargestellt. Im Vergleich zur herkömmlichen Produktion mit Plasmid-basierter Kotransfektion führt dieses Verfahren zu gleichen Virus-Titern bei weniger eingesetzter DNS.

### BEISPIEL 15:

### Vergleich der Herstellung rekombinanter viraler AAV Partikel mit dem "2-Plasmid-System" und deren Qualitätsprüfung hinsichtlich ihrer Produktivität und Freiheit von unerwünschten bakteriellen Sequenzen.

4 verschiedene DNA-Präparationen (Plasmid pDP2rs, Plasmid pssGFP, Minicircle MC.DP2rs, Minicircle MC.ssGFP) wurden in 4 möglichen Kombinationen und äquimolaren Mengen gemischt und zur Kotransfektion wie oben beschrieben eingesetzt. Außerdem wurden in zwei Kontrollansätzen Transfektionen durchgeführt, in denen keinerlei Transfer-Plasmid (pssGFP) bzw. keinerlei Tranfer-Minicircle (MC-ssGFP) zugesetzt wurde. Die jeweils verwendeten DNS-Mengen sind in Tabelle 1 dargestellt:

**Tabelle 1: In den Experimenten von Beispiel 15 zur Transfektion verwendete Mengen an Plasmiden.**

| Ansatz Nr. | pDP2rs | pssGFP | MC.DP2rs | MC.ssGFP |
|---|---|---|---|---|
| 2720-1 | 120 µg | 30 µg | | |
| 2720-2 | 120 µg | 30 µg | | |
| 2721-1 | 120 µg | | | 17,2 µg |
| 2721-2 | 120 µg | | | 17,2 µg |
| 2722-1 | | 30 µg | 110,4 µg | |
| 2722-2 | | 30 µg | 110,4 µg | |
| 2723-1 | | | 110,4 µg | 17,2 µg |
| 2723-2 | | | 100 µg | 15,6 µg |
| 2724 | | | 110,4 µg | |
| 2725 | 120 µg | | | |

Somit können sowohl die Kombination ausschließlich von Plasmid-Vektoren (2720-1 und 2720-2), als auch aus Mischungen von Plasmid und Minicircle (2721-1 und 2721-2 für den Verpackungsvektor als Plasmid und den Transfervektor als Minicircle, entsprechend Beispiel 9; 2722-1 und 2722-2 für den Verpackungsvektor als Minicircle und das Transfervektor als Plasmid, entsprechend Beispiel 10), sowie die Kombination ausschließlich von Minicircle-Vektoren und (2723-1 und 2723-2, entsprechend Beispiel 8) getestet werden.

Bei der Herstellung von rekombinanten AAV-Partikeln ist den Fachleuten bekannt, dass sowohl leere, als auch volle infektiöse und volle nicht-infektiöse Partikel entstehen. Außerdem sind immer mehr als nur ein infektiöses Viruspartikel nötig, um erfolgreich eine Zelle vorzugsweise per Rezeptor-vermittelter Endozytose zu infizieren. Damit ist eine Abhängigkeit von der zu infizierenden Zelle gegeben. Im vorliegenden Beispiel wurden HeLa-Zellen verwendet.

Gemessen wurden per quantitativer PCR die Titer gefüllter Partikel (genomischer Titer) und per ELISA die Capsid-Titer. Aus dem Verhältnis von genomischem Titer zu Capsid-Titer ergibt sich rechnerisch die Verpackungseffizienz. Der Transduktionstiter, was der Zahl der infektiösen Partikel entspricht wurde per FACS Analyse bestimmt Die Ergebnisse sind in Tabelle 2 dargestellt.

**Tabelle 2: Verpackungseffizienz und Transduktionstiter der viralen Vektoren aus Beispiel 15.**

| Ansatz Nr. | genomischer Titer | Capsid-Titer | Verpackungseffizienz | Transduktionstiter |
|---|---|---|---|---|
| 2720-1 | 3,96 · 10¹¹ | 8,25 · 10¹¹ | 0,50 | 2,50 · 10⁹ |
| 2720-2 | 3,70 · 10¹¹ | 5,77 · 10¹¹ | 0,64 | 1,04 · 10⁹ |
| 2721-1 | 9,82 · 10¹¹ | 1,01 · 10¹² | 0,97 | 4,53 · 10⁹ |
| 2721-2 | 3,96 · 10¹¹ | 7,44 · 10¹¹ | 0,53 | 2,91 · 10⁹ |
| 2722-1 | 3,70 · 10¹¹ | 8,85 · 10¹¹ | 0,42 | 7,90 · 10⁸ |
| 2722-2 | 1,75 · 10¹¹ | 8,59 · 10¹¹ | 0,20 | 6,32 · 10⁸ |
| 2723-1 | 4,82 · 10¹¹ | 8,01 · 10¹¹ | 0,60 | 2,64 · 10⁹ |
| 2723-2 | 5,70 · 10¹¹ | 9,25 · 10¹¹ | 0,62 | 1,56 · 10⁹ |

Im Western Blot wurde die Anwesenheit der 3 Leitproteine VP1 (89 kD), VP2 (72 kD) und VP3 (62 kD) festgestellt (siehe FIG. 24). Die Größe und das Verhältnis der Capsidproteine (VP1 : VP2 : VP3 = 1 : 1 : 10) war in allen Fällen korrekt. Alle Daten belegen, dass die Verwendung von Minicircle-DNS in dem hier durchgeführten Vergleich und mit HeLa-Zellen mindestens ebenso zuverlässig zur Herstellung rekombinanter viraler AAV-Partikel geeignet ist wie Plasmid-basierte Systeme.

Tabelle 3 zeigt den wesentlichen Aspekt des Systems: Es wurde untersucht, in welchen AAV-Präparationen noch bakterielle Sequenzen auftreten, die durch falsches Verpacken in die Viruspartikel gelangt sind und somit eine Gefahr für die pharmazeutische Verwendung dieser Vektoren darstellen. Durchgeführt wurde eine quantitative PCR-Detektion von Ampicillin-Resistenz-Sequenzen in den Präparationen.

**Tabelle 3: Anzahl der benötigten PCR-Zyklen bis zur Detektion von Ampicillin-Resistenz-Sequenzen und rechnerische Anzahl der Sequenzen pro Mikroliter Präparat.**

| Ansatz Nr. | Anzahl benötigte PCR-Zyklen | Pirtikelzahl pro µl |
|---|---|---|
| 2720-1 | 22,04 | 9,74 · 10⁵ |
| 2720-2 | 21,55 | 1,40 · 10⁶ |
| 2721-1 | 26,17 | 3,12 · 10⁴ |
| 2721-2 | 27,26 | 1,14 · 10⁴ |
| 2722-1 | 21,23 | 1,78 · 10⁶ |
| 2722-2 | 22,71 | 5,80 · 10⁵ |
| 2723-1 | 30,86 | 3,08 · 10² |
| 2723-2 | 31.14 | 2,28 · 10² |
| 2724 | 30,92 | 2,90 · 10² |
| 2725 | 28,92 | 2,26 · 10³ |
| H₂O | 31,64 | |

Die Negativkontrolle mit Wasser zeigt den Hintergrund des Systems (> 30 Zyklen). Alle Präparationen, an denen Plasmid-basierte DNS-Komponenten beteiligt waren (2720-1 und 2720-2, 2721-1 und 2721-2, 2722-1 und 2722-2) zeigen hohe Kontaminationsraten. Im Falle der Kombination 2721-1 und 2721-2 (für den Verpackungsvektor als Plasmid und den Transfervektor als Minicircle entsprechend Beispiel 9) ist der Effekt signifikant verringert, aber noch zu stark für eine sichere medizinische Verwendung. Hier befindet sich die Quelle der Kontamination auf dem sehr großen Verpackungsvektor und nicht auf dem Transfervektor. Lediglich bei Verwendung von keinerlei Plasmid-DNS (beide Komponenten auf Minicircle-Basis) sind keine Ampicillin-Resistenz-Sequenzen mehr vorhanden und werden somit auch nicht verpackt - dennoch werden hervorragende Virus-Titer erreicht.

## Patentansprüche

1. Ein Minicircle umfassend:
a. eine Transfersequenz,
b. spezifische Verpackungssignale zu beiden Seiten der Transfersequenz zur Verpackung der Transfersequenz in Partikel eines viralen Vektors,
wobei der Minicircle eine zirkuläre doppelsträngige DNS ist, die frei ist von prokaryotischen oder bakteriellen Nukehnsäuresequenzen.

2. Der Minicircle nach Anspruch 1, wobei der Minicircle zusätzlich umfasst:
c. mindestens eine Stuffersequenz innerhalb des Bereichs zwischen den spezifischen Verpackungssignalen oder mindestens eine Stuffersequenz außerhalb des Bereichs zwischen den spezifischen Verpackungssignalen.

3. Der Minicircle nach Anspruch 1 oder 2, wobei der Minicircle eine zirkuläre doppelsträngige DNS ist, die frei ist von Resistenzgenen gegen Antibiotika und/ oder Markergenen und/ oder bakteriellen Replikationsstartpunkten.

4. Der Minicircle nach einem der Ansprüche 1-3, wobei die spezifischen Verpackungssignale ausgewählt sind aus der Gruppe bestehend aus LTR-Sequenzen für lentivirale oder retrovirale Vektoren und ITR-Sequenzen für AAV-oder Ad-Vektoren.

5. Der Minicircle aus einem der Ansprüche 1-4, wobei jener virale Vektor ein Retrovirus, bevorzugt ein Lentivirus, bevorzugter ein AAV Virus ist.

6. Der Minicircle nach einem der Ansprüche 1-5, weiter umfassend mindestens eine Verpackungs-Expressionskassette, wobei auf der mindestens einen Verpackungs-Expressionskassette sämtliche Proteine kodiert sind und exprimiert werden können, die für die Verpackung der Transfersequenz in Partikel des viralen Vektors notwendig sind.

7. Eine Zelle umfassend den Minicircle aus einem der Ansprüche 1-6.

8. Verfahren zur Herstellung eines AAV-Vektors, das Verfahren umfassend:
a) Transfektion einer eukaryotischen Zelle mit - im Sinne einer Monotransfektion - mindestens einem Minicircle gemäß Anspruch 6, umfassend jene Transfersequenz und spezifische Verpackungssignale zu beiden Seiten der Transfersequenz zur Verpackung der Transfersequenz in Partikel jenes AAV-Vektors und mindestens eine Verpackungs-Expressionskassette, wobei auf der mindestens einen Expressions-Kassette sämtliche Proteine kodiert sind und exprimiert werden können, die für die Verpackung jener Transfersequenz in Partikel jenes AAV-Vektors notwendig sind;
wobei der Minicircle eine zirkuläre doppelsträngige DNS ist, die frei ist von Resistenzgenen gegen Antibiotika und bakteriellen Replikationsstartpunkten:
b) Expression der mindestens einen Verpackungs-Expressionskassette; und
c) Isolierung des AAV-Vektors aus der eukaryotischen Zelle oder einem ihrer Nachkommen, oder aus dem Medium, in dem sich diese befinden.

9. Das Verfahren nach Anspruch 8, wobei die eukaryotische Zelle eine Säugetierzelle ist.

## Claims

1. A minicircle comprising:
a. a transfer sequence,
b. specific packaging signals on both sides of the transfer sequence for packaging the transfer sequence into viral vector particles,
wherein the minicircle is a circular double stranded DNA that is free of prokaryotic or bacterial nucleic acid sequences.

2. The minicircle of claim 1, wherein the minicircle additionally comprises:
c. at least a stuffer sequence within the region between the specific packaging signals or at least a stuffer sequence outside the region between the specific packaging signals.

3. The minicircle of claim 1 or 2, wherein the minicircle is a circular double stranded DNA that is free of antibiotic resistance genes and/ or marker genes and/ or bacterial origins of replication.

4. The minicircle of any of claim 1-3, wherein the specific packaging signals are selected from the group consisting of LTR sequences for lentiviral or retroviral vectors and ITR sequences for AAV or Ad vectors.

5. The minicircle of any of claims 1-4, wherein said viral vector is a retro virus, preferably a lentivirus, more preferably an AAV virus.

6. The minicircle of any of claims 1-5, further comprising at least one packaging expression cassette, wherein all proteins necessary for packaging the transfer sequence into viral vector particles are encoded on and expressed from the at least one packaging expression cassette.

7. A cell comprising the minicircle of any of claims 1-6.

8. Method for producing an AAV vector, the method comprising:
a) transfecting an eukaryotic cell with - in the sense of monotransfection - at least one minicircle according to claim 6 comprising said transfer sequence and specific packaging signals on both sides of the transfer sequence for packaging the transfer sequence into particles of said AAV vector and at least one packaging expression cassette, wherein all proteins necessary for packaging said transfer sequence into particles of said AAV vector are encoded on and expressed from the at least one packaging expression cassette;
wherein the minicircle is a circular double stranded DNA that is free of antibiotic resistance genes and bacterial origins of replication;
b) expressing the at least one packaging expression cassette; and
c) isolating the AAV vector from the eukaryotic cell or from one of its progenies or from the medium in which they are located.

9. The method of claim 8, wherein the eukaryotic cell is a mammalian cell.

## Revendications

1. Mini-cercle comprenant :
a. une séquence de transfert,
b. des signaux d'encapsidation spécifiques des deux côtés de la séquence de transfert pour l'encapsidation de la séquence de transfert dans des particules d'un vecteur viral,
ledit mini-cercle étant un ADN circulaire double brin qui est exempt de séquences d'acides nucléiques procaryotes ou bactériennes.

2. Mini-cercle selon la revendication 1, ledit mini-cercle comprenant en plus :
c. au moins une séquence de remplissage à l'intérieur de la zone entre les signaux d'encapsidation spécifiques ou au moins une séquence de remplissage en dehors de la zone entre les signaux d'encapsidation spécifiques.

3. Mini-cercle selon la revendication 1 ou 2, ledit mini-cercle étant un ADN circulaire double brin qui est exempt de gènes de résistance contre les antibiotiques et/ou de gènes marqueurs et/ou de points de départ de réplication bactérienne.

4. Mini-cercle selon l'une des revendications 1 à 3, dans lequel les signaux d'encapsidation spécifiques sont choisis dans le groupe constitué de séquences LTR pour des vecteurs lentiviraux ou rétroviraux et de séquences ITR pour des vecteurs AAV ou Ad.

5. Mini-cercle selon l'une des revendications 1 à 4, dans lequel ledit vecteur viral est un rétrovirus, de préférence un lentivirus, de manière davantage préférée un virus AAV.

6. Mini-cercle selon l'une des revendications 1 à 5, comprenant en outre au moins une cassette d'expression d'encapsidation, toutes les protéines nécessaires à l'encapsidation de la séquence de transfert dans des particules du vecteur viral étant codées et pouvant être exprimées dans au moins une cassette d'expression d'encapsidation.

7. Cellule comprenant le mini-cercle de l'une des revendications 1 à 6.

8. Procédé de production d'un vecteur AAV, ledit procédé comprenant :
a) la transfection d'une cellule eucaryote avec - au sens d'une monotransfection - au moins un mini-cercle selon la revendication 6, comprenant ladite séquence de transfert et lesdits signaux d'encapsidation spécifiques des deux côtés de la séquence de transfert pour l'encapsidation de la séquence de transfert dans des particules dudit vecteur AAV et au moins une cassette d'expression d'encapsidation, toutes les protéines nécessaires à l'encapsidation de ladite séquence de transfert dans des particules dudit vecteur AAV étant codées et pouvant être exprimées dans au moins une cassette d'expression d'encapsidation ;
ledit mini-cercle étant un ADN circulaire double brin qui est exempt de gènes de résistance contre les antibiotiques et de points de départ de réplication bactérienne ;
b) l'expression d'au moins une cassette d'expression d'encapsidation ; et
c) l'isolation du vecteur AAV à partir de la cellule eucaryote ou d'un de ses descendants ou du milieu dans lequel ceux-ci se trouvent.

9. Procédé selon la revendication 8, dans lequel la cellule eucaryote est une cellule de mammifère.


### SEQUENCE LISTING

<110> PlasmidFactory GmbH & Co. KG
<120> MINICIRCLES MIT VIRALEN EXPRESSIONSKASSETTEN UND IHRE VERWENDUNG ZUR TRANSFORMATION VON ZELLEN ZUR ERZEUGUNG REKOMBINANTER VIREN ODER VIRALER GENVEKTOREN
<130> 113-042EPT1
<150> 14188981.6
   <151> 15.10.2014
<150> 102013220859.6
   <151> 15.10.2013
<160> 9
<170> PatentIn version 3.5
<210> 1
   <211> 23677
   <212> DNA
   <213> Künstliche Sequenz
<400> 1
<210> 2
   <211> 21846
   <212> DNA
   <213> Künstliche Sequenz
<400> 2
   ggatccgaat tcttaattaa catcatcaat aatatacctt attttggatt gaagccaata 60
   tgataatgag ggggtggagt ttgtgacgtg gcgcggggcg tgggaacggg gcgggtgacg 120
   tagtagtgtg gcggaagtgt gatgttgcaa gtgtggcgga acacatgtaa gcgacggatg 180
   tggcaaaagt gacgtttttg gtgtgcgccg gtgtacacag gaagtgacaa ttttcgcgcg 240
   gttttaggcg gatgttgtag taaatttggg cgtaaccgag taagatttgg ccattttcgc 300
   gggaaaactg aataagagga agtgaaatct gaataatttt gtgttactca tagcgcgtaa 360
   tatttgtcta gggccgcggg gactttgacc gtttacgtgg agactcgccc aggtgttttt 420
   ctcaggtgtt ttccgcgttc cgggtcaaag ttggcgtttt attattatag tcagggggat 480
   cctctagaac tagtggatcc gtccctcagg cctagaagta aaaaagggaa aaaagagtgt 540
   gtttgtcaaa ataggagaca ggtggtggca accaaggact tataggggac cttacatcta 600
   cagaccaaca gatgccccct taccatatac aggaagatat gacttaaatt gggataggtg 660
   ggtcacaatc aacggctata aagtgttata cagatccctc ccctcccctt tcgtgaaaga 720
   ctcgccagag ctagacctcc ttggtgtatg ctaactgaga agagaaagac gacatgaaac 780
   aacaggtaca tgattatatt tatctaggaa caggaatgca cttttgggga aaggttttcc 840
   ataccaagga aggggcagtg gctggactga tagaacatta ttctgcaaaa acttatggta 900
   tgagttatta tgattagcct ttatttgccc aaccttgcgg ttcccagggt ttaaataagt 960
   ttatggttac aaactgttct taaaacgagg atgtgagaca agtggtttcc tgacttggtt 1020
   tggtaatcaa atgttctgat ctgagctctt agtgttctat tttcctatgt tcttttggaa 1080
   tctatccaag tcttatgtaa atgcttatgt aaaccataat ataaaagagt gctgattttt 1140
   tgagtaaact tgcaacagtc ctaacattct tctctcgtgt gtttgtgtct gttcgccatc 1200
   ccgtctccgc tcgtcactta tccttcactt ttcagagggt ccccccgcag atcccggtca 1260
   ccctcaggtc gggacctgca gaagacgccc gagtgagcac gcagggtctc cattttgaag 1320
   cgggaggttt gaacgcgcag ccgccatgcc ggggttttac gagattgtga ttaaggtccc 1380
   cagcgacctt gacgggcatc tgcccggcat ttctgacagc tttgtgaact gggtggccga 1440
   gaaggaatgg gagttgccgc cagattctga catggatctg aatctgattg agcaggcacc 1500
   cctgaccgtg gccgagaagc tgcagcgcga ctttctgacg gaatggcgcc gtgtgagtaa 1560
   ggccccggag gcccttttct ttgtgcaatt tgagaaggga gagagctact tccacatgca 1620
   cgtgctcgtg gaaaccaccg gggtgaaatc catggttttg ggacgtttcc tgagtcagat 1680
   tcgcgaaaaa ctgattcaga gaatttaccg cgggatcgag ccgactttgc caaactggtt 1740
   cgcggtcaca aagaccagaa atggcgccgg aggcgggaac aaggtggtgg atgagtgcta 1800
   catccccaat tacttgctcc ccaaaaccca gcctgagctc cagtgggcgt ggactaatat 1860
   ggaacagtat ttaagcgcct gtttgaatct cacggagcgt aaacggttgg tggcgcagca 1920
   tctgacgcac gtgtcgcaga cgcaggagca gaacaaagag aatcagaatc ccaattctga 1980
   tgcgccggtg atcagatcaa aaacttcagc caggtacatg gagctggtcg ggtggctcgt 2040
   ggacaagggg attacctcgg agaagcagtg gatccaggag gaccaggcct catacatctc 2100
   cttcaatgcg gcctccaact cgcggtccca aatcaaggct gccttggaca atgcgggaaa 2160
   gattatgagc ctgactaaaa ccgcccccga ctacctggtg ggccagcagc ccgtggagga 2220
   catttccagc aatcggattt ataaaatttt ggaactaaac gggtacgatc cccaatatgc 2280
   ggcttccgtc tttctgggat gggccacgaa aaagttcggc aagaggaaca ccatctggct 2340
   gtttgggcct gcaactaccg ggaagaccaa catcgcggag gccatagccc acactgtgcc 2400
   cttctacggg tgcgtaaact ggaccaatga gaactttccc ttcaacgact gtgtcgacaa 2460
   gatggtgatc tggtgggagg aggggaagat gaccgccaag gtcgtggagt cggccaaagc 2520
   cattctcgga ggaagcaagg tgcgcgtgga ccagaaatgc aagtcctcgg cccagataga 2580
   cccgactccc gtgatcgtca cctccaacac caacatgtgc gccgtgattg acgggaactc 2640
   aacgaccttc gaacaccagc agccgttgca agaccggatg ttcaaatttg aactcacccg 2700
   ccgtctggat catgactttg ggaaggtcac caagcaggaa gtcaaagact ttttccggtg 2760
   ggcaaaggat cacgtggttg aggtggagca tgaattctac gtcaaaaagg gtggagccaa 2820
   gaaaagaccc gcccccagtg acgcagatat aagtgagccc aaacgggtgc gcgagtcagt 2880
   tgcgcagcca tcgacgtcag acgcggaagc ttcgatcaac tacgcagaca ggtaccaaaa 2940
   caaatgttct cgtcacgtgg gcatgaatct gatgctgttt ccctgcagac aatgcgagag 3000
   aatgaatcag aattcaaata tctgcttcac tcacggacag aaagactgtt tagagtgctt 3060
   tcccgtgtca gaatctcaac ccgtttctgt cgtcaaaaag gcgtatcaga aactgtgcta 3120
   cattcatcat atcatgggaa aggtgccaga cgcttgcact gcctgcgatc tggtcaatgt 3180
   ggatttggat gactgcatct ttgaacaata aatgatttaa atcaggtatg gctgccgatg 3240
   gttatcttcc agattggctc gaggacactc tctctgaagg aataagacag tggtggaagc 3300
   tcaaacctgg cccaccacca ccaaagcccg cagagcggca taaggacgac agcaggggtc 3360
   ttgtgcttcc tgggtacaag tacctcggac ccttcaacgg actcgacaag ggagagccgg 3420
   tcaacgaggc agacgccgcg gccctcgagc acgtacaaag cctacgaccg gcagctcgac 3480
   agcggagaca acccgtacct caagtacaac cacgccgacg cggagtttca ggagcgcctt 3540
   aaagaagata cgtcttttgg gggcaacctc ggacgagcag tcttccaggc gaaaaagagg 3600
   gttcttgaac ctctgggcct ggttgaggaa cctgttaaga cggctccggg aaaaaagagg 3660
   ccggtagagc actctcctgt ggagccagac tcctcctcgg gaaccggaaa ggcgggccag 3720
   cagcctgcaa gaaaaagatt gaattttggt cagactggag acgcagactc agtacctgac 3780
   ccccagcctc tcggacagcc accagcagcc ccctctggtc tgggaactaa tacgatggct 3840
   acaggcagtg gcgcaccaat ggcagacaat aacgagggcg ccgacggagt gggtaattcc 3900
   tccggaaatt ggcattgcga ttccacatgg atgggcgaca gagtcatcac caccagcacc 3960
   cgaacctggg ccctgcccac ctacaacaac cacctctaca aacaaatttc cagccaatca 4020
   ggagcctcga acgacaatca ctactttggc tacagcaccc cttgggggta ttttgacttc 4080
   aacagattcc actgccactt ttcaccacgt gactggcaaa gactcatcaa caacaactgg 4140
   ggattccgac ccaagagact caacttcaag ctctttaaca ttcaagtcaa agaggtcacg 4200
   cagaatgacg gtacgacgac gattgccaat aaccttacca gcacggttca ggtgtttact 4260
   gactcggagt accagctccc gtacgtcctc ggctcggcgc atcaaggatg cctcccgccg 4320
   ttcccagcag acgtcttcat ggtgccacag tatggatacc tcaccctgaa caacgggagt 4380
   caggcagtag gacgctcttc attttactgc ctggagtact ttccttctca gatgctgcgt 4440
   accggaaaca actttacctt cagctacact tttgaggacg ttcctttcca cagcagctac 4500
   gctcacagcc agagtctgga ccgtctcatg aatcctctca tcgaccagta cctgtattac 4560
   ttgagcagaa caaacactcc aagtggaacc accacgcagt caaggcttca gttttctcag 4620
   gccggagcga gtgacattcg ggaccagtct aggaactggc ttcctggacc ctgttaccgc 4680
   cagcagcgag tatcaaagac atctgcggat aacaacaaca gtgaatactc gtggactgga 4740
   gctaccaagt accacctcaa tggcagagac tctctggtga atccggccat ggcaagccac 4800
   aaggacgatg aagaaaagtt ttttcctcag agcggggttc tcatctttgg gaagcaaggc 4860
   tcagagaaaa caaatgtgaa cattgaaaag gtcatgatta cagacgaaga ggaaatcgga 4920
   acaaccaatc ccgtggctac ggagcagtat ggttctgtat ctaccaacct ccagagaggc 4980
   aacagacaag cagctaccgc agatgtcaac acacaaggcg ttcttccagg catggtctgg 5040
   caggacagag atgtgtacct tcaggggccc atctgggcaa agattccaca cacggacgga 5100
   cattttcacc cctctcccct catgggtgga ttcggactta aacaccctcc tccacagatt 5160
   ctcatcaaga acaccccggt acctgcgaat ccttcgacca ccttcagtgc ggcaaagttt 5220
   gcttccttca tcacacagta ctccacggga cacggtcagc gtggagatcg agtgggagct 5280
   gcagaaggaa aacagcaaac gctggaatcc cgaaattcag tacacttcca actacaacaa 5340
   gtctgttaat cgtggactta ccgtggatac taatggcgtg tattcagagc ctcgccccat 5400
   tggcaccaga tacctgactc gtaatctgta attgcttgtt aatcaataaa ccgtttaatt 5460
   cgtttcagtt gaactttggt ctctgcgtat ttctttctta tctagtttcc atggctacat 5520
   cagcttatcg atgcatgtcc ttgggtccgg cctgctgaat gcgcaggcgg tcggccatgc 5580
   cccaggcttc gttttgacat cggcgcaggt ctttgtagta gtcttgcatg agcctttcta 5640
   ccggcacttc ttcttctcct tcctcttgtc ctgcatctct tgcatctatc gctgcggcgg 5700
   cggcggagtt tggccgtagg tggcgccctc ttcctcccat gcgtgtgacc ccgaagcccc 5760
   tcatcggctg aagcagggct aggtcggcga caacgcgctc ggctaatatg gcctgctgca 5820
   cctgcgtgag ggtagactgg aagtcatcca tgtccacaaa gcggtggtat gcgcccgtgt 5880
   tgatggtgta agtgcagttg gccataacgg accagttaac ggtctggtga cccggctgcg 5940
   agagctcggt gtacctgaga cgcgagtaag ccctcgagtc aaatacgtag tcgttgcaag 6000
   tccgcaccag gtactggtat cccaccaaaa agtgcggcgg cggctggcgg tagaggggcc 6060
   agcgtagggt ggccggggct ccgggggcga gatcttccaa cataaggcga tgatatccgt 6120
   agatgtacct ggacatccag gtgatgccgg cggcggtggt ggaggcgcgc ggaaagtcgc 6180
   ggacgcggtt ccagatgttg cgcagcggca aaaagtgctc catggtcggg acgctctggc 6240
   cggtcaggcg cgcgcaatcg ttgacgctct agaccgtgca aaaggagagc ctgtaagcgg 6300
   gcactcttcc gtggtctggt ggataaattc gcaagggtat catggcggac gaccggggtt 6360
   cgagccccgt atccggccgt ccgccgtgat ccatgcggtt accgcccgcg tgtcgaaccc 6420
   aggtgtgcga cgtcagacaa cgggggagtg ctccttttgg cttccttcca ggcgcggcgg 6480
   ctgctgcgct agcttttttg gccactggcc gcgcgcagcg taagcggtta ggctggaaag 6540
   cgaaagcatt aagtggctcg ctccctgtag ccggagggtt attttccaag ggttgagtcg 6600
   cgggaccccc ggttcgagtc tcggaccggc cggactgcgg cgaacggggg tttgcctccc 6660
   cgtcatgcaa gaccccgctt gcaaattcct ccggaaacag ggacgagccc cttttttgct 6720
   tttcccagat gcatccggtg ctgcggcaga tgcgcccccc tcctcagcag cggcaagagc 6780
   aagagcagcg gcagacatgc agggcaccct cccctcctcc taccgcgtca ggaggggcga 6840
   catccgcggt tgacgcggca gcagatggtg attacgaacc cccgcggcgc cgggcccggc 6900
   actacctgga cttggaggag ggcgagggcc tggcgcggct aggagcgccc tctcctgagc 6960
   ggtacccaag ggtgcagctg aagcgtgata cgcgtgaggc gtacgtgccg cggcagaacc 7020
   tgtttcgcga ccgcgaggga gaggagcccg aggagatgcg ggatcgaaag ttccacgcag 7080
   ggcgcgagct gcggcatggc ctgaatcgcg agcggttgct gcgcgaggag gactttgagc 7140
   ccgacgcgcg aaccgggatt agtcccgcgc gcgcacacgt ggcggccgcc gacctggtaa 7200
   ccgcatacga gcagacggtg aaccagggcg atcgcaccct ttggcgcatc ccattctcca 7260
   gtaactttat gtccatgggc gcactcacag acctgggcca aaaccttctc tacgccaact 7320
   ccgcccacgc gctagacatg acttttgagg tggatcccat ggacgagccc acccttcttt 7380
   atgttttgtt tgaagtcttt gacgtggtcc gtgtgcaccg gccgcaccgc ggcgtcatcg 7440
   aaaccgtgta cctgcgcacg cccttctcgg ccggcaacgc cacaacataa agaagcaagc 7500
   aacatcaaca acagctgccg ccatgggctc cagtgagcag gaactgaaag ccattgtcaa 7560
   agatcttggt tgtgggccat attttttggg cacctatgac aagcgctttc caggctttgt 7620
   ttctccacac aagctcgcct gcgccatagt caatacggcc ggtcgcgaga ctgggggcgt 7680
   acactggatg gcctttgcct ggaacccgca ctcaaaaaca tgctacctct ttgagccctt 7740
   tggcttttct gaccagcgac tcaagcaggt ttaccagttt gagtacgagt cactcctgcg 7800
   ccgtagcgcc attgcttctt cccccgaccg ctgtataacg ctggaaaagt ccacccaaag 7860
   cgtacagggg cccaactcgg ccgcctgtgg actattctgc tgcatgtttc tccacgcctt 7920
   tgccaactgg ccccaaactc ccatggatca caaccccacc atgaacctta ttaccggggt 7980
   acccaactcc atgctcaaca gtccccaggt acagcccacc ctgcgtcgca accaggaaca 8040
   gctctacagc ttcctggagc gccactcgcc ctacttccgc agccacagtg cgcagattag 8100
   gagcgccact tctttttgtc acttgaaaaa catgtaaaaa taatgtacta gagacacttt 8160
   caataaaggc aaatgctttt atttgtacac tctcgggtga ttatttaccc ccacccttgc 8220
   cgtctgcgcc gtttaaaaat caaaggggtt ctgccgcgca tcgctatgcg ccactggcag 8280
   ggacacgttg cgatactggt gtttagtgct ccacttaaac tcaggcacaa ccatccgcgg 8340
   cagctcggtg aagttttcac tccacaggct gcgcaccatc accaacgcgt ttagcaggtc 8400
   gggcgccgat atcttgaagt cgcagttggg gcctccgccc tgcgcgcgcg agttgcgata 8460
   cacagggttg cagcactgga acactatcag cgccgggtgg tgcacgctgg ccagcacgct 8520
   cttgtcggag atcagatccg cgtccaggtc ctccgcgttg ctcagggcga acggagtcaa 8580
   ctttggtagc tgccttccca aaaagggcgc gtgcccaggc tttgagttgc actcgcaccg 8640
   tagtggcatc aaaaggtgac cgtgcccggt ctgggcgtta ggatacagcg cctgcataaa 8700
   agccttgatc tgcttaaaag ccacctgagc ctttgcgcct tcagagaaga acatgccgca 8760
   agacttgccg gaaaactgat tggccggaca ggccgcgtcg tgcacgcagc accttgcgtc 8820
   ggtgttggag atctgcacca catttcggcc ccaccggttc ttcacgatct tggccttgct 8880
   agactgctcc ttcagcgcgc gctgcccgtt ttcgctcgtc acatccattt caatcacgtg 8940
   ctccttattt atcataatgc ttccgtgtag acacttaagc tcgccttcga tctcagcgca 9000
   gcggtgcagc cacaacgcgc agcccgtggg ctcgtgatgc ttgtaggtca cctctgcaaa 9060
   cgactgcagg tacgcctgca ggaatcgccc catcatcgtc acaaaggtct tgttgctggt 9120
   gaaggtcagc tgcaacccgc ggtgctcctc gttcagccag gtcttgcata cggccgccag 9180
   agcttccact tggtcaggca gtagtttgaa gttcgccttt agatcgttat ccacgtggta 9240
   cttgtccatc agcgcgcgcg cagcctccat gcccttctcc cacgcagaca cgatcggcac 9300
   actcagcggg ttcatcaccg taatttcact ttccgcttcg ctgggctctt cctcttcctc 9360
   ttgcgtccgc ataccacgcg ccactgggtc gtcttcattc agccgccgca ctgtgcgctt 9420
   acctcctttg ccatgcttga ttagcaccgg tgggttgctg aaacccacca tttgtagcgc 9480
   cacatcttct ctttcttcct cgctgtccac gattacctct ggtgatggcg ggcgctcggg 9540
   cttgggagaa gggcgcttct ttttcttctt gggcgcaatg gccaaatccg ccgccgaggt 9600
   cgatggccgc gggctgggtg tgcgcggcac cagcgcgtct tgtgatgagt cttcctcgtc 9660
   ctcggactcg atacgccgcc tcatccgctt ttttgggggc gcccggggag gcggcggcga 9720
   cggggacggg gacgacacgt cctccatggt tgggggacgt cgcgccgcac cgcgtccgcg 9780
   ctcgggggtg gtttcgcgct gctcctcttc ccgactggcc atttccttct cctataggca 9840
   gaaaaagatc atggagtcag tcgagaagaa ggacagccta accgccccct ctgagttcgc 9900
   caccaccgcc tccaccgatg ccgccaacgc gcctaccacc ttccccgtcg aggcaccccc 9960
   gcttgaggag gaggaagtga ttatcgagca ggacccaggt tttgtaagcg aagacgacga 10020
   ggaccgctca gtaccaacag aggataaaaa gcaagaccag gacaacgcag aggcaaacga 10080
   ggaacaagtc gggcgggggg acgaaaggca tggcgactac ctagatgtgg gagacgacgt 10140
   gctgttgaag catctgcagc gccagtgcgc cattatctgc gacgcgttgc aagagcgcag 10200
   cgatgtgccc ctcgccatag cggatgtcag ccttgcctac gaacgccacc tattctcacc 10260
   gcgcgtaccc cccaaacgcc aagaaaacgg cacatgcgag cccaacccgc gcctcaactt 10320
   ctaccccgta tttgccgtgc cagaggtgct tgccacctat cacatctttt tccaaaactg 10380
   caagataccc ctatcctgcc gtgccaaccg cagccgagcg gacaagcagc tggccttgcg 10440
   gcagggcgct gtcatacctg atatcgcctc gctcaacgaa gtgccaaaaa tctttgaggg 10500
   tcttggacgc gacgagaagc gcgcggcaaa cgctctgcaa caggaaaaca gcgaaaatga 10560
   aagtcactct ggagtgttgg tggaactcga gggtgacaac gcgcgcctag ccgtactaaa 10620
   acgcagcatc gaggtcaccc actttgccta cccggcactt aacctacccc ccaaggtcat 10680
   gagcacagtc atgagtgagc tgatcgtgcg ccgtgcgcag cccctggaga gggatgcaaa 10740
   tttgcaagaa caaacagagg agggcctacc cgcagttggc gacgagcagc tagcgcgctg 10800
   gcttcaaacg cgcgagcctg ccgacttgga ggagcgacgc aaactaatga tggccgcagt 10860
   gctcgttacc gtggagcttg agtgcatgca gcggttcttt gctgacccgg agatgcagcg 10920
   caagctagag gaaacattgc actacacctt tcgacagggc tacgtacgcc aggcctgcaa 10980
   gatctccaac gtggagctct gcaacctggt ctcctacctt ggaattttgc acgaaaaccg 11040
   ccttgggcaa aacgtgcttc attccacgct caagggcgag gcgcgccgcg actacgtccg 11100
   cgactgcgtt tacttatttc tatgctacac ctggcagacg gccatgggcg tttggcagca 11160
   gtgcttggag gagtgcaacc tcaaggagct gcagaaactg ctaaagcaaa acttgaagga 11220
   cctatggacg gccttcaacg agcgctccgt ggccgcgcac ctggcggaca tcattttccc 11280
   cgaacgcctg cttaaaaccc tgcaacaggg tctgccagac ttcaccagtc aaagcatgtt 11340
   gcagaacttt aggaacttta tcctagagcg ctcaggaatc ttgcccgcca cctgctgtgc 11400
   acttcctagc gactttgtgc ccattaagta ccgcgaatgc cctccgccgc tttggggcca 11460
   ctgctacctt ctgcagctag ccaactacct tgcctaccac tctgacataa tggaagacgt 11520
   gagcggtgac ggtctactgg agtgtcactg tcgctgcaac ctatgcaccc cgcaccgctc 11580
   cctggtttgc aattcgcagc tgcttaacga aagtcaaatt atcggtacct ttgagctgca 11640
   gggtccctcg cctgacgaaa agtccgcggc tccggggttg aaactcactc cggggctgtg 11700
   gacgtcggct taccttcgca aatttgtacc tgaggactac cacgcccacg agattaggtt 11760
   ctacgaagac caatcccgcc cgccaaatgc ggagcttacc gcctgcgtca ttacccaggg 11820
   ccacattctt ggccaattgc aagccatcaa caaagcccgc caagagtttc tgctacgaaa 11880
   gggacggggg gtttacttgg acccccagtc cggcgaggag ctcaacccaa tccccccgcc 11940
   gccgcagccc tatcagcagc agccgcgggc ccttgcttcc caggatggca cccaaaaaga 12000
   agctgcagct gccgccgcca cccacggacg aggaggaata ctgggacagt caggcagagg 12060
   aggttttgga cgaggaggag gaggacatga tggaagactg ggagagccta gacgaggaag 12120
   cttccgaggt cgaagaggtg tcagacgaaa caccgtcacc ctcggtcgca ttcccctcgc 12180
   cggcgcccca gaaatcggca accggttcca gcatggctac aacctccgct cctcaggcgc 12240
   cgccggcact gcccgttcgc cgacccaacc gtagatggga caccactgga accagggccg 12300
   gtaagtccaa gcagccgccg ccgttagccc aagagcaaca acagcgccaa ggctaccgct 12360
   catggcgcgg gcacaagaac gccatagttg cttgcttgca agactgtggg ggcaacatct 12420
   ccttcgcccg ccgctttctt ctctaccatc acggcgtggc cttcccccgt aacatcctgc 12480
   attactaccg tcatctctac agcccatact gcaccggcgg cagcggcagc ggcagcaaca 12540
   gcagcggcca cacagaagca aaggcgaccg gatagcaaga ctctgacaaa gcccaagaaa 12600
   tccacagcgg cggcagcagc aggaggagga gcgctgcgtc tggcgcccaa cgaacccgta 12660
   tcgacccgcg agcttagaaa caggattttt cccactctgt atgctatatt tcaacagagc 12720
   aggggccaag aacaagagct gaaaataaaa aacaggtctc tgcgatccct cacccgcagc 12780
   tgcctgtatc acaaaagcga agatcagctt cggcgcacgc tggaagacgc ggaggctctc 12840
   ttcagtaaat actgcgcgct gactcttaag gactagtttc gcgccctttc tcaaatttaa 12900
   gcgcgaaaac tacgtcatct ccagcggcca cacccggcgc cagcacctgt cgtcagcgcc 12960
   attatgagca aggaaattcc cacgccctac atgtggagtt accagccaca aatgggactt 13020
   gcggctggag ctgcccaaga ctactcaacc cgaataaact acatgagcgc gggaccccac 13080
   atgatatccc gggtcaacgg aatccgcgcc caccgaaacc gaattctctt ggaacaggcg 13140
   gctattacca ccacacctcg taataacctt aatccccgta gttggcccgc tgccctggtg 13200
   taccaggaaa gtcccgctcc caccactgtg gtacttccca gagacgccca ggccgaagtt 13260
   cagatgacta actcaggggc gcagcttgcg ggcggctttc gtcacagggt gcggtcgccc 13320
   gggcagggta taactcacct gacaatcaga gggcgaggta ttcagctcaa cgacgagtcg 13380
   gtgagctcct cgcttggtct ccgtccggac gggacatttc agatcggcgg cgccggccgc 13440
   tcttcattca cgcctcgtca ggcaatccta actctgcaga cctcgtcctc tgagccgcgc 13500
   tctggaggca ttggaactct gcaatttatt gaggagtttg tgccatcggt ctactttaac 13560
   cccttctcgg gacctcccgg ccactatccg gatcaattta ttcctaactt tgacgcggta 13620
   aaggactcgg cggatggcta cgactgaatg ttaagtggag aggcagagca actgcgcctg 13680
   aaacacctgg tccactgtcg ccgccacaag tgctttgccc gcgactccgg tgagttttgc 13740
   tactttgaat tgcccgagga tcatatcgag ggcccggcgc acggcgtccg gcttaccgcc 13800
   cagggagagc ttgcccgtag cctgattcgg gagtttaccc agcgccccct gctagttgag 13860
   cgggacaggg gaccctgtgt tctcactgtg atttgcaact gtcctaaccc tggattacat 13920
   caagatcttt gttgccatct ctgtgctgag tataataaat acagaaatta aaatatactg 13980
   gggctcctat cgccatcctg taaacgccac cgtcttcacc cgcccaagca aaccaaggcg 14040
   aaccttacct ggtactttta acatctctcc ctctgtgatt tacaacagtt tcaacccaga 14100
   cggagtgagt ctacgagaga acctctccga gctcagctac tccatcagaa aaaacaccac 14160
   cctccttacc tgccgggaac gtacgagtgc gtcaccggcc gctgcaccac acctaccgcc 14220
   tgaccgtaaa ccagactttt tccggacaga cctcaataac tctgtttacc agaacaggag 14280
   gtgagcttag aaaaccctta gggtattagg ccaaaggcgc agctactgtg gggtttatga 14340
   acaattcaag caactctacg ggctattcta attcaggttt ctctagaaat ggacggaatt 14400
   attacagagc agcgcctgct agaaagacgc agggcagcgg ccgagcaaca gcgcatgaat 14460
   caagagctcc aagacatggt taacttgcac cagtgcaaaa ggggtatctt ttgtctggta 14520
   aagcaggcca aagtcaccta cgacagtaat accaccggac accgccttag ctacaagttg 14580
   ccaaccaagc gtcagaaatt ggtggtcatg gtgggagaaa agcccattac cataactcag 14640
   cactcggtag aaaccgaagg ctgcattcac tcaccttgtc aaggacctga ggatctctgc 14700
   acccttatta agaccctgtg cggtctcaaa gatcttattc cctttaacta ataaaaaaaa 14760
   ataataaagc atcacttact taaaatcagt tagcaaattt ctgtccagtt tattcagcag 14820
   cacctccttg ccctcctccc agctctggta ttgcagcttc ctcctggctg caaactttct 14880
   ccacaatcta aatggaatgt cagtttcctc ctgttcctgt ccatccgcac ccactatctt 14940
   catgttgttg cagatgaagc gcgcaagacc gtctgaagat accttcaacc ccgtgtatcc 15000
   atatgacacg gaaaccggtc ctccaactgt gccttttctt actcctccct ttgtatcccc 15060
   caatgggttt caagagagtc cccctggggt actctctttg cgcctatccg aacctctagt 15120
   tacctccaat ggcatgcttg cgctcaaaat gggcaacggc ctctctctgg acgaggccgg 15180
   caaccttacc tcccaaaatg taaccactgt gagcccacct ctcaaaaaaa ccaagtcaaa 15240
   cataaacctg gaaatatctg cacccctcac agttacctca gaagccctaa ctgtggctgc 15300
   cgccgcacct ctaatggtcg cgggcaacac actcaccatg caatcacagg ccccgctaac 15360
   cgtgcacgac tccaaactta gcattgccac ccaaggaccc ctcacagtgt cagaaggaaa 15420
   gctagccctg caaacatcag gccccctcac caccaccgat agcagtaccc ttactatcac 15480
   tgcctcaccc cctctaacta ctgccactgg tagcttgggc attgacttga aagagcccat 15540
   ttatacacaa aatggaaaac taggactaaa gtacggggct cctttgcatg taacagacga 15600
   cctaaacact ttgaccgtag caactggtcc aggtgtgact attaataata cttccttgca 15660
   aactaaagtt actggagcct tgggttttga ttcacaaggc aatatgcaac ttaatgtagc 15720
   aggaggacta aggattgatt ctcaaaacag acgccttata cttgatgtta gttatccgtt 15780
   tgatgctcaa aaccaactaa atctaagact aggacagggc cctcttttta taaactcagc 15840
   ccacaacttg gatattaact acaacaaagg cctttacttg tttacagctt caaacaattc 15900
   caaaaagctt gaggttaacc taagcactgc caaggggttg atgtttgacg ctacagccat 15960
   agccattaat gcaggagatg ggcttgaatt tggttcacct aatgcaccaa acacaaatcc 16020
   cctcaaaaca aaaattggcc atggcctaga atttgattca aacaaggcta tggttcctaa 16080
   actaggaact ggccttagtt ttgacagcac aggtgccatt acagtaggaa acaaaaataa 16140
   tgataagcta actttgtgga ccacaccagc tccatctcct aactgtagac taaatgcaga 16200
   gaaagatgct aaactcactt tggtcttaac aaaatgtggc agtcaaatac ttgctacagt 16260
   ttcagttttg gctgttaaag gcagtttggc tccaatatct ggaacagttc aaagtgctca 16320
   tcttattata agatttgacg aaaatggagt gctactaaac aattccttcc tggacccaga 16380
   atattggaac tttagaaatg gagatcttac tgaaggcaca gcctatacaa acgctgttgg 16440
   atttatgcct aacctatcag cttatccaaa atctcacggt aaaactgcca aaagtaacat 16500
   tgtcagtcaa gtttacttaa acggagacaa aactaaacct gtaacactaa ccattacact 16560
   aaacggtaca caggaaacag gagacacaac tccaagtgca tactctatgt cattttcatg 16620
   ggactggtct ggccacaact acattaatga aatatttgcc acatcctctt acactttttc 16680
   atacattgcc caagaataaa gaatcgtttg tgttatgttt caacgtgttt atttttcaat 16740
   tgcagaaaat ttcaagtcat ttttcattca gtagtatagc cccaccacca catagcttat 16800
   acagatcacc gtaccttaat caaactcaca gaaccctagt attcaacctg ccacctccct 16860
   cccaacacac agagtacaca gtcctttctc cccggctggc cttaaaaagc atcatatcat 16920
   gggtaacaga catattctta ggtgttatat tccacacggt ttcctgtcga gccaaacgct 16980
   catcagtgat attaataaac tccccgggca gctcacttaa gttcatgtcg ctgtccagct 17040
   gctgagccac aggctgctgt ccaacttgcg gttgcttaac gggcggcgaa ggagaagtcc 17100
   acgcctacat gggggtagag tcataatcgt gcatcaggat agggcggtgg tgctgcagca 17160
   gcgcgcgaat aaactgctgc cgccgccgct ccgtcctgca ggaatacaac atggcagtgg 17220
   tctcctcagc gatgattcgc accgcccgca gcataaggcg ccttgtcctc cgggcacagc 17280
   agcgcaccct gatctcactt aaatcagcac agtaactgca gcacagcacc acaatattgt 17340
   tcaaaatccc acagtgcaag gcgctgtatc caaagctcat ggcggggacc acagaaccca 17400
   cgtggccatc ataccacaag cgcaggtaga ttaagtggcg acccctcata aacacgctgg 17460
   acataaacat tacctctttt ggcatgttgt aattcaccac ctcccggtac catataaacc 17520
   tctgattaaa catggcgcca tccaccacca tcctaaacca gctggccaaa acctgcccgc 17580
   cggctataca ctgcagggaa ccgggactgg aacaatgaca gtggagagcc caggactcgt 17640
   aaccatggat catcatgctc gtcatgatat caatgttggc acaacacagg cacacgtgca 17700
   tacacttcct caggattaca agctcctccc gcgttagaac catatcccag ggaacaaccc 17760
   attcctgaat cagcgtaaat cccacactgc agggaagacc tcgcacgtaa ctcacgttgt 17820
   gcattgtcaa agtgttacat tcgggcagca gcggatgatc ctccagtatg gtagcgcggg 17880
   tttctgtctc aaaaggaggt agacgatccc tactgtacgg agtgcgccga gacaaccgag 17940
   atcgtgttgg tcgtagtgtc atgccaaatg gaacgccgga cgtagtcata tttcctgaag 18000
   caaaaccagg tgcgggcgtg acaaacagat ctgcgtctcc ggtctcgccg cttagatcgc 18060
   tctgtgtagt agttgtagta tatccactct ctcaaagcat ccaggcgccc cctggcttcg 18120
   ggttctatgt aaactccttc atgcgccgct gccctgataa catccaccac cgcagaataa 18180
   gccacaccca gccaacctac acattcgttc tgcgagtcac acacgggagg agcgggaaga 18240
   gctggaagaa ccatgttttt ttttttattc caaaagatta tccaaaacct caaaatgaag 18300
   atctattaag tgaacgcgct cccctccggt ggcgtggtca aactctacag ccaaagaaca 18360
   gataatggca tttgtaagat gttgcacaat ggcttccaaa aggcaaacgg ccctcacgtc 18420
   caagtggacg taaaggctaa acccttcagg gtgaatctcc tctataaaca ttccagcacc 18480
   ttcaaccatg cccaaataat tctcatctcg ccaccttctc aatatatctc taagcaaatc 18540
   ccgaatatta agtccggcca ttgtaaaaat ctgctccaga gcgccctcca ccttcagcct 18600
   caagcagcga atcatgattg caaaaattca ggttcctcac agacctgtat aagattcaaa 18660
   agcggaacat taacaaaaat accgcgatcc cgtaggtccc ttcgcagggc cagctgaaca 18720
   taatcgtgca ggtctgcacg gaccagcgcg gccacttccc cgccaggaac catgacaaaa 18780
   gaacccacac tgattatgac acgcatactc ggagctatgc taaccagcgt agccccgatg 18840
   taagcttgtt gcatgggcgg cgatataaaa tgcaaggtgc tgctcaaaaa atcaggcaaa 18900
   gcctcgcgca aaaaagaaag cacatcgtag tcatgctcat gcagataaag gcaggtaagc 18960
   tccggaacca ccacagaaaa agacaccatt tttctctcaa acatgtctgc gggtttctgc 19020
   ataaacacaa aataaaataa caaaaaaaca tttaaacatt agaagcctgt cttacaacag 19080
   gaaaaacaac ccttataagc ataagacgga ctacggccat gccggcgtga ccgtaaaaaa 19140
   actggtcacc gtgattaaaa agcaccaccg acagctcctc ggtcatgtcc ggagtcataa 19200
   tgtaagactc ggtaaacaca tcaggttgat tcacatcggt cagtgctaaa aagcgaccga 19260
   aatagcccgg gggaatacat acccgcaggc gtagagacaa cattacagcc cccataggag 19320
   gtataacaaa attaatagga gagaaaaaca cataaacacc tgaaaaaccc tcctgcctag 19380
   gcaaaatagc accctcccgc tccagaacaa catacagcgc ttccacagcg gcagccataa 19440
   cagtcagcct taccagtaaa aaagaaaacc tattaaaaaa acaccactcg acacggcacc 19500
   agctcaatca gtcacagtgt aaaaaagggc caagtgcaga gcgagtatat ataggactaa 19560
   aaaatgacgt aacggttaaa gtccacaaaa aacacccaga aaaccgcacg cgaacctacg 19620
   cccagaaacg aaagccaaaa aacccacaac ttcctcaaat cgtcacttcc gttttcccac 19680
   gttacgtcac ttcccatttt aagaaaacta caattcccaa cacatacaag ttactccgcc 19740
   ctaaaaccta cgtcacccgc cccgttccca cgccccgcgc cacgtcacaa actccacccc 19800
   ctcattatca tattggcttc aatccaaaat aaggtatatt attgatgatg ttaattaaga 19860
   attcggatct gcgacgcgag gctggatggc cttccccatt atgattcttc tcgcttccgg 19920
   cggcatcggg atgcccgcgt tgcaggccat gctgtccagg caggtagatg acgaccatca 19980
   gggacagctt cacggccagc aaaaggccag gaaccgtaaa aaggccgcgt tgctggcgtt 20040
   tttccatagg ctccgccccc ctgacgagca tcacaaaaat cgacgctcaa gtcagaggtg 20100
   gcgaaacccg acaggactat aaagatacca ggcgtttccc cctggaagct ccctcgtgcg 20160
   ctctcctgtt ccgaccctgc cgcttaccgg atacctgtcc gcctttctcc cttcgggaag 20220
   cgtggcgctt tctcatagct cacgctgtag gtatctcagt tcggtgtagg tcgttcgctc 20280
   caagctgggc tgtgtgcacg aaccccccgt tcagcccgac cgctgcgcct tatccggtaa 20340
   ctatcgtctt gagtccaacc cggtaagaca cgacttatcg ccactggcag cagccactgg 20400
   taacaggatt agcagagcga ggtatgtagg cggtgctaca gagttcttga agtggtggcc 20460
   taactacggc tacactagaa gaacagtatt tggtatctgc gctctgctga agccagttac 20520
   cttcggaaaa agagttggta gctcttgatc cggcaaacaa accaccgctg gtagcggtgg 20580
   tttttttgtt tgcaagcagc agattacgcg cagaaaaaaa ggatctcaag aagatccttt 20640
   gatcttttct acggggtctg acgctcagtg gaacgaaaac tcacgttaag ggattttggt 20700
   catgagatta tcaaaaagga tcttcaccta gatcctttta aatcaatcta aagtatatat 20760
   gagtaaactt ggtctgacag ttaccaatgc ttaatcagtg aggcacctat ctcagcgatc 20820
   tgtctatttc gttcatccat agttgcctga ctccccgtcg tgtagataac tacgatacgg 20880
   gagggcttac catctggccc cagtgctgca atgataccgc gagacccacg ctcaccggct 20940
   ccagatttat cagcaataaa ccagccagcc ggaagggccg agcgcagaag tggtcctgca 21000
   actttatccg cctccatcca gtctattaat tgttgccggg aagctagagt aagtagttcg 21060
   ccagttaata gtttgcgcaa cgttgttgcc attgctgcag gcatcgtggt gtcacgctcg 21120
   tcgtttggta tggcttcatt cagctccggt tcccaacgat caaggcgagt tacatgatcc 21180
   cccatgttgt gcaaaaaagc ggttagctcc ttcggtcctc cgatcgttgt cagaagtaag 21240
   ttggcagcag tgttatcact catggttatg gcagcactgc ataattctct tactgtcatg 21300
   ccatccgtaa gatgcttttc tgtgactggt gagtactcaa ccaagtcatt ctgagaatag 21360
   tgtatgcggc gaccgagttg ctcttgcccg gcgtcaacac gggataatac cgcaccacat 21420
   agcagaactt taaaagtgct catcattgga aaacgttctt cggggcgaaa actctcaagg 21480
   atcttaccgc tgttgagatc cagttcgatg taacccactc gtgcacccaa ctgatcttca 21540
   gcatctttta ctttcaccag cgtttctggg tgagcaaaaa caggaaggca aaatgccgca 21600
   aaaaagggaa taagggcgac acgaaaatgt tgaatactca tactcttcct ttttcaatat 21660
   tattgaagca tttatcaggg ttattgtctc atgagcggat acatatttga atgtatttag 21720
   aaaaataaac aaataggggt tccgcgcaca tttccccgaa aagtgccacc tgacgtctaa 21780
   gaaaccatta ttatcatgac attaacctat aaaaataggc gtatcacgag gccctttcgt 21840
   cttcaa 21846
<210> 3
   <211> 7905
   <212> DNA
   <213> Künstliche Sequenz
<220>
   <221> misc_feature
   <222> (4404) .. (4404)
   <223> n is a, c, g, or t
<220>
   <221> misc_feature
   <222> (5675) .. (5675)
   <223> n is a, c, g, or t
<400> 3
   aagcggaaga gcgcccaata cgcaaaccgc ctctccccgc gcgttggccg attcattaat        60
   gcagctggcg cgctcgctcg ctcactgagg ccgcccgggc aaagcccggg cgtcgggcga       120
   cctttggtcg cccggcctca gtgagcgagc gagcgcgcag agagggagtg gccaactcca      180
   tcactagggg ttccttgtag ttaatgatta acccgccatg ctacttatct acgtagccat      240
   gctctagtcg accaattctc atgtttgaca gcttatcatc gcagatccgg gcaacgttgt      300
   tgcattgctg caggcgcaga actggtaggt atggaagatc tatacattga atcaatattg      360
   gcaattagcc atattagtca ttggttatat agcataaatc aatattggct attggccatt      420
   gcatacgttg tatctatatc ataatatgta catttatatt ggctcatgtc caatatgacc      480
   gccatgttga cattgattat tgactagtta ttaatagtaa tcaattacgg ggtcattagt      540
   tcatagccca tatatggagt tccgcgttac ataacttacg gtaaatggcc cgcctggctg      600
   accgcccaac gacccccgcc cattgacgtc aataatgacg tatgttccca tagtaacgcc      660
   aatagggact ttccattgac gtcaatgggt ggagtattta cggtaaactg cccacttggc      720
   agtacatcaa gtgtatcata tgccaagtcc gccccctatt gacgtcaatg acggtaaatg      780
   gcccgcctgg cattatgccc agtacatgac cttacgggac tttcctactt ggcagtacat      840
   ctacgtatta gtcatcgcta ttaccatggt gatgcggttt tggcagtaca ccaatgggcg      900
   tggatagcgg tttgactcac ggggatttcc aagtctccac cccattgacg tcaatgggag      960
   tttgttttgg caccaaaatc aacgggactt tccaaaatgt cgtaataacc ccgccccgtt     1020
   gacgcaaatg ggcggtaggc gtgtacggtg ggaggtctat ataagcagag ctcgtttagt     1080
   gaaccgtcag atctctagaa gctgggtacc gcgggcccgg gatccaccgg tcgccaccat     1140
   ggtgagcaag ggcgaggagc tgttcaccgg ggtggtgccc atcctggtcg agctggacgg     1200
   cgacgtaaac ggccacaagt tcagcgtgtc cggcgagggc gagggcgatg ccacctacgg     1260
   caagctgacc ctgaagttca tctgcaccac cggcaagctg cccgtgccct ggcccaccct     1320
   cgtgaccacc ctgacctacg gcgtgcagtg cttcagccgc taccccgacc acatgaagca     1380
   gcacgacttc ttcaagtccg ccatgcccga aggctacgtc caggagcgca ccatcttctt     1440
   caaggacgac ggcaactaca agacccgcgc cgaggtgaag ttcgagggcg acaccctggt     1500
   gaaccgcatc gagctgaagg gcatcgactt caaggaggac ggcaacatcc tggggcacaa     1560
   gctggagtac aactacaaca gccacaacgt ctatatcatg gccgacaagc agaagaacgg     1620
   catcaaggtg aacttcaaga tccgccacaa catcgaggac ggcagcgtgc agctcgccga     1680
   ccactaccag cagaacaccc ccatcggcga cggccccgtg ctgctgcccg acaaccacta     1740
   cctgagcacc cagtccgccc tgagcaaaga ccccaacgag aagcgcgatc acatggtcct     1800
   gctggagttc gtgaccgccg ccgggatcac tctcggcatg gacgagctgt acaagtaaag     1860
   cggccgctcg aggccggcaa ggccggatcc agacatgata agatacattg atgagtttgg     1920
   acaaaccaca actagaatgc agtgaaaaaa atgctttatt tgtgaaattt gtgatgctat     1980
   tgctttattt gtaaccatta taagctgcaa taaacaagtt aacaacaaca attgcattca     2040
   ttttatgttt caggttcagg gggaggtggg gaggtttttt aaagcaagta aaacctctac 2100
   aaatgtggta tggctgatta tgatccggct gcctcgcgcg tttcggtgat gacggtgaaa 2160
   acctctgaca catgcagctc ccggagacgg tcacagcttg tctgtaagcg gatgccggga 2220
   gcagacaagc ccgtcagggc gcgtcagcgg gtgttggcgg gtgtcggggc gcagccatga 2280
   ccggtcgact agcttggcac gccagaaatc cgcgcggtgg tttttggggg tcgggggtgt 2340
   ttggcagcca cagacgcccg gtgttcgtgt cgcgccagta catgcggtcc atgcccaggc 2400
   catccaaaaa ccatgggtct gtctgctcag tccagtcgtg gaccagaccc cacgcaacgc 2460
   ccaaaataat aacccccacg aaccataaac cattccccat gggggacccc gtccctaacc 2520
   cacggggcca gtggctatgg cagggcctgc cgccccgacg ttggctgcga gccctgggcc 2580
   ttcacccgaa cttggggggt ggggtgggga aaaggaagaa acgcgggcgt attggcccca 2640
   atggggtctc ggtggggtat cgacagagtg ccagccctgg gaccgaaccc cgcgtttatg 2700
   aacaaacgac ccaacacccg tgcgttttat tctgtctttt tattgccgtc atagcgcggg 2760
   ttccttccgg tattgtctcc ttccgtgttt cagttagcct cccccatctc ccctattcct 2820
   ttgccctcgg acgagtgctg gggcgtcggt ttccactatc ggcgagtact tctacacagc 2880
   catcggtcca gacggccgcg cttctgcggg cgatttgtgt acgcccgaca gtcccggctc 2940
   cggatcggac gattgcgtcg catcgaccct gcgcccaagc tgcatcatcg aaattgccgt 3000
   caaccaagct ctgatagagt tggtcaagac caatgcggag catatacgcc cggagccgcg 3060
   gcgatcctgc aagctccgga tgcctccgct cgaagtagcg cgtctgctgc tccatacaag 3120
   ccaaccacgg cctccagaag aagatgttgg cgacctcgta ttgggaatcc ccgaacatcg 3180
   cctcgctcca gtcaatgacc gctgttatgc ggccattgtc cgtcaggaca ttgttggagc 3240
   cgaaatccgc gtgcacgagg tgccggactt cggggcagtc ctcggcccaa agcatcagct 3300
   catcgagagc ctgcgcgacg gacgcactga cggtgtcgtc catcacagtt tgccagtgat 3360
   acacatgggg atcagcaatc gcgcatatga aatcacgcca tgtagtgtat tgaccgattc 3420
   cttgcggtcc gaatgggccg aacccgctcg tctggctaag atcggccgca gcgatcgcat 3480
   ccatggcctc cgcgaccggc tgcagaacag cgggcagttc ggtttcaggc aggtcttgca 3540
   acgtgacacc ctgtgcacgg cgggagatgc aataggtcag gctctcgctg aattccccaa 3600
   tgtcaagcac ttccggaatc gggagcgcgg ccgatgcaaa gtgccgataa acataacgat 3660
   ctttgtagaa accatcggcg cagctattta cccgcaggac atatccacgc cctcctacat 3720
   cgaagctgaa agcacgagat tcttcgccct ccgagagctg catcaggtcg gagacgctgt 3780
   cgaacttttc gatcagaaac ttctcgacag acgtcgcggt gagttcaggc tttttcatat 3840
   ctcattgccc gggatctgcg gcacgctgtt gacgctgtta agcgggtcgc tgcagggtcg 3900
   ctcggtgttc gaggccacac gcgtcacctt aatatgcgaa gtggacctgg gaccgcgccg 3960
   ccccgactgc atctgcgtgt tcgaattcgc caatgacaag acgctgggcg gggtttgtgt 4020
   catcatagaa ctaaagacat gcaaatatat ttcttccggg gacaccgcca gcaaacgcga 4080
   gcaacgggcc acggggatga agcagggcat ggcggccgac gcgctgggct acgtcttgct 4140
   ggcgttcgct agagcatggc tacgtagata agtagcatgg cgggttaatc attaactaca 4200
   aggaacccct agtgatggag ttggccactc cctctctgcg cgctcgctcg ctcactgagg 4260
   ccgggcgacc aaaggtcgcc cgacgcccgg gctttgcccg ggcggcctca gtgagcgagc 4320
   gagcgcgcca gctggcgtaa tagcgaagag gcccgcaccg atcgcccttc ccaacagttg 4380
   cgcagcctga atggcgaatg gaanttccag acgattgagc gtcaaaatgt aggtatttcc 4440
   atgagcgttt ttcctgttgc aatggctggc ggtaatattg ttctggatat taccagcaag 4500
   gccgatagtt tgagttcttc tactcaggca agtgatgtta ttactaatca aagaagtatt 4560
   gcgacaacgg ttaatttgcg tgatggacag actcttttac tcggtggcct cactgattat 4620
   aaaaacactt ctcaggattc tggcgtaccg ttcctgtcta aaatcccttt aatcggcctc 4680
   ctgtttagct cccgctctga ttctaacgag gaaagcacgt tatacgtgct cgtcaaagca 4740
   accatagtac gcgccctgta gcggcgcatt aagcgcggcg ggtgtggtgg ttacgcgcag 4800
   cgtgaccgct acacttgcca gcgccctagc gcccgctcct ttcgctttct tcccttcctt 4860
   tctcgccacg ttcgccggct ttccccgtca agctctaaat cgggggctcc ctttagggtt 4920
   ccgatttagt gctttacggc acctcgaccc caaaaaactt gattagggtg atggttcacg 4980
   tagtgggcca tcgccctgat agacggtttt tcgccctttg acgttggagt ccacgttctt 5040
   taatagtgga ctcttgttcc aaactggaac aacactcaac cctatctcgg tctattcttt 5100
   tgatttataa gggattttgc cgatttcggc ctattggtta aaaaatgagc tgatttaaca 5160
   aaaatttaac gcgaatttta acaaaatatt aacgtttaca atttaaatat ttgcttatac 5220
   aatcttcctg tttttggggc ttttctgatt atcaaccggg gtacatatga ttgacatgct 5280
   agttttacga ttaccgttca tcgattctct tgtttgctcc agactctcag gcaatgacct 5340
   gatagccttt gtagagacct ctcaaaaata gctaccctct ccggcatgaa tttatcagct 5400
   agaacggttg aatatcatat tgatggtgat ttgactgtct ccggcctttc tcacccgttt 5460
   gaatctttac ctacacatta ctcaggcatt gcatttaaaa tatatgaggg ttctaaaaat 5520
   ttttatcctt gcgttgaaat aaaggcttct cccgcaaaag tattacaggg tcataatgtt 5580
   tttggtacaa ccgatttagc tttatgctct gaggctttat tgcttaattt tgctaattct 5640
   ttgccttgcc tgtatgattt attggatgtt ggaanttcct gatgcggtat tttctcctta 5700
   cgcatctgtg cggtatttca caccgcatat ggtgcactct cagtacaatc tgctctgatg 5760
   ccgcatagtt aagccagccc cgacacccgc caacacccgc tgacgcgccc tgacgggctt 5820
   gtctgctccc ggcatccgct tacagacaag ctgtgaccgt ctccgggagc tgcatgtgtc 5880
   agaggttttc accgtcatca ccgaaacgcg cgagacgaaa gggcctcgtg atacgcctat 5940
   ttttataggt taatgtcatg ataataatgg tttcttagac gtcaggtggc acttttcggg 6000
   gaaatgtgcg cggaacccct atttgtttat ttttctaaat acattcaaat atgtatccgc 6060
   tcatgagaca ataaccctga taaatgcttc aataatattg aaaaaggaag agtatgagta 6120
   ttcaacattt ccgtgtcgcc cttattccct tttttgcggc attttgcctt cctgtttttg 6180
   ctcacccaga aacgctggtg aaagtaaaag atgctgaaga tcagttgggt gcacgagtgg 6240
   gttacatcga actggatctc aacagcggta agatccttga gagttttcgc cccgaagaac 6300
   gttttccaat gatgagcact tttaaagttc tgctatgtgg cgcggtatta tcccgtattg 6360
   acgccgggca agagcaactc ggtcgccgca tacactattc tcagaatgac ttggttgagt 6420
   actcaccagt cacagaaaag catcttacgg atggcatgac agtaagagaa ttatgcagtg 6480
   ctgccataac catgagtgat aacactgcgg ccaacttact tctgacaacg atcggaggac 6540
   cgaaggagct aaccgctttt ttgcacaaca tgggggatca tgtaactcgc cttgatcgtt 6600
   gggaaccgga gctgaatgaa gccataccaa acgacgagcg tgacaccacg atgcctgtag 6660
   caatggcaac aacgttgcgc aaactattaa ctggcgaact acttactcta gcttcccggc 6720
   aacaattaat agactggatg gaggcggata aagttgcagg accacttctg cgctcggccc 6780
   ttccggctgg ctggtttatt gctgataaat ctggagccgg tgagcgtggg tctcgcggta 6840
   tcattgcagc actggggcca gatggtaagc cctcccgtat cgtagttatc tacacgacgg 6900
   ggagtcaggc aactatggat gaacgaaata gacagatcgc tgagataggt gcctcactga 6960
   ttaagcattg gtaactgtca gaccaagttt actcatatat actttagatt gatttaaaac 7020
   ttcattttta atttaaaagg atctaggtga agatcctttt tgataatctc atgaccaaaa 7080
   tcccttaacg tgagttttcg ttccactgag cgtcagaccc cgtagaaaag atcaaaggat 7140
   cttcttgaga tccttttttt ctgcgcgtaa tctgctgctt gcaaacaaaa aaaccaccgc 7200
   taccagcggt ggtttgtttg ccggatcaag agctaccaac tctttttccg aaggtaactg 7260
   gcttcagcag agcgcagata ccaaatactg tccttctagt gtagccgtag ttaggccacc 7320
   acttcaagaa ctctgtagca ccgcctacat acctcgctct gctaatcctg ttaccagtgg 7380
   ctgctgccag tggcgataag tcgtgtctta ccgggttgga ctcaagacga tagttaccgg 7440
   ataaggcgca gcggtcgggc tgaacggggg gttcgtgcac acagcccagc ttggagcgaa 7500
   cgacctacac cgaactgaga tacctacagc gtgagctatg agaaagcgcc acgcttcccg 7560
   aagggagaaa ggcggacagg tatccggtaa gcggcagggt cggaacagga gagcgcacga 7620
   gggagcttcc agggggaaac gcctggtatc tttatagtcc tgtcgggttt cgccacctct 7680
   gacttgagcg tcgatttttg tgatgctcgt caggggggcg gagcctatgg aaaaacgcca     7740
   gcaacgcggc ctttttacgg ttcctggcct tttgctggcc ttttgctcac atgttctttc     7800
   ctgcgttatc ccctgattct gtggataacc gtattaccgc ctttgagtga gctgataccg     7860
   ctcgccgcag ccgaacgacc gagcgcagcg agtcagtgag cgagg                     7905
<210> 4
   <211> 5964
   <212> DNA
   <213> Künstliche Sequenz
<220>
   <221> misc_feature
   <222> (2395) .. (2395)
   <223> n is a, c, g, or t
<220>
   <221> misc_feature
   <222> (3666) .. (3666)
   <223> n is a, c, g, or t
<400> 4
   cgcgctcgct cgctcactga ggccgcccgg gcaaagcccg ggcgtcgggc gacctttggt      60
   cgcccggcct cagtgagcga gcgagcgcgc agagagggag tgggtcgttc tagtcgacca     120
   attctcatgt ttgacagctt atcatcgcag atccgggcaa cgttgttgca ttgctgcagg     180
   cgcagaactg gtaggtatgg aagatctata cattgaatca atattggcaa ttagccatat     240
   tagtcattgg ttatatagca taaatcaata ttggctattg gccattgcat acgttgtatc     300
   tatatcataa tatgtacatt tatattggct catgtccaat atgaccgcca tgttgacatt     360
   gattattgac tagttattaa tagtaatcaa ttacggggtc attagttcat agcccatata     420
   tggagttccg cgttacataa cttacggtaa atggcccgcc tggctgaccg cccaacgacc     480
   cccgcccatt gacgtcaata atgacgtatg ttcccatagt aacgccaata gggactttcc     540
   attgacgtca atgggtggag tatttacggt aaactgccca cttggcagta catcaagtgt     600
   atcatatgcc aagtccgccc cctattgacg tcaatgacgg taaatggccc gcctggcatt     660
   atgcccagta catgacctta cgggactttc ctacttggca gtacatctac gtattagtca     720
   tcgctattac catggtgatg cggttttggc agtacaccaa tgggcgtgga tagcggtttg     780
   actcacgggg atttccaagt ctccacccca ttgacgtcaa tgggagtttg ttttggcacc     840
   aaaatcaacg ggactttcca aaatgtcgta ataaccccgc cccgttgacg caaatgggcg     900
   gtaggcgtgt acggtgggag gtctatataa gcagagctcg tttagtgaac cgtcagatct     960
   ctagaagctg ggtaccgcgg gcccgggatc caccggtcgc caccatggtg agcaagggcg    1020
   aggagctgtt caccggggtg gtgcccatcc tggtcgagct ggacggcgac gtaaacggcc    1080
   acaagttcag cgtgtccggc gagggcgagg gcgatgccac ctacggcaag ctgaccctga    1140
   agttcatctg caccaccggc aagctgcccg tgccctggcc caccctcgtg accaccctga 1200
   cctacggcgt gcagtgcttc agccgctacc ccgaccacat gaagcagcac gacttcttca 1260
   agtccgccat gcccgaaggc tacgtccagg agcgcaccat cttcttcaag gacgacggca 1320
   actacaagac ccgcgccgag gtgaagttcg agggcgacac cctggtgaac cgcatcgagc 1380
   tgaagggcat cgacttcaag gaggacggca acatcctggg gcacaagctg gagtacaact 1440
   acaacagcca caacgtctat atcatggccg acaagcagaa gaacggcatc aaggtgaact 1500
   tcaagatccg ccacaacatc gaggacggca gcgtgcagct cgccgaccac taccagcaga 1560
   acacccccat cggcgacggc cccgtgctgc tgcccgacaa ccactacctg agcacccagt 1620
   ccgccctgag caaagacccc aacgagaagc gcgatcacat ggtcctgctg gagttcgtga 1680
   ccgccgccgg gatcactctc ggcatggacg agctgtacaa gtaaagcggc cgctcgaggc 1740
   cggcaaggcc ggatccagac atgataagat acattgatga gtttggacaa accacaacta 1800
   gaatgcagtg aaaaaaatgc tttatttgtg aaatttgtga tgctattgct ttatttgtaa 1860
   ccattataag ctgcaataaa caagttaaca acaacaattg cattcatttt atgtttcagg 1920
   ttcaggggga ggtggggagg ttttttaaag caagtaaaac ctctacaaat gtggtatggc 1980
   tgattatgat ccggctgcct cgcgcgtttc ggtgatgacg gtgaaaacct ctgacacatg 2040
   cagctcccgg agacggtcac agcttgtctg taagcggatg ccgggagcag acaagcccgt 2100
   cagggcgcgt cagcgggtgt tggcgggtgt cggggcgcag ccatgaccgg tcgagtagat 2160
   aagtagcatg gcgggttaat cattaactac aaggaacccc tagtgatgga gttggccact 2220
   ccctctctgc gcgctcgctc gctcactgag gccgggcgac caaaggtcgc ccgacgcccg 2280
   ggctttgccc gggcggcctc agtgagcgag cgagcgcgcc agctggcgta atagcgaaga 2340
   ggcccgcacc gatcgccctt cccaacagtt gcgcagcctg aatggcgaat ggaanttcca 2400
   gacgattgag cgtcaaaatg taggtatttc catgagcgtt tttcctgttg caatggctgg 2460
   cggtaatatt gttctggata ttaccagcaa ggccgatagt ttgagttctt ctactcaggc 2520
   aagtgatgtt attactaatc aaagaagtat tgcgacaacg gttaatttgc gtgatggaca 2580
   gactctttta ctcggtggcc tcactgatta taaaaacact tctcaggatt ctggcgtacc 2640
   gttcctgtct aaaatccctt taatcggcct cctgtttagc tcccgctctg attctaacga 2700
   ggaaagcacg ttatacgtgc tcgtcaaagc aaccatagta cgcgccctgt agcggcgcat 2760
   taagcgcggc gggtgtggtg gttacgcgca gcgtgaccgc tacacttgcc agcgccctag 2820
   cgcccgctcc tttcgctttc ttcccttcct ttctcgccac gttcgccggc tttccccgtc 2880
   aagctctaaa tcgggggctc cctttagggt tccgatttag tgctttacgg cacctcgacc 2940
   ccaaaaaact tgattagggt gatggttcac gtagtgggcc atcgccctga tagacggttt 3000
   ttcgcccttt gacgttggag tccacgttct ttaatagtgg actcttgttc caaactggaa 3060
   caacactcaa ccctatctcg gtctattctt ttgatttata agggattttg ccgatttcgg 3120
   cctattggtt aaaaaatgag ctgatttaac aaaaatttaa cgcgaatttt aacaaaatat 3180
   taacgtttac aatttaaata tttgcttata caatcttcct gtttttgggg cttttctgat 3240
   tatcaaccgg ggtacatatg attgacatgc tagttttacg attaccgttc atcgattctc 3300
   ttgtttgctc cagactctca ggcaatgacc tgatagcctt tgtagagacc tctcaaaaat 3360
   agctaccctc tccggcatga atttatcagc tagaacggtt gaatatcata ttgatggtga 3420
   tttgactgtc tccggccttt ctcacccgtt tgaatcttta cctacacatt actcaggcat 3480
   tgcatttaaa atatatgagg gttctaaaaa tttttatcct tgcgttgaaa taaaggcttc 3540
   tcccgcaaaa gtattacagg gtcataatgt ttttggtaca accgatttag ctttatgctc 3600
   tgaggcttta ttgcttaatt ttgctaattc tttgccttgc ctgtatgatt tattggatgt 3660
   tggaanttcc tgatgcggta ttttctcctt acgcatctgt gcggtatttc acaccgcata 3720
   tggtgcactc tcagtacaat ctgctctgat gccgcatagt taagccagcc ccgacacccg 3780
   ccaacacccg ctgacgcgcc ctgacgggct tgtctgctcc cggcatccgc ttacagacaa 3840
   gctgtgaccg tctccgggag ctgcatgtgt cagaggtttt caccgtcatc accgaaacgc 3900
   gcgagacgaa agggcctcgt gatacgccta tttttatagg ttaatgtcat gataataatg 3960
   gtttcttaga cgtcaggtgg cacttttcgg ggaaatgtgc gcggaacccc tatttgttta 4020
   tttttctaaa tacattcaaa tatgtatccg ctcatgagac aataaccctg ataaatgctt 4080
   caataatatt gaaaaaggaa gagtatgagt attcaacatt tccgtgtcgc ccttattccc 4140
   ttttttgcgg cattttgcct tcctgttttt gctcacccag aaacgctggt gaaagtaaaa 4200
   gatgctgaag atcagttggg tgcacgagtg ggttacatcg aactggatct caacagcggt 4260
   aagatccttg agagttttcg ccccgaagaa cgttttccaa tgatgagcac ttttaaagtt 4320
   ctgctatgtg gcgcggtatt atcccgtatt gacgccgggc aagagcaact cggtcgccgc 4380
   atacactatt ctcagaatga cttggttgag tactcaccag tcacagaaaa gcatcttacg 4440
   gatggcatga cagtaagaga attatgcagt gctgccataa ccatgagtga taacactgcg 4500
   gccaacttac ttctgacaac gatcggagga ccgaaggagc taaccgcttt tttgcacaac 4560
   atgggggatc atgtaactcg ccttgatcgt tgggaaccgg agctgaatga agccatacca 4620
   aacgacgagc gtgacaccac gatgcctgta gcaatggcaa caacgttgcg caaactatta 4680
   actggcgaac tacttactct agcttcccgg caacaattaa tagactggat ggaggcggat 4740
   aaagttgcag gaccacttct gcgctcggcc cttccggctg gctggtttat tgctgataaa 4800
   tctggagccg gtgagcgtgg gtctcgcggt atcattgcag cactggggcc agatggtaag 4860
   ccctcccgta tcgtagttat ctacacgacg gggagtcagg caactatgga tgaacgaaat 4920
   agacagatcg ctgagatagg tgcctcactg attaagcatt ggtaactgtc agaccaagtt 4980
   tactcatata tactttagat tgatttaaaa cttcattttt aatttaaaag gatctaggtg 5040
   aagatccttt ttgataatct catgaccaaa atcccttaac gtgagttttc gttccactga 5100
   gcgtcagacc ccgtagaaaa gatcaaagga tcttcttgag atcctttttt tctgcgcgta 5160
   atctgctgct tgcaaacaaa aaaaccaccg ctaccagcgg tggtttgttt gccggatcaa 5220
   gagctaccaa ctctttttcc gaaggtaact ggcttcagca gagcgcagat accaaatact 5280
   gtccttctag tgtagccgta gttaggccac cacttcaaga actctgtagc accgcctaca 5340
   tacctcgctc tgctaatcct gttaccagtg gctgctgcca gtggcgataa gtcgtgtctt 5400
   accgggttgg actcaagacg atagttaccg gataaggcgc agcggtcggg ctgaacgggg 5460
   ggttcgtgca cacagcccag cttggagcga acgacctaca ccgaactgag atacctacag 5520
   cgtgagctat gagaaagcgc cacgcttccc gaagggagaa aggcggacag gtatccggta 5580
   agcggcaggg tcggaacagg agagcgcacg agggagcttc cagggggaaa cgcctggtat 5640
   ctttatagtc ctgtcgggtt tcgccacctc tgacttgagc gtcgattttt gtgatgctcg 5700
   tcaggggggc ggagcctatg gaaaaacgcc agcaacgcgg cctttttacg gttcctggcc 5760
   ttttgctggc cttttgctca catgttcttt cctgcgttat cccctgattc tgtggataac 5820
   cgtattaccg cctttgagtg agctgatacc gctcgccgca gccgaacgac cgagcgcagc 5880
   gagtcagtga gcgaggaagc ggaagagcgc ccaatacgca aaccgcctct ccccgcgcgt 5940
   tggccgattc attaatgcag ctgg 5964
<210> 5
   <211> 21529
   <212> DNA
   <213> Künstliche Sequenz
<220>
   <221> misc_feature
   <222> (20778) .. (20778)
   <223> n is a, c, g, or t
<400> 5
   ggatccgaat tcttaattaa catcatcaat aatatacctt attttggatt gaagccaata        60
   tgataatgag ggggtggagt ttgtgacgtg gcgcggggcg tgggaacggg gcgggtgacg       120
   tagtagtgtg gcggaagtgt gatgttgcaa gtgtggcgga acacatgtaa gcgacggatg       180
   tggcaaaagt gacgtttttg gtgtgcgccg gtgtacacag gaagtgacaa ttttcgcgcg       240
   gttttaggcg gatgttgtag taaatttggg cgtaaccgag taagatttgg ccattttcgc       300
   gggaaaactg aataagagga agtgaaatct gaataatttt gtgttactca tagcgcgtaa       360
   tatttgtcta gggccgcggg gactttgacc gtttacgtgg agactcgccc aggtgttttt       420
   ctcaggtgtt ttccgcgttc cgggtcaaag ttggcgtttt attattatag tcagggggat 480
   cctctagaac tagtggatct tggccactcc ctctctgcgc gctcgctcgc tcactgaggc 540
   cgcccgggca aagcccgggc gtcgggcgac ctttggtcgc ccggcctcag tgagcgagcg 600
   agcgcgcaga gagggagtgg ccaactccat cactaggggt tccttgtagt taatgattaa 660
   cccgccatgc tacttatcta cgtagccatg gaaactagat aagaaagaaa tacgcagaga 720
   ccaaagttca actgaaacga attaaacggt ttattgatta acaagcaatt acagattacg 780
   agtcaggtat ctggtgccaa tggggcgagg ctctgaatac acgccattag tatccacggt 840
   aagtccacga ttaacagact tgttgtagtt ggaagtgtac tgaatttcgg gattccagcg 900
   tttgctgttt tccttctgca gctcccactc gatctccacg ctgaccgtgt cccgtggagt 960
   actgtgtgat gaaggaagca aactttgccg cactgaaggt ggtcgaagga ttcgcaggta 1020
   ccggggtgtt cttgatgaga atctgtggag gagggtgttt aagtccgaat ccacccatga 1080
   ggggagaggg gtgaaaatgt ccgtccgtgt gtggaatctt tgcccagatg ggcccctgaa 1140
   ggtacacatc tctgtcctgc cagaccatgc ctggaagaac gccttgtgtg ttgacatctg 1200
   cggtagctgc ttgtctgttg cctctctgga ggttggtaga tacagaacca tactgctccg 1260
   tagccacggg attggttgtt ccgatttcct cttcgtctgt aatcatgacc ttttcaatgt 1320
   tcacatttgt tttctctgag ccttgcttcc caaagatgag aaccccgctc tgaggaaaaa 1380
   acttttcttc atcgtccttg tggcttgcca tggccggatt caccagagag tctctgccat 1440
   tgaggtggta cttggtagct ccagtccacg agtattcact gttgttgtta tccgcagatg 1500
   tctttgatac tcgctgctgg cggtaacagg gtccaggaag ccagttccta gactggtccc 1560
   gaatgtcact cgctccggcc tgagaaaact gaagccttga ctgcgtggtg gttccacttg 1620
   gagtgtttgt tctgctcaag taatacaggt actggtcgat gagaggattc atgagacggt 1680
   ccagactctg gctgtgagcg tagctgctgt ggaaaggaac gtcctcaaaa gtgtagctga 1740
   aggtaaagtt gtttccggta cgcagcatct gagaaggaaa gtactccagg cagtaaaatg 1800
   aagagcgtcc tactgcctga ctcccgttgt tcagggtgag gtatccatac tgtggcacca 1860
   tgaagacgtc tgctgggaac ggcgggaggc atccttgatg cgccgagccg aggacgtacg 1920
   ggagctggta ctccgagtca gtaaacacct gaaccgtgct ggtaaggtta ttggcaatcg 1980
   tcgtcgtacc gtcattctgc gtgacctctt tgacttgaat gttaaagagc ttgaagttga 2040
   gtctcttggg tcggaatccc cagttgttgt tgatgagtct ttgccagtca cgtggtgaaa 2100
   agtggcagtg gaatctgttg aagtcaaaat acccccaagg ggtgctgtag ccaaagtagt 2160
   gattgtcgtt cgaggctcct gattggctgg aaatttgttt gtagaggtgg ttgttgtagg 2220
   tgggcagggc ccaggttcgg gtgctggtgg tgatgactct gtcgcccatc catgtggaat 2280
   cgcaatgcca atttccggag gaattaccca ctccgtcggc gccctcgtta ttgtctgcca 2340
   ttggtgcgcc actgcctgta gccatcgtat tagttcccag accagagggg gctgctggtg 2400
   gctgtccgag aggctggggg tcaggtactg agtctgcgtc tccagtctga ccaaaattca 2460
   atctttttct tgcaggctgc tggcccgcct ttccggttcc cgaggaggag tctggctcca 2520
   caggagagtg ctctaccggc ctcttttttc ccggagccgt cttaacaggt tcctcaacca 2580
   ggcccagagg ttcaagaacc ctctttttcg cctggaagac tgctcgtccg aggttgcccc 2640
   caaaagacgt atcttcttta aggcgctcct gaaactccgc gtcggcgtgg ttgtacttga 2700
   ggtacgggtt gtctccgctg tcgagctgcc ggtcgtaggc tttgtacgtg ctcgagggcc 2760
   gcggcgtctg cctcgttgac cggctctccc ttgtcgagtc cgttgaaggg tccgaggtac 2820
   ttgtacccag gaagcacaag acccctgctg tcgtccttat gccgctctgc gggctttggt 2880
   ggtggtgggc caggtttgag cttccaccac tgtcttattc cttcagagag agtgtcctcg 2940
   agccaatctg gaagataacc atcggcagcc atacctgatt taaatcattt attgttcaaa 3000
   gatgcagtca tccaaatcca cattgaccag atcgcaggca gtgcaagcgt ctggcacctt 3060
   tcccatgata tgatgaatgt agcacagttt ctgatacgcc tttttgacga cagaaacggg 3120
   ttgagattct gacacgggaa agcactctaa acagtctttc tgtccgtgag tgaagcagat 3180
   atttgaattc tgattcattc tctcgcattg tctgcaggga aacagcatca gattcatgcc 3240
   cacgtgacga gaacatttgt tttggtacct gtctgcgtag ttgatcgaag cttccgcgtc 3300
   tgacgtcgat ggctgcgcaa ctgactcgcg cacccgtttg ggctcactta tatctgcgtc 3360
   actgggggcg ggtcttttct tggctccacc ctttttgacg tagaattcat gctccacctc 3420
   aaccacgtga tcctttgccc accggaaaaa gtctttgact tcctgcttgg tgaccttccc 3480
   aaagtcatga tccagacggc gggtgagttc aaatttgaac atccggtctt gcaacggctg 3540
   ctggtgttcg aaggtcgttg agttcccgtc aatcacggcg cacatgttgg tgttggaggt 3600
   gacgatcacg ggagtcgggt ctatctgggc cgaggacttg catttctggt ccacgcgcac 3660
   cttgcttcct ccgagaatgg ctttggccga ctccacgacc ttggcggtca tcttcccctc 3720
   ctcccaccag atcaccatct tgtcgacaca gtcgttgaag ggaaagttct cattggtcca 3780
   gtttacgcac ccgtagaagg gcacagtgtg ggctatggcc tccgcgatgt tggtcttccc 3840
   ggtagttgca ggcccaaaca gccagatggt gttcctcttg ccgaactttt tcgtggccca 3900
   tcccagaaag acggaagccg catattgggg atcgtacccg tttagttcca aaattttata 3960
   aatccgattg ctggaaatgt cctccacggg ctgctggccc accaggtagt cgggggcggt 4020
   tttagtcagg ctcataatct ttcccgcatt gtccaaggca gccttgattt gggaccgcga 4080
   gttggaggcc gcattgaagg agatgtatga ggcctggtcc tcctggatcc actgcttctc 4140
   cgaggtaatc cccttgtcca cgagccaccc gaccagctcc atgtacctgg ctgaagtttt 4200
   tgatctgatc accggcgcat cagaattggg attctgattc tctttgttct gctcctgcgt 4260
   ctgcgacacg tgcgtcagat gctgcgccac caaccgttta cgctccgtga gattcaaaca 4320
   ggcgcttaaa tactgttcca tattagtcca cgcccactgg agctcaggct gggttttggg 4380
   gagcaagtaa ttggggatgt agcactcatc caccaccttg ttcccgcctc cggcgccatt 4440
   tctggtcttt gtgaccgcga accagtttgg caaagtcggc tcgatcccgc ggtaaattct 4500
   ctgaatcagt ttttcgcgaa tctgactcag gaaacgtccc aaaaccatgg atttcacccc 4560
   ggtggtttcc acgagcacgt gcatgtggaa gtagctctct cccttctcaa attgcacaaa 4620
   gaaaagggcc tccggggcct tactcacacg gcgccattcc gtcagaaagt cgcgctgcag 4680
   cttctcggcc acggtcaggg gtgcctgctc aatcagattc agatccatgt cagaatctgg 4740
   cggcaactcc cattccttct cggccaccca gttcacaaag ctgtcagaaa tgccgggcag 4800
   atgcccgtca aggtcgctgg ggaccttaat cacaatctcg taaaaccccg gcatggcggc 4860
   tgcgcgttca aacctcccgc ttcaaaatgg agaccctgcg tgctcactcg ggcttaaata 4920
   cccagcgtga ccacatggtg tcgcaaaatg tcgcaaaaca ctcacgtgac ctctaataca 4980
   ggacctccct aaccctatga cgtaattcac gtcacgactc cacccctcca ggaaccccta 5040
   gtgatggagt tggccactcc ctctctgcgc gctcgctcgc tcactgaggc cgcccgggca 5100
   aagcccgggc gtcgggcgac ctttggtcgc ccggcctcag tgagcgagcg agcgcgcaga 5160
   gagggagtgg ccaagatccc ccgggctgca ggaattcgat atcaagctta tcgatgcatg 5220
   tccttgggtc cggcctgctg aatgcgcagg cggtcggcca tgccccaggc ttcgttttga 5280
   catcggcgca ggtctttgta gtagtcttgc atgagccttt ctaccggcac ttcttcttct 5340
   ccttcctctt gtcctgcatc tcttgcatct atcgctgcgg cggcggcgga gtttggccgt 5400
   aggtggcgcc ctcttcctcc catgcgtgtg accccgaagc ccctcatcgg ctgaagcagg 5460
   gctaggtcgg cgacaacgcg ctcggctaat atggcctgct gcacctgcgt gagggtagac 5520
   tggaagtcat ccatgtccac aaagcggtgg tatgcgcccg tgttgatggt gtaagtgcag 5580
   ttggccataa cggaccagtt aacggtctgg tgacccggct gcgagagctc ggtgtacctg 5640
   agacgcgagt aagccctcga gtcaaatacg tagtcgttgc aagtccgcac caggtactgg 5700
   tatcccacca aaaagtgcgg cggcggctgg cggtagaggg gccagcgtag ggtggccggg 5760
   gctccggggg cgagatcttc caacataagg cgatgatatc cgtagatgta cctggacatc 5820
   caggtgatgc cggcggcggt ggtggaggcg cgcggaaagt cgcggacgcg gttccagatg 5880
   ttgcgcagcg gcaaaaagtg ctccatggtc gggacgctct ggccggtcag gcgcgcgcaa 5940
   tcgttgacgc tctagaccgt gcaaaaggag agcctgtaag cgggcactct tccgtggtct 6000
   ggtggataaa ttcgcaaggg tatcatggcg gacgaccggg gttcgagccc cgtatccggc 6060
   cgtccgccgt gatccatgcg gttaccgccc gcgtgtcgaa cccaggtgtg cgacgtcaga 6120
   caacggggga gtgctccttt tggcttcctt ccaggcgcgg cggctgctgc gctagctttt 6180
   ttggccactg gccgcgcgca gcgtaagcgg ttaggctgga aagcgaaagc attaagtggc 6240
   tcgctccctg tagccggagg gttattttcc aagggttgag tcgcgggacc cccggttcga 6300
   gtctcggacc ggccggactg cggcgaacgg gggtttgcct ccccgtcatg caagaccccg 6360
   cttgcaaatt cctccggaaa cagggacgag cccctttttt gcttttccca gatgcatccg 6420
   gtgctgcggc agatgcgccc ccctcctcag cagcggcaag agcaagagca gcggcagaca 6480
   tgcagggcac cctcccctcc tcctaccgcg tcaggagggg cgacatccgc ggttgacgcg 6540
   gcagcagatg gtgattacga acccccgcgg cgccgggccc ggcactacct ggacttggag 6600
   gagggcgagg gcctggcgcg gctaggagcg ccctctcctg agcggtaccc aagggtgcag 6660
   ctgaagcgtg atacgcgtga ggcgtacgtg ccgcggcaga acctgtttcg cgaccgcgag 6720
   ggagaggagc ccgaggagat gcgggatcga aagttccacg cagggcgcga gctgcggcat 6780
   ggcctgaatc gcgagcggtt gctgcgcgag gaggactttg agcccgacgc gcgaaccggg 6840
   attagtcccg cgcgcgcaca cgtggcggcc gccgacctgg taaccgcata cgagcagacg 6900
   gtgaaccagg gcgatcgcac cctttggcgc atcccattct ccagtaactt tatgtccatg 6960
   ggcgcactca cagacctggg ccaaaacctt ctctacgcca actccgccca cgcgctagac 7020
   atgacttttg aggtggatcc catggacgag cccacccttc tttatgtttt gtttgaagtc 7080
   tttgacgtgg tccgtgtgca ccggccgcac cgcggcgtca tcgaaaccgt gtacctgcgc 7140
   acgcccttct cggccggcaa cgccacaaca taaagaagca agcaacatca acaacagctg 7200
   ccgccatggg ctccagtgag caggaactga aagccattgt caaagatctt ggttgtgggc 7260
   catatttttt gggcacctat gacaagcgct ttccaggctt tgtttctcca cacaagctcg 7320
   cctgcgccat agtcaatacg gccggtcgcg agactggggg cgtacactgg atggcctttg 7380
   cctggaaccc gcactcaaaa acatgctacc tctttgagcc ctttggcttt tctgaccagc 7440
   gactcaagca ggtttaccag tttgagtacg agtcactcct gcgccgtagc gccattgctt 7500
   cttcccccga ccgctgtata acgctggaaa agtccaccca aagcgtacag gggcccaact 7560
   cggccgcctg tggactattc tgctgcatgt ttctccacgc ctttgccaac tggccccaaa 7620
   ctcccatgga tcacaacccc accatgaacc ttattaccgg ggtacccaac tccatgctca 7680
   acagtcccca ggtacagccc accctgcgtc gcaaccagga acagctctac agcttcctgg 7740
   agcgccactc gccctacttc cgcagccaca gtgcgcagat taggagcgcc acttcttttt 7800
   gtcacttgaa aaacatgtaa aaataatgta ctagagacac tttcaataaa ggcaaatgct 7860
   tttatttgta cactctcggg tgattattta cccccaccct tgccgtctgc gccgtttaaa 7920
   aatcaaaggg gttctgccgc gcatcgctat gcgccactgg cagggacacg ttgcgatact 7980
   ggtgtttagt gctccactta aactcaggca caaccatccg cggcagctcg gtgaagtttt 8040
   cactccacag gctgcgcacc atcaccaacg cgtttagcag gtcgggcgcc gatatcttga 8100
   agtcgcagtt ggggcctccg ccctgcgcgc gcgagttgcg atacacaggg ttgcagcact 8160
   ggaacactat cagcgccggg tggtgcacgc tggccagcac gctcttgtcg gagatcagat 8220
   ccgcgtccag gtcctccgcg ttgctcaggg cgaacggagt caactttggt agctgccttc 8280
   ccaaaaaggg cgcgtgccca ggctttgagt tgcactcgca ccgtagtggc atcaaaaggt 8340
   gaccgtgccc ggtctgggcg ttaggataca gcgcctgcat aaaagccttg atctgcttaa 8400
   aagccacctg agcctttgcg ccttcagaga agaacatgcc gcaagacttg ccggaaaact 8460
   gattggccgg acaggccgcg tcgtgcacgc agcaccttgc gtcggtgttg gagatctgca 8520
   ccacatttcg gccccaccgg ttcttcacga tcttggcctt gctagactgc tccttcagcg 8580
   cgcgctgccc gttttcgctc gtcacatcca tttcaatcac gtgctcctta tttatcataa 8640
   tgcttccgtg tagacactta agctcgcctt cgatctcagc gcagcggtgc agccacaacg 8700
   cgcagcccgt gggctcgtga tgcttgtagg tcacctctgc aaacgactgc aggtacgcct 8760
   gcaggaatcg ccccatcatc gtcacaaagg tcttgttgct ggtgaaggtc agctgcaacc 8820
   cgcggtgctc ctcgttcagc caggtcttgc atacggccgc cagagcttcc acttggtcag 8880
   gcagtagttt gaagttcgcc tttagatcgt tatccacgtg gtacttgtcc atcagcgcgc 8940
   gcgcagcctc catgcccttc tcccacgcag acacgatcgg cacactcagc gggttcatca 9000
   ccgtaatttc actttccgct tcgctgggct cttcctcttc ctcttgcgtc cgcataccac 9060
   gcgccactgg gtcgtcttca ttcagccgcc gcactgtgcg cttacctcct ttgccatgct 9120
   tgattagcac cggtgggttg ctgaaaccca ccatttgtag cgccacatct tctctttctt 9180
   cctcgctgtc cacgattacc tctggtgatg gcgggcgctc gggcttggga gaagggcgct 9240
   tctttttctt cttgggcgca atggccaaat ccgccgccga ggtcgatggc cgcgggctgg 9300
   gtgtgcgcgg caccagcgcg tcttgtgatg agtcttcctc gtcctcggac tcgatacgcc 9360
   gcctcatccg cttttttggg ggcgcccggg gaggcggcgg cgacggggac ggggacgaca 9420
   cgtcctccat ggttggggga cgtcgcgccg caccgcgtcc gcgctcgggg gtggtttcgc 9480
   gctgctcctc ttcccgactg gccatttcct tctcctatag gcagaaaaag atcatggagt 9540
   cagtcgagaa gaaggacagc ctaaccgccc cctctgagtt cgccaccacc gcctccaccg 9600
   atgccgccaa cgcgcctacc accttccccg tcgaggcacc cccgcttgag gaggaggaag 9660
   tgattatcga gcaggaccca ggttttgtaa gcgaagacga cgaggaccgc tcagtaccaa 9720
   cagaggataa aaagcaagac caggacaacg cagaggcaaa cgaggaacaa gtcgggcggg 9780
   gggacgaaag gcatggcgac tacctagatg tgggagacga cgtgctgttg aagcatctgc 9840
   agcgccagtg cgccattatc tgcgacgcgt tgcaagagcg cagcgatgtg cccctcgcca 9900
   tagcggatgt cagccttgcc tacgaacgcc acctattctc accgcgcgta ccccccaaac 9960
   gccaagaaaa cggcacatgc gagcccaacc cgcgcctcaa cttctacccc gtatttgccg 10020
   tgccagaggt gcttgccacc tatcacatct ttttccaaaa ctgcaagata cccctatcct 10080
   gccgtgccaa ccgcagccga gcggacaagc agctggcctt gcggcagggc gctgtcatac 10140
   ctgatatcgc ctcgctcaac gaagtgccaa aaatctttga gggtcttgga cgcgacgaga 10200
   agcgcgcggc aaacgctctg caacaggaaa acagcgaaaa tgaaagtcac tctggagtgt 10260
   tggtggaact cgagggtgac aacgcgcgcc tagccgtact aaaacgcagc atcgaggtca 10320
   cccactttgc ctacccggca cttaacctac cccccaaggt catgagcaca gtcatgagtg 10380
   agctgatcgt gcgccgtgcg cagcccctgg agagggatgc aaatttgcaa gaacaaacag 10440
   aggagggcct acccgcagtt ggcgacgagc agctagcgcg ctggcttcaa acgcgcgagc 10500
   ctgccgactt ggaggagcga cgcaaactaa tgatggccgc agtgctcgtt accgtggagc 10560
   ttgagtgcat gcagcggttc tttgctgacc cggagatgca gcgcaagcta gaggaaacat 10620
   tgcactacac ctttcgacag ggctacgtac gccaggcctg caagatctcc aacgtggagc 10680
   tctgcaacct ggtctcctac cttggaattt tgcacgaaaa ccgccttggg caaaacgtgc 10740
   ttcattccac gctcaagggc gaggcgcgcc gcgactacgt ccgcgactgc gtttacttat 10800
   ttctatgcta cacctggcag acggccatgg gcgtttggca gcagtgcttg gaggagtgca 10860
   acctcaagga gctgcagaaa ctgctaaagc aaaacttgaa ggacctatgg acggccttca 10920
   acgagcgctc cgtggccgcg cacctggcgg acatcatttt ccccgaacgc ctgcttaaaa 10980
   ccctgcaaca gggtctgcca gacttcacca gtcaaagcat gttgcagaac tttaggaact 11040
   ttatcctaga gcgctcagga atcttgcccg ccacctgctg tgcacttcct agcgactttg 11100
   tgcccattaa gtaccgcgaa tgccctccgc cgctttgggg ccactgctac cttctgcagc 11160
   tagccaacta ccttgcctac cactctgaca taatggaaga cgtgagcggt gacggtctac 11220
   tggagtgtca ctgtcgctgc aacctatgca ccccgcaccg ctccctggtt tgcaattcgc 11280
   agctgcttaa cgaaagtcaa attatcggta cctttgagct gcagggtccc tcgcctgacg 11340
   aaaagtccgc ggctccgggg ttgaaactca ctccggggct gtggacgtcg gcttaccttc 11400
   gcaaatttgt acctgaggac taccacgccc acgagattag gttctacgaa gaccaatccc 11460
   gcccgccaaa tgcggagctt accgcctgcg tcattaccca gggccacatt cttggccaat 11520
   tgcaagccat caacaaagcc cgccaagagt ttctgctacg aaagggacgg ggggtttact 11580
   tggaccccca gtccggcgag gagctcaacc caatcccccc gccgccgcag ccctatcagc 11640
   agcagccgcg ggcccttgct tcccaggatg gcacccaaaa agaagctgca gctgccgccg 11700
   ccacccacgg acgaggagga atactgggac agtcaggcag aggaggtttt ggacgaggag 11760
   gaggaggaca tgatggaaga ctgggagagc ctagacgagg aagcttccga ggtcgaagag 11820
   gtgtcagacg aaacaccgtc accctcggtc gcattcccct cgccggcgcc ccagaaatcg 11880
   gcaaccggtt ccagcatggc tacaacctcc gctcctcagg cgccgccggc actgcccgtt 11940
   cgccgaccca accgtagatg ggacaccact ggaaccaggg ccggtaagtc caagcagccg 12000
   ccgccgttag cccaagagca acaacagcgc caaggctacc gctcatggcg cgggcacaag 12060
   aacgccatag ttgcttgctt gcaagactgt gggggcaaca tctccttcgc ccgccgcttt 12120
   cttctctacc atcacggcgt ggccttcccc cgtaacatcc tgcattacta ccgtcatctc 12180
   tacagcccat actgcaccgg cggcagcggc agcggcagca acagcagcgg ccacacagaa 12240
   gcaaaggcga ccggatagca agactctgac aaagcccaag aaatccacag cggcggcagc 12300
   agcaggagga ggagcgctgc gtctggcgcc caacgaaccc gtatcgaccc gcgagcttag 12360
   aaacaggatt tttcccactc tgtatgctat atttcaacag agcaggggcc aagaacaaga 12420
   gctgaaaata aaaaacaggt ctctgcgatc cctcacccgc agctgcctgt atcacaaaag 12480
   cgaagatcag cttcggcgca cgctggaaga cgcggaggct ctcttcagta aatactgcgc 12540
   gctgactctt aaggactagt ttcgcgccct ttctcaaatt taagcgcgaa aactacgtca 12600
   tctccagcgg ccacacccgg cgccagcacc tgtcgtcagc gccattatga gcaaggaaat 12660
   tcccacgccc tacatgtgga gttaccagcc acaaatggga cttgcggctg gagctgccca 12720
   agactactca acccgaataa actacatgag cgcgggaccc cacatgatat cccgggtcaa 12780
   cggaatccgc gcccaccgaa accgaattct cttggaacag gcggctatta ccaccacacc 12840
   tcgtaataac cttaatcccc gtagttggcc cgctgccctg gtgtaccagg aaagtcccgc 12900
   tcccaccact gtggtacttc ccagagacgc ccaggccgaa gttcagatga ctaactcagg 12960
   ggcgcagctt gcgggcggct ttcgtcacag ggtgcggtcg cccgggcagg gtataactca 13020
   cctgacaatc agagggcgag gtattcagct caacgacgag tcggtgagct cctcgcttgg 13080
   tctccgtccg gacgggacat ttcagatcgg cggcgccggc cgctcttcat tcacgcctcg 13140
   tcaggcaatc ctaactctgc agacctcgtc ctctgagccg cgctctggag gcattggaac 13200
   tctgcaattt attgaggagt ttgtgccatc ggtctacttt aaccccttct cgggacctcc 13260
   cggccactat ccggatcaat ttattcctaa ctttgacgcg gtaaaggact cggcggatgg 13320
   ctacgactga atgttaagtg gagaggcaga gcaactgcgc ctgaaacacc tggtccactg 13380
   tcgccgccac aagtgctttg cccgcgactc cggtgagttt tgctactttg aattgcccga 13440
   ggatcatatc gagggcccgg cgcacggcgt ccggcttacc gcccagggag agcttgcccg 13500
   tagcctgatt cgggagttta cccagcgccc cctgctagtt gagcgggaca ggggaccctg 13560
   tgttctcact gtgatttgca actgtcctaa ccctggatta catcaagatc tttgttgcca 13620
   tctctgtgct gagtataata aatacagaaa ttaaaatata ctggggctcc tatcgccatc 13680
   ctgtaaacgc caccgtcttc acccgcccaa gcaaaccaag gcgaacctta cctggtactt 13740
   ttaacatctc tccctctgtg atttacaaca gtttcaaccc agacggagtg agtctacgag 13800
   agaacctctc cgagctcagc tactccatca gaaaaaacac caccctcctt acctgccggg 13860
   aacgtacgag tgcgtcaccg gccgctgcac cacacctacc gcctgaccgt aaaccagact 13920
   ttttccggac agacctcaat aactctgttt accagaacag gaggtgagct tagaaaaccc 13980
   ttagggtatt aggccaaagg cgcagctact gtggggttta tgaacaattc aagcaactct 14040
   acgggctatt ctaattcagg tttctctaga aatggacgga attattacag agcagcgcct 14100
   gctagaaaga cgcagggcag cggccgagca acagcgcatg aatcaagagc tccaagacat 14160
   ggttaacttg caccagtgca aaaggggtat cttttgtctg gtaaagcagg ccaaagtcac 14220
   ctacgacagt aataccaccg gacaccgcct tagctacaag ttgccaacca agcgtcagaa 14280
   attggtggtc atggtgggag aaaagcccat taccataact cagcactcgg tagaaaccga 14340
   aggctgcatt cactcacctt gtcaaggacc tgaggatctc tgcaccctta ttaagaccct 14400
   gtgcggtctc aaagatctta ttccctttaa ctaataaaaa aaaataataa agcatcactt 14460
   acttaaaatc agttagcaaa tttctgtcca gtttattcag cagcacctcc ttgccctcct 14520
   cccagctctg gtattgcagc ttcctcctgg ctgcaaactt tctccacaat ctaaatggaa 14580
   tgtcagtttc ctcctgttcc tgtccatccg cacccactat cttcatgttg ttgcagatga 14640
   agcgcgcaag accgtctgaa gataccttca accccgtgta tccatatgac acggaaaccg 14700
   gtcctccaac tgtgcctttt cttactcctc cctttgtatc ccccaatggg tttcaagaga 14760
   gtccccctgg ggtactctct ttgcgcctat ccgaacctct agttacctcc aatggcatgc 14820
   ttgcgctcaa aatgggcaac ggcctctctc tggacgaggc cggcaacctt acctcccaaa 14880
   atgtaaccac tgtgagccca cctctcaaaa aaaccaagtc aaacataaac ctggaaatat 14940
   ctgcacccct cacagttacc tcagaagccc taactgtggc tgccgccgca cctctaatgg 15000
   tcgcgggcaa cacactcacc atgcaatcac aggccccgct aaccgtgcac gactccaaac 15060
   ttagcattgc cacccaagga cccctcacag tgtcagaagg aaagctagcc ctgcaaacat 15120
   caggccccct caccaccacc gatagcagta cccttactat cactgcctca ccccctctaa 15180
   ctactgccac tggtagcttg ggcattgact tgaaagagcc catttataca caaaatggaa 15240
   aactaggact aaagtacggg gctcctttgc atgtaacaga cgacctaaac actttgaccg 15300
   tagcaactgg tccaggtgtg actattaata atacttcctt gcaaactaaa gttactggag 15360
   ccttgggttt tgattcacaa ggcaatatgc aacttaatgt agcaggagga ctaaggattg 15420
   attctcaaaa cagacgcctt atacttgatg ttagttatcc gtttgatgct caaaaccaac 15480
   taaatctaag actaggacag ggccctcttt ttataaactc agcccacaac ttggatatta 15540
   actacaacaa aggcctttac ttgtttacag cttcaaacaa ttccaaaaag cttgaggtta 15600
   acctaagcac tgccaagggg ttgatgtttg acgctacagc catagccatt aatgcaggag 15660
   atgggcttga atttggttca cctaatgcac caaacacaaa tcccctcaaa acaaaaattg 15720
   gccatggcct agaatttgat tcaaacaagg ctatggttcc taaactagga actggcctta 15780
   gttttgacag cacaggtgcc attacagtag gaaacaaaaa taatgataag ctaactttgt 15840
   ggaccacacc agctccatct cctaactgta gactaaatgc agagaaagat gctaaactca 15900
   ctttggtctt aacaaaatgt ggcagtcaaa tacttgctac agtttcagtt ttggctgtta 15960
   aaggcagttt ggctccaata tctggaacag ttcaaagtgc tcatcttatt ataagatttg 16020
   acgaaaatgg agtgctacta aacaattcct tcctggaccc agaatattgg aactttagaa 16080
   atggagatct tactgaaggc acagcctata caaacgctgt tggatttatg cctaacctat 16140
   cagcttatcc aaaatctcac ggtaaaactg ccaaaagtaa cattgtcagt caagtttact 16200
   taaacggaga caaaactaaa cctgtaacac taaccattac actaaacggt acacaggaaa 16260
   caggagacac aactccaagt gcatactcta tgtcattttc atgggactgg tctggccaca 16320
   actacattaa tgaaatattt gccacatcct cttacacttt ttcatacatt gcccaagaat 16380
   aaagaatcgt ttgtgttatg tttcaacgtg tttatttttc aattgcagaa aatttcaagt 16440
   catttttcat tcagtagtat agccccacca ccacatagct tatacagatc accgtacctt 16500
   aatcaaactc acagaaccct agtattcaac ctgccacctc cctcccaaca cacagagtac 16560
   acagtccttt ctccccggct ggccttaaaa agcatcatat catgggtaac agacatattc 16620
   ttaggtgtta tattccacac ggtttcctgt cgagccaaac gctcatcagt gatattaata 16680
   aactccccgg gcagctcact taagttcatg tcgctgtcca gctgctgagc cacaggctgc 16740
   tgtccaactt gcggttgctt aacgggcggc gaaggagaag tccacgccta catgggggta 16800
   gagtcataat cgtgcatcag gatagggcgg tggtgctgca gcagcgcgcg aataaactgc 16860
   tgccgccgcc gctccgtcct gcaggaatac aacatggcag tggtctcctc agcgatgatt 16920
   cgcaccgccc gcagcataag gcgccttgtc ctccgggcac agcagcgcac cctgatctca 16980
   cttaaatcag cacagtaact gcagcacagc accacaatat tgttcaaaat cccacagtgc 17040
   aaggcgctgt atccaaagct catggcgggg accacagaac ccacgtggcc atcataccac 17100
   aagcgcaggt agattaagtg gcgacccctc ataaacacgc tggacataaa cattacctct 17160
   tttggcatgt tgtaattcac cacctcccgg taccatataa acctctgatt aaacatggcg 17220
   ccatccacca ccatcctaaa ccagctggcc aaaacctgcc cgccggctat acactgcagg 17280
   gaaccgggac tggaacaatg acagtggaga gcccaggact cgtaaccatg gatcatcatg 17340
   ctcgtcatga tatcaatgtt ggcacaacac aggcacacgt gcatacactt cctcaggatt 17400
   acaagctcct cccgcgttag aaccatatcc cagggaacaa cccattcctg aatcagcgta 17460
   aatcccacac tgcagggaag acctcgcacg taactcacgt tgtgcattgt caaagtgtta 17520
   cattcgggca gcagcggatg atcctccagt atggtagcgc gggtttctgt ctcaaaagga 17580
   ggtagacgat ccctactgta cggagtgcgc cgagacaacc gagatcgtgt tggtcgtagt 17640
   gtcatgccaa atggaacgcc ggacgtagtc atatttcctg aagcaaaacc aggtgcgggc 17700
   gtgacaaaca gatctgcgtc tccggtctcg ccgcttagat cgctctgtgt agtagttgta 17760
   gtatatccac tctctcaaag catccaggcg ccccctggct tcgggttcta tgtaaactcc 17820
   ttcatgcgcc gctgccctga taacatccac caccgcagaa taagccacac ccagccaacc 17880
   tacacattcg ttctgcgagt cacacacggg aggagcggga agagctggaa gaaccatgtt 17940
   ttttttttta ttccaaaaga ttatccaaaa cctcaaaatg aagatctatt aagtgaacgc 18000
   gctcccctcc ggtggcgtgg tcaaactcta cagccaaaga acagataatg gcatttgtaa 18060
   gatgttgcac aatggcttcc aaaaggcaaa cggccctcac gtccaagtgg acgtaaaggc 18120
   taaacccttc agggtgaatc tcctctataa acattccagc accttcaacc atgcccaaat 18180
   aattctcatc tcgccacctt ctcaatatat ctctaagcaa atcccgaata ttaagtccgg 18240
   ccattgtaaa aatctgctcc agagcgccct ccaccttcag cctcaagcag cgaatcatga 18300
   ttgcaaaaat tcaggttcct cacagacctg tataagattc aaaagcggaa cattaacaaa 18360
   aataccgcga tcccgtaggt cccttcgcag ggccagctga acataatcgt gcaggtctgc 18420
   acggaccagc gcggccactt ccccgccagg aaccatgaca aaagaaccca cactgattat 18480
   gacacgcata ctcggagcta tgctaaccag cgtagccccg atgtaagctt gttgcatggg 18540
   cggcgatata aaatgcaagg tgctgctcaa aaaatcaggc aaagcctcgc gcaaaaaaga 18600
   aagcacatcg tagtcatgct catgcagata aaggcaggta agctccggaa ccaccacaga 18660
   aaaagacacc atttttctct caaacatgtc tgcgggtttc tgcataaaca caaaataaaa 18720
   taacaaaaaa acatttaaac attagaagcc tgtcttacaa caggaaaaac aacccttata 18780
   agcataagac ggactacggc catgccggcg tgaccgtaaa aaaactggtc accgtgatta 18840
   aaaagcacca ccgacagctc ctcggtcatg tccggagtca taatgtaaga ctcggtaaac 18900
   acatcaggtt gattcacatc ggtcagtgct aaaaagcgac cgaaatagcc cgggggaata 18960
   catacccgca ggcgtagaga caacattaca gcccccatag gaggtataac aaaattaata 19020
   ggagagaaaa acacataaac acctgaaaaa ccctcctgcc taggcaaaat agcaccctcc 19080
   cgctccagaa caacatacag cgcttccaca gcggcagcca taacagtcag ccttaccagt 19140
   aaaaaagaaa acctattaaa aaaacaccac tcgacacggc accagctcaa tcagtcacag 19200
   tgtaaaaaag ggccaagtgc agagcgagta tatataggac taaaaaatga cgtaacggtt 19260
   aaagtccaca aaaaacaccc agaaaaccgc acgcgaacct acgcccagaa acgaaagcca 19320
   aaaaacccac aacttcctca aatcgtcact tccgttttcc cacgttacgt cacttcccat 19380
   tttaagaaaa ctacaattcc caacacatac aagttactcc gccctaaaac ctacgtcacc 19440
   cgccccgttc ccacgccccg cgccacgtca caaactccac cccctcatta tcatattggc 19500
   ttcaatccaa aataaggtat attattgatg atgttaatta agaattcgga tctgcgacgc 19560
   gaggctggat ggccttcccc attatgattc ttctcgcttc cggcggcatc gggatgcccg 19620
   cgttgcaggc catgctgtcc aggcaggtag atgacgacca tcagggacag cttcacggcc 19680
   agcaaaaggc caggaaccgt aaaaaggccg cgttgctggc gtttttccat aggctccgcc 19740
   cccctgacga gcatcacaaa aatcgacgct caagtcagag gtggcgaaac ccgacaggac 19800
   tataaagata ccaggcgttt ccccctggaa gctccctcgt gcgctctcct gttccgaccc 19860
   tgccgcttac cggatacctg tccgcctttc tcccttcggg aagcgtggcg ctttctcaat 19920
   gctcacgctg taggtatctc agttcggtgt aggtcgttcg ctccaagctg ggctgtgtgc 19980
   acgaaccccc cgttcagccc gaccgctgcg ccttatccgg taactatcgt cttgagtcca 20040
   acccggtaag acacgactta tcgccactgg cagcagccac tggtaacagg attagcagag 20100
   cgaggtatgt aggcggtgct acagagttct tgaagtggtg gcctaactac ggctacacta 20160
   gaaggacagt atttggtatc tgcgctctgc tgaagccagt taccttcgga aaaagagttg 20220
   gtagctcttg atccggcaaa caaaccaccg ctggtagcgg tggttttttt gtttgcaagc 20280
   agcagattac gcgcagaaaa aaaggatctc aagaagatcc tttgatcttt tctacggggt 20340
   ctgacgctca gtggaacgaa aactcacgtt aagggatttt ggtcatgaga ttatcaaaaa 20400
   ggatcttcac ctagatcctt ttaaatcaat ctaaagtata tatgagtaaa cttggtctga 20460
   cagttaccaa tgcttaatca gtgaggcacc tatctcagcg atctgtctat ttcgttcatc 20520
   catagttgcc tgactccccg tcgtgtagat aactacgata cgggagggct taccatctgg 20580
   ccccagtgct gcaatgatac cgcgagaccc acgctcaccg gctccagatt tatcagcaat 20640
   aaaccagcca gccggaaggg ccgagcgcag aagtggtcct gcaactttat ccgcctccat 20700
   ccagtctatt aattgttgcc gggaagctag agtaagtagt tcgccagtta atagtttgcg 20760
   caacgttgtt gccattgntg caggcatcgt ggtgtcacgc tcgtcgtttg gtatggcttc 20820
   attcagctcc ggttcccaac gatcaaggcg agttacatga tcccccatgt tgtgcaaaaa 20880
   agcggttagc tccttcggtc ctccgatcgt tgtcagaagt aagttggccg cagtgttatc 20940
   actcatggtt atggcagcac tgcataattc tcttactgtc atgccatccg taagatgctt 21000
   ttctgtgact ggtgagtact caaccaagtc attctgagaa tagtgtatgc ggcgaccgag 21060
   ttgctcttgc ccggcgtcaa cacgggataa taccgcgcca catagcagaa ctttaaaagt 21120
   gctcatcatt ggaaaacgtt cttcggggcg aaaactctca aggatcttac cgctgttgag 21180
   atccagttcg atgtaaccca ctcgtgcacc caactgatct tcagcatctt ttactttcac    21240
   cagcgtttct gggtgagcaa aaacaggaag gcaaaatgcc gcaaaaaagg gaataagggc    21300
   gacacggaaa tgttgaatac tcatactctt cctttttcaa tattattgaa gcatttatca    21360
   gggttattgt ctcatgagcg gatacatatt tgaatgtatt tagaaaaata aacaaatagg    21420
   ggttccgcgc acatttcccc gaaaagtgcc acctgacgtc taagaaacca ttattatcat    21480
   gacattaacc tataaaaata ggcgtatcac gaggcccttt cgtcttcaa                21529
<210> 6
   <211> 5824
   <212> DNA
   <213> Künstliche Sequenz
<400> 6
   ggatcccctg agggggcccc catgggctag aggatccggc ctcggcctct gcataaataa      60
   aaaaaattag tcagccatga gcttggccca ttgcatacgt tgtatccata tcataatatg     120
   tacatttata ttggctcatg tccaacatta ccgccatgtt gacattgatt attgactagt     180
   tattaatagt aatcaattac ggggtcatta gttcatagcc catatatgga gttccgcgtt     240
   acataactta cggtaaatgg cccgcctggc tgaccgccca acgacccccg cccattgacg     300
   tcaataatga cgtatgttcc catagtaacg ccaataggga ctttccattg acgtcaatgg     360
   gtggagtatt tacggtaaac tgcccacttg gcagtacatc aagtgtatca tatgccaagt     420
   acgcccccta ttgacgtcaa tgacggtaaa tggcccgcct ggcattatgc ccagtacatg     480
   accttatggg actttcctac ttggcagtac atctacgtat tagtcatcgc tattaccatg     540
   gtgatgcggt tttggcagta catcaatggg cgtggatagc ggtttgactc acggggattt     600
   ccaagtctcc accccattga cgtcaatggg agtttgtttt ggcaccaaaa tcaacgggac     660
   tttccaaaat gtcgtaacaa ctccgcccca ttgacgcaaa tgggcggtag gcgtgtacgg     720
   tgggaggtct atataagcag agctcgttta gtgaaccgtc agatcgcctg gagacgccat     780
   ccacgctgtt ttgacctcca tagaagacac cgggaccgat ccagcctccc ctcgaagctt     840
   acatgtggta ccgagctcgg atcctgagaa cttcagggtg agtctatggg acccttgatg     900
   ttttctttcc ccttcttttc tatggttaag ttcatgtcat aggaagggga gaagtaacag     960
   ggtacacata ttgaccaaat cagggtaatt ttgcatttgt aattttaaaa aatgctttct    1020
   tcttttaata tacttttttg tttatcttat ttctaatact ttccctaatc tctttctttc    1080
   agggcaataa tgatacaatg tatcatgcct ctttgcacca ttctaaagaa taacagtgat    1140
   aatttctggg ttaaggcaat agcaatattt ctgcatataa atatttctgc atataaattg    1200
   taactgatgt aagaggtttc atattgctaa tagcagctac aatccagcta ccattctgct    1260
   tttattttat ggttgggata aggctggatt attctgagtc caagctaggc ccttttgcta    1320
   atcatgttca tacctcttat cttcctccca cagctcctgg gcaacgtgct ggtctgtgtg 1380
   ctggcccatc actttggcaa agcacgtgag atctgaattc aacagagatc gatctgtttc 1440
   cttgacacta tgaagtgcct tttgtactta gcctttttat tcattggggt gaattgcaag 1500
   ttcaccatag tttttccaca caaccaaaaa ggaaactgga aaaatgttcc ttctaattac 1560
   cattattgcc cgtcaagctc agatttaaat tggcataatg acttaatagg cacagccata 1620
   caagtcaaaa tgcccaagag tcacaaggct attcaagcag acggttggat gtgtcatgct 1680
   tccaaatggg tcactacttg tgatttccgc tggtatggac cgaagtatat aacacagtcc 1740
   atccgatcct tcactccatc tgtagaacaa tgcaaggaaa gcattgaaca aacgaaacaa 1800
   ggaacttggc tgaatccagg cttccctcct caaagttgtg gatatgcaac tgtgacggat 1860
   gccgaagcag tgattgtcca ggtgactcct caccatgtgc tggttgatga atacacagga 1920
   gaatgggttg attcacagtt catcaacgga aaatgcagca attacatatg ccccactgtc 1980
   cataactcta caacctggca ttctgactat aaggtcaaag ggctatgtga ttctaacctc 2040
   atttccatgg acatcacctt cttctcagag gacggagagc tatcatccct gggaaaggag 2100
   ggcacagggt tcagaagtaa ctactttgct tatgaaactg gaggcaaggc ctgcaaaatg 2160
   caatactgca agcattgggg agtcagactc ccatcaggtg tctggttcga gatggctgat 2220
   aaggatctct ttgctgcagc cagattccct gaatgcccag aagggtcaag tatctctgct 2280
   ccatctcaga cctcagtgga tgtaagtcta attcaggacg ttgagaggat cttggattat 2340
   tccctctgcc aagaaacctg gagcaaaatc agagcgggtc ttccaatctc tccagtggat 2400
   ctcagctatc ttgctcctaa aaacccagga accggtcctg ctttcaccat aatcaatggt 2460
   accctaaaat actttgagac cagatacatc agagtcgata ttgctgctcc aatcctctca 2520
   agaatggtcg gaatgatcag tggaactacc acagaaaggg aactgtggga tgactgggca 2580
   ccatatgaag acgtggaaat tggacccaat ggagttctga ggaccagttc aggatataag 2640
   tttcctttat acatgattgg acatggtatg ttggactccg atcttcatct tagctcaaag 2700
   gctcaggtgt tcgaacatcc tcacattcaa gacgctgctt cgcaacttcc tgatgatgag 2760
   agtttatttt ttggtgatac tgggctatcc aaaaatccaa tcgagcttgt agaaggttgg 2820
   ttcagtagtt ggaaaagctc tattgcctct tttttcttta tcatagggtt aatcattgga 2880
   ctattcttgg ttctccgagt tggtatccat ctttgcatta aattaaagca caccaagaaa 2940
   agacagattt atacagacat agagatgaac cgacttggaa agtaactcaa atcctgcaca 3000
   acagattctt catgtttgga ccaaatcaac ttgtgatacc atgctcaaag aggcctcaat 3060
   tatatttgag tttttaattt ttatggaatt caccccacca gtgcaggctg cctatcagaa 3120
   agtggtggct ggtgtggcta atgccctggc ccacaagttt cactaagctc gcttccttgc 3180
   tgtccaattt ctattaaagg ttccttggtt ccctaagtcc aactactaaa ctgggggata 3240
   ttatgaaggg ccttgagcat ctggattctg cctaataaaa aacatttatt ttcattgcaa 3300
   tgatgtattt aaattatttc tgaatatttt actaaaaagg gaatgtggga ggtcagtgca 3360
   tttaaaacat aaagaaatga agagctagtt caaaccttgg gaaaatacac tatatcttaa 3420
   actccatgaa agaaggtgag gctgcaaaca gctaatgcac attggcaaca gccctgatgc 3480
   ctatgcctta ttcatccctc agaaaaggat tcaagtagag gcttgatttg gaggttaaag 3540
   tttggctatg ctgtatttta cattacttat tgttttagct gtcctcatga atgtcttttc 3600
   actacccatt tgcttatcct gcatctctca gccttgactc cactcagttc tcttgcttag 3660
   agataccacc tttcccctga agtgttcctt ccatgtttta cggcgagatg gtttctcctc 3720
   gcctggccac tcagccttag ttgtctctgt tgtcttatag aggtctactt gaagaaggaa 3780
   aaacaggggg catggtttga ctgtcctgtg agcccttctt ccctgcctcc cccactcaca 3840
   gtgacccgga atccctcgac atggcagtct agcactagtg cggccgcaga tctgcttcct 3900
   cgctcactga ctcgctgcgc tcggtcgttc ggctgcggcg agcggtatca gctcactcaa 3960
   aggcggtaat acggttatcc acagaatcag gggataacgc aggaaagaac atgtgagcaa 4020
   aaggccagca aaaggccagg aaccgtaaaa aggccgcgtt gctggcgttt ttccataggc 4080
   tccgcccccc tgacgagcat cacaaaaatc gacgctcaag tcagaggtgg cgaaacccga 4140
   caggactata aagataccag gcgtttcccc ctggaagctc cctcgtgcgc tctcctgttc 4200
   cgaccctgcc gcttaccgga tacctgtccg cctttctccc ttcgggaagc gtggcgcttt 4260
   ctcaatgctc acgctgtagg tatctcagtt cggtgtaggt cgttcgctcc aagctgggct 4320
   gtgtgcacga accccccgtt cagcccgacc gctgcgcctt atccggtaac tatcgtcttg 4380
   agtccaaccc ggtaagacac gacttatcgc cactggcagc agccactggt aacaggatta 4440
   gcagagcgag gtatgtaggc ggtgctacag agttcttgaa gtggtggcct aactacggct 4500
   acactagaag gacagtattt ggtatctgcg ctctgctgaa gccagttacc ttcggaaaaa 4560
   gagttggtag ctcttgatcc ggcaaacaaa ccaccgctgg tagcggtggt ttttttgttt 4620
   gcaagcagca gattacgcgc agaaaaaaag gatctcaaga agatcctttg atcttttcta 4680
   cggggtctga cgctcagtgg aacgaaaact cacgttaagg gattttggtc atgagattat 4740
   caaaaaggat cttcacctag atccttttaa attaaaaatg aagttttaaa tcaatctaaa 4800
   gtatatatga gtaaacttgg tctgacagtt accaatgctt aatcagtgag gcacctatct 4860
   cagcgatctg tctatttcgt tcatccatag ttgcctgact ccccgtcgtg tagataacta 4920
   cgatacggga gggcttacca tctggcccca gtgctgcaat gataccgcga gacccacgct 4980
   caccggctcc agatttatca gcaataaacc agccagccgg aagggccgag cgcagaagtg 5040
   gtcctgcaac tttatccgcc tccatccagt ctattaattg ttgccgggaa gctagagtaa 5100
   gtagttcgcc agttaatagt ttgcgcaacg ttgttgccat tgctacaggc atcgtggtgt     5160
   cacgctcgtc gtttggtatg gcttcattca gctccggttc ccaacgatca aggcgagtta     5220
   catgatcccc catgttgtgc aaaaaagcgg ttagctcctt cggtcctccg atcgttgtca     5280
   gaagtaagtt ggccgcagtg ttatcactca tggttatggc agcactgcat aattctctta     5340
   ctgtcatgcc atccgtaaga tgcttttctg tgactggtga gtactcaacc aagtcattct     5400
   gagaatagtg tatgcggcga ccgagttgct cttgcccggc gtcaatacgg gataataccg     5460
   cgccacatag cagaacttta aaagtgctca tcattggaaa acgttcttcg gggcgaaaac     5520
   tctcaaggat cttaccgctg ttgagatcca gttcgatgta acccactcgt gcacccaact     5580
   gatcttcagc atcttttact ttcaccagcg tttctgggtg agcaaaaaca ggaaggcaaa     5640
   atgccgcaaa aaagggaata agggcgacac ggaaatgttg aatactcata ctcttccttt     5700
   ttcaatatta ttgaagcatt tatcagggtt attgtctcat gagcggatac atatttgaat     5760
   gtatttagaa aaataaacaa ataggggttc cgcgcacatt tccccgaaaa gtgccacctg     5820
   acgt                                                                  5824
<210> 7
   <211> 127
   <212> DNA
   <213> Adeno-assoziiertes Virus 2
<400> 7
   cgcgctcgct cgctcactga ggccgcccgg gcaaagcccg ggcgtcgggc gacctttggt       60
   cgcccggcct cagtgagcga gcgagcgcgc agagagggag tggccaactc catcactagg      120
   ggttcct                                                               127
<210> 8
   <211> 123
   <212> DNA
   <213> Adeno-assoziiertes Virus 2
<400> 8
   aggaacccct agtgatggag ttggccactc cctctctgcg cgctcgctcg ctcactgagg       60
   ccgggcgacc aaaggtcgcc cgacgcccgg gctttgcccg ggcggcctca gtgagcgagc      120
   gag                                                                   123
<210> 9
   <211> 103
   <212> DNA
   <213> Künstliche Sequenz
<400> 9
   cgcgctcgct cgctcactga ggccgcccgg gcaaagcccg ggcgtcgggc gacctttggt 60
   cgcccggcct cagtgagcga gcgagcgcgc agagagggag tgg 103